(19) Europäisches Patentamt
European Patent Office
Office européen des brevets

(11) Numéro de publication: **0 424 244 B1**

(12) # FASCICULE DE BREVET EUROPEEN

(45) Date de publication du fascicule du brevet: **09.08.95**

(51) Int. Cl.$^6$: **C07D 263/24**, A61K 31/42

(21) Numéro de dépôt: **90402892.5**

(22) Date de dépôt: **16.10.90**

Le dossier contient des informations techniques présentées postérieurement au dépôt de la demande et ne figurant pas dans le présent fascicule.

(54) **Dérivés d'aryl-3 oxazolidinone, leur procédé de préparation et leur application en thérapeutique.**

(30) Priorité: **17.10.89 FR 8913555**

(43) Date de publication de la demande:
**24.04.91 Bulletin 91/17**

(45) Mention de la délivrance du brevet:
**09.08.95 Bulletin 95/32**

(84) Etats contractants désignés:
**AT BE CH DE DK ES FR GB GR IT LI LU NL SE**

(56) Documents cités:
**FR-A- 2 458 547**
**GB-A- 2 003 151**

(73) Titulaire: **SYNTHELABO**
**22, Avenue Galilée**
**F-92350 Le Plessis Robinson (FR)**

(72) Inventeur: **Jarreau, François-Xavier**
**5, rue Louis Hervé**
**F-78000 Versailles (FR)**
Inventeur: **Rovei, Vincenzo**
**44, rue Danton**
**F-92500 Rueil Malmaison (FR)**
Inventeur: **Koenig, Jean-Jacques**
**99, avenue du Général de Gaulle**
**F-78600 Maisons Laffitte (FR)**
Inventeur: **Schoofs, Alain**
**242, rue de la Croix-Nivert**
**F-75015 Paris (FR)**

(74) Mandataire: **Kedinger, Jean-Paul et al**
**c/o Cabinet Malemont**
**42, avenue du Président Wilson**
**F-75116 Paris (FR)**

EP 0 424 244 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet de nouveaux dérivés d'aryl-3 oxazolidinone-2, leur procédé de préparation et leur application en thérapeutique.

Ces dérivés répondent plus précisément à la formule :

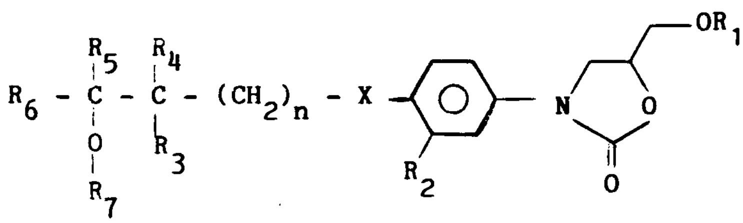

(I)

où :

- $R_1$ représente H ou alkyle en $C_1$-$C_4$ ;
- X représente soit un atome d'oxygène auquel cas $R_2$ = H ou halogène, soit un groupe méthylène ou un groupe -CH=CH-, auquel cas $R_2$ = H ;
- n prend la valeur 1 ou 2 quand X est un atome d'oxygène ou un groupe méthylène et la valeur 0 ou 1 quand X représente le groupe -CH=CH- ;
- $R_3$ et $R_4$ représentent chacun indépendamment l'un de l'autre, H, alkyle en $C_1$-$C_4$ ou cycloalkyle en $C_4$-$C_7$;
- $R_5$ représente H et $R_6$ un groupe $CF_3$ ou cycloalkyle en $C_4$-$C_7$ ou bien $R_5$ est un groupe alkyle en $C_1$-$C_4$ et $R_6$ un groupe alkyle en $C_1$-$C_4$ ou cycloalkyle en $C_4$-$C_7$;
- $R_4$ et $R_6$ peuvent en outre former ensemble une chaîne $-(CH_2)_3-$ ou $-(CH_2)_4-$ ;
- $R_5$ et $R_6$ peuvent en outre former ensemble une chaîne $-(CH_2)_4-$ ou $-(CH_2)_5-$ : et
- $R_7$ représente H, alkyle en $C_1$-$C_4$, ou benzyle.

Par ailleurs, il convient de souligner que les dérivés (I) comportent un ou plusieurs atomes de carbone asymétriques. Ils peuvent donc se présenter sous la forme de diastéréoisomères ou d'énantiomères ou sous forme cis ou trans ou encore sous la forme d'un mélange de toutes ces formes, y compris les formes racémiques. La présente invention s'étend par conséquent aux diverses formes ainsi définies.

La formule (I) ci-dessus couvre notamment les dérivés pour lesquels $R_1$ = H ou $CH_3$ ; $R_2$ = H ; X = oxygène ou $CH_2$ ; n = 1 ou 2 ; $R_3$, $R_4$ et $R_5$ représentant H ou $CH_3$ ; $R_6$ représente $CH_3$ ou $CF_3$ ; et $R_7$ représente H, $CH_3$ ou acétyle.

On citera en particulier :

- les dérivés pour lesquels $R_1$ = $CH_3$ ; $R_2$ = H ; X = oxygène ; n = 1 ou 2 ; $R_3$ = $R_4$ = $R_5$ = H ; $R_6$ = $CF_3$ ; et $R_7$ = H ;
- les dérivés pour lesquels $R_1$ = $CH_3$ ; $R_2$ = H ; X = méthylène ; n = 1 ou 2 ; $R_3$ = $R_4$ = $R_5$ = H ; $R_6$ = $CF_3$ ; et $R_7$ = H ; et
- les dérivés pour lesquels $R_1$ = $CH_3$ ; $R_2$ = H ; X représente CH = CH ; n = 0 ou 1 ; $R_3$ = $R_4$ = $R_5$ = H ; $R_6$ = $CF_3$ ; et $R_7$ = H.

La présente invention a par ailleurs pour objet les procédés de préparation des dérivés (I).

Ces procédés sont essentiellement basés sur deux voies générales de synthèse.

La première de ces voies comprend l'élaboration d'une entité comportant le fragment oxazolidinone-2 (schémas 1 et 2), suivie du greffage sur cette entité de la chaîne comportant le reste

$$-\overset{|}{\underset{|}{C}}-O-R_7 \text{ (schémas 3 et 4)}$$

ou un groupe précurseur de

$$-\overset{|}{\underset{|}{C}}-O-R_7 \text{ (schéma 5).}$$

La deuxième de ces voies comprend, à l'inverse, d'abord l'élaboration de la chaîne comportant le reste

$$-\overset{|}{\underset{|}{C}}-O-R_7 \text{ (schémas 6 et 7)}$$

ou un groupe précurseur de

$$-\overset{|}{\underset{|}{C}}-O-R_7 \text{ (schémas 8 et 9)},$$

suivie de l'élaboration et greffage sur cette chaîne d'une entité comportant le fragment oxazolidinone-2 (schémas 10, 11 et 12).

Ces douze schémas sont représentés ci-après. Sauf indication contraire, les symboles $R_1$, $R_2$, X, n, $R_4$, $R_3$ , $R_5$, $R_6$, et $R_7$ apparaissant dans ces schémas, ont la même signification que dans la formule (I).

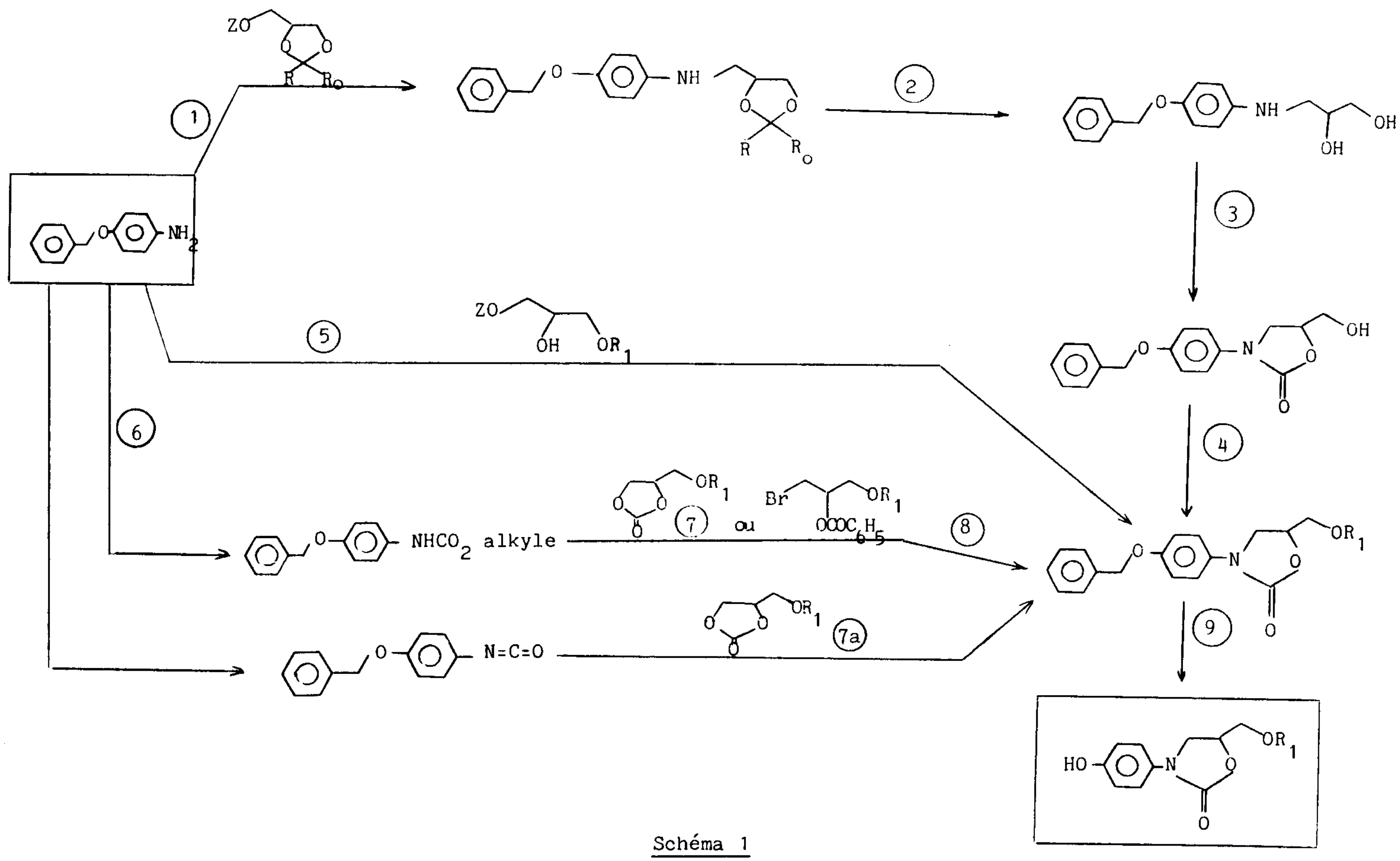


Schéma 1

. Z = Ms ou Ts

. R et $R_o$ représentent alkyle en $C_1$-$C_3$ ou forment ensemble $-(CH_2)_5-$

. $R_1 \neq H$

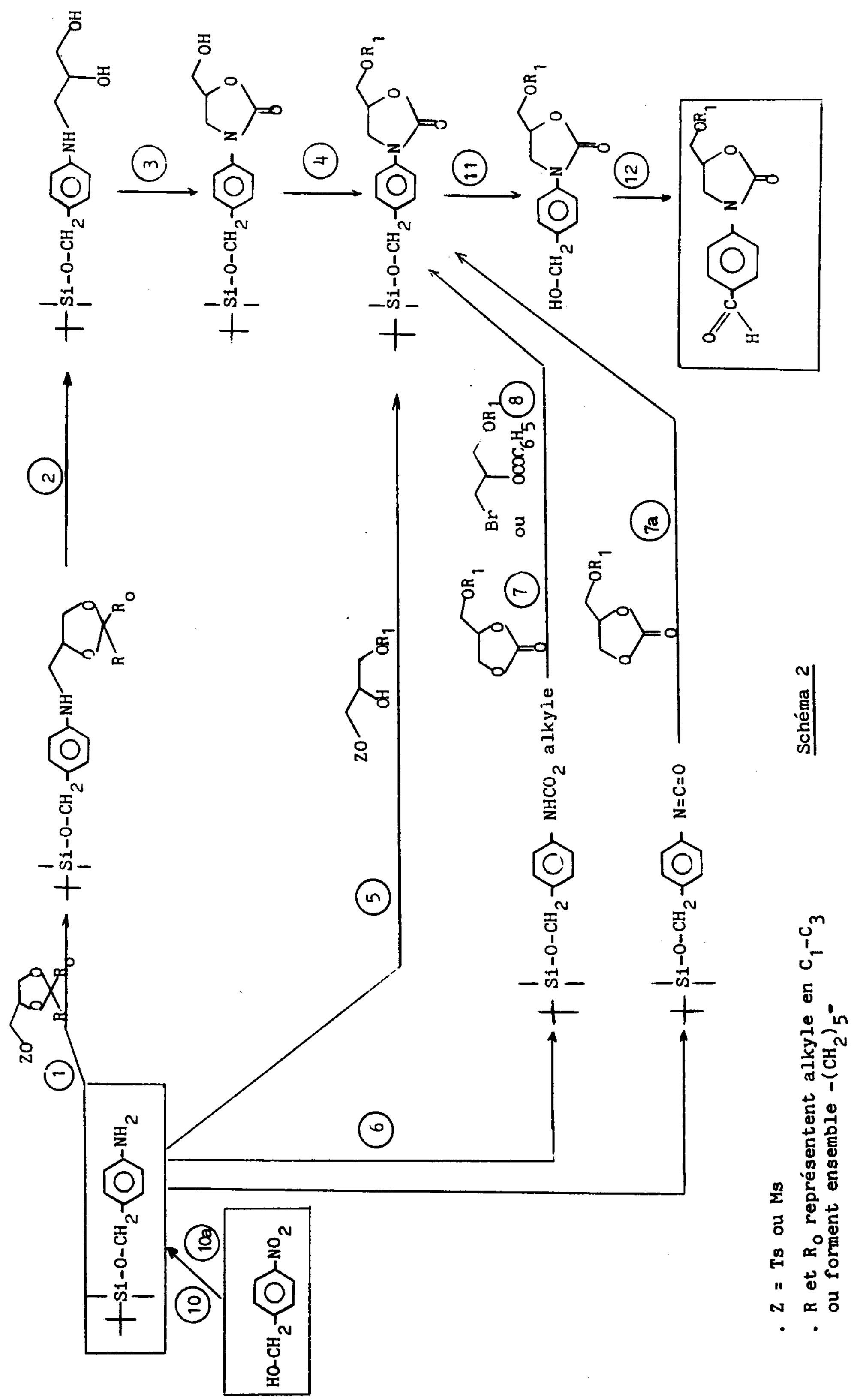


Schéma 2

. Z = Ts ou Ms

. R et $R_o$ représentent alkyle en $C_1$-$C_3$ ou forment ensemble $-(CH_2)_5-$

. $R_1 \neq H$

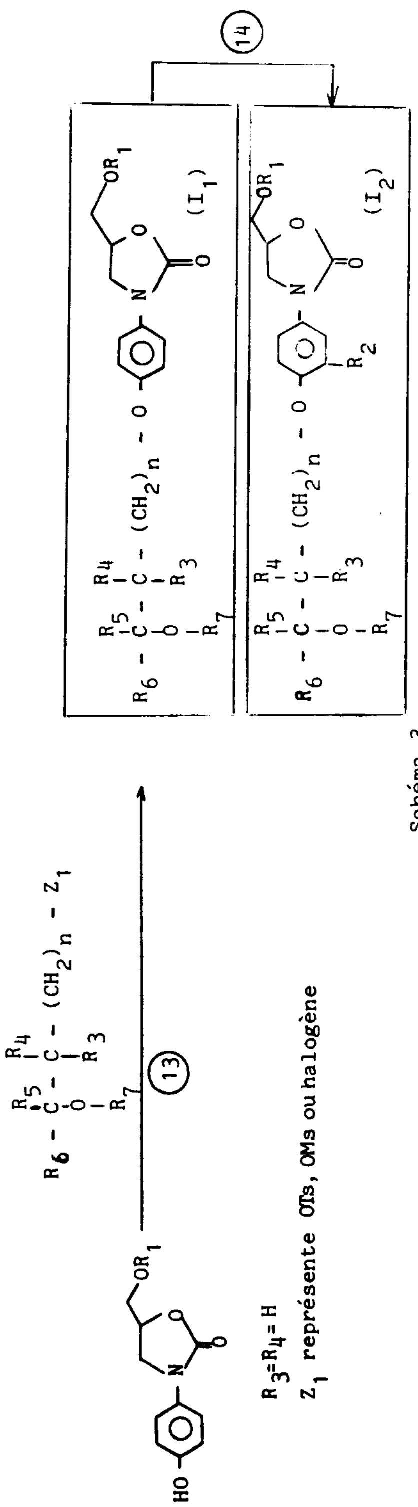


Schéma 3

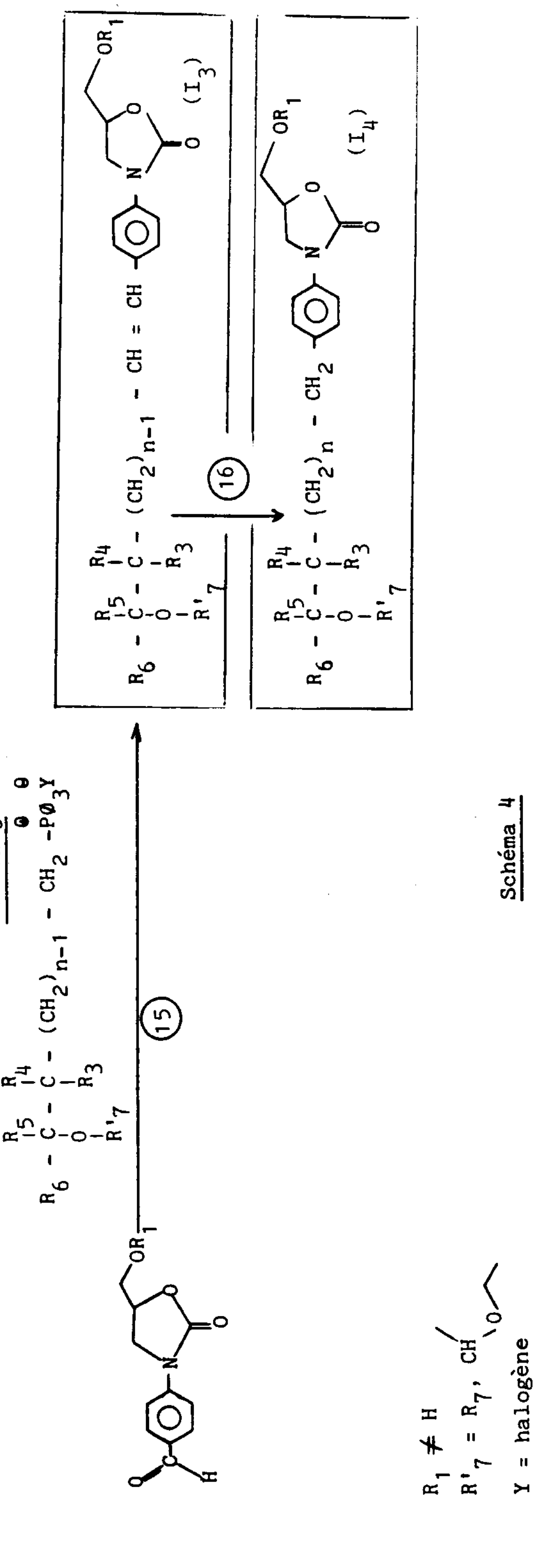


Schéma 4

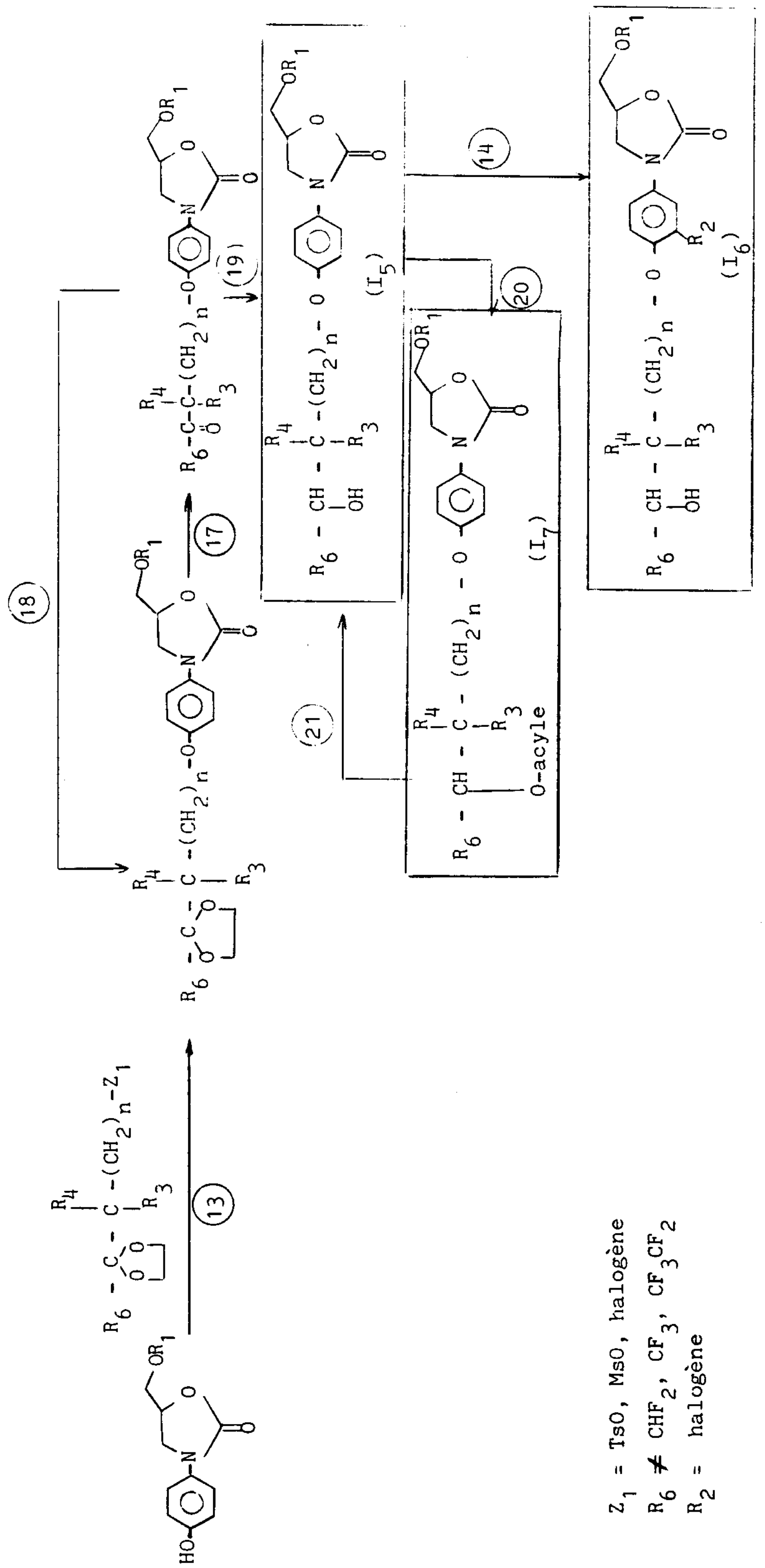


$(I_5)$ $(I_7)$ $(I_6)$

$Z_1$ = TsO, MsO, halogène

$R_6 \neq CHF_2, CF_3, CF_3CF_2$

$R_2$ = halogène

Schéma 5

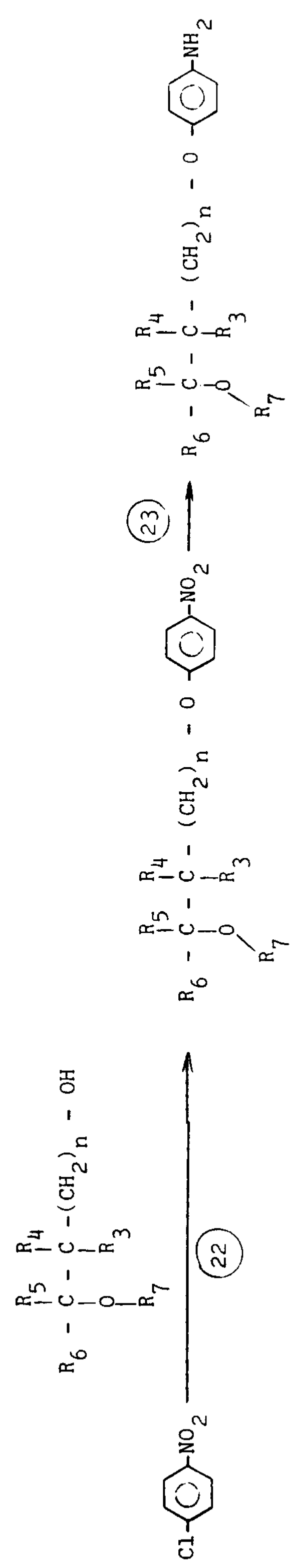


Schéma 6

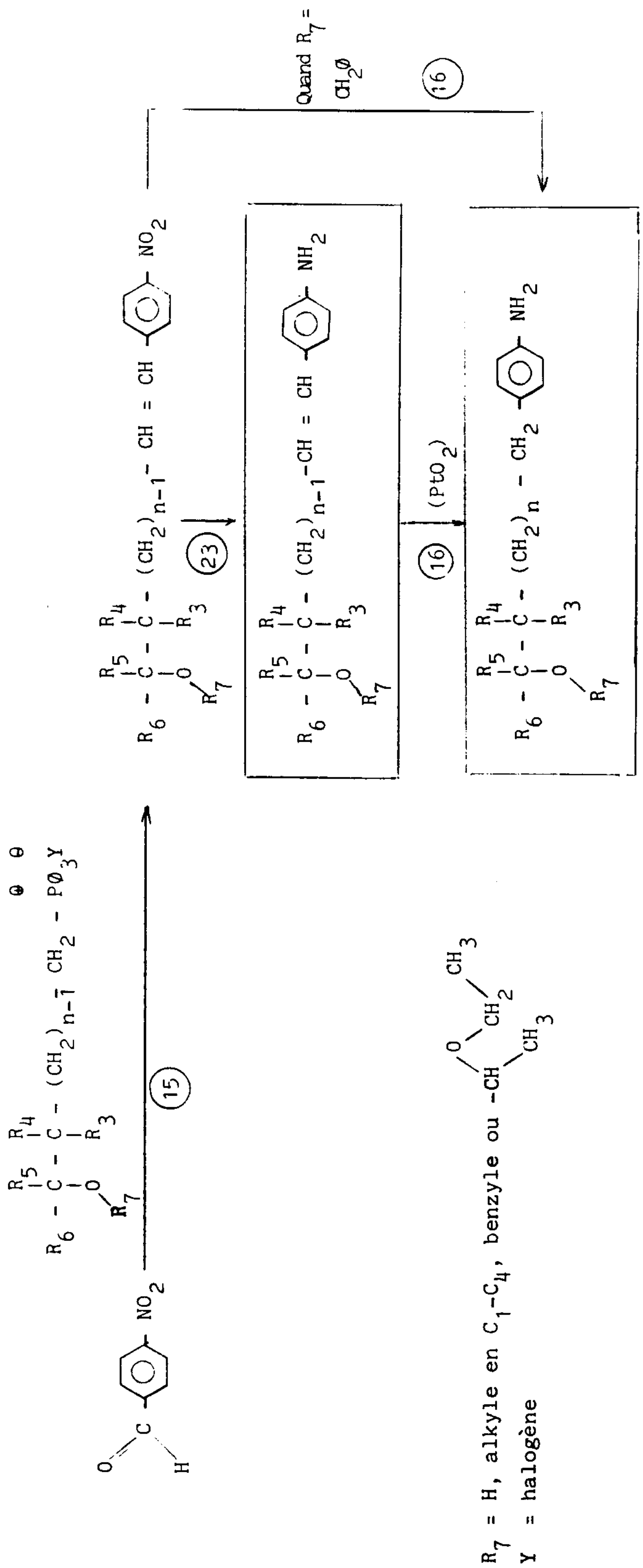


$R_7$ = H, alkyle en $C_1$-$C_4$, benzyle ou

Y = halogène

Schéma 7

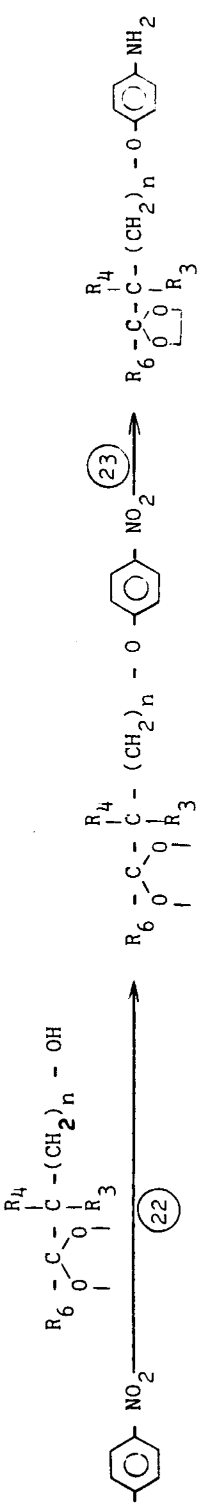


Schéma 8

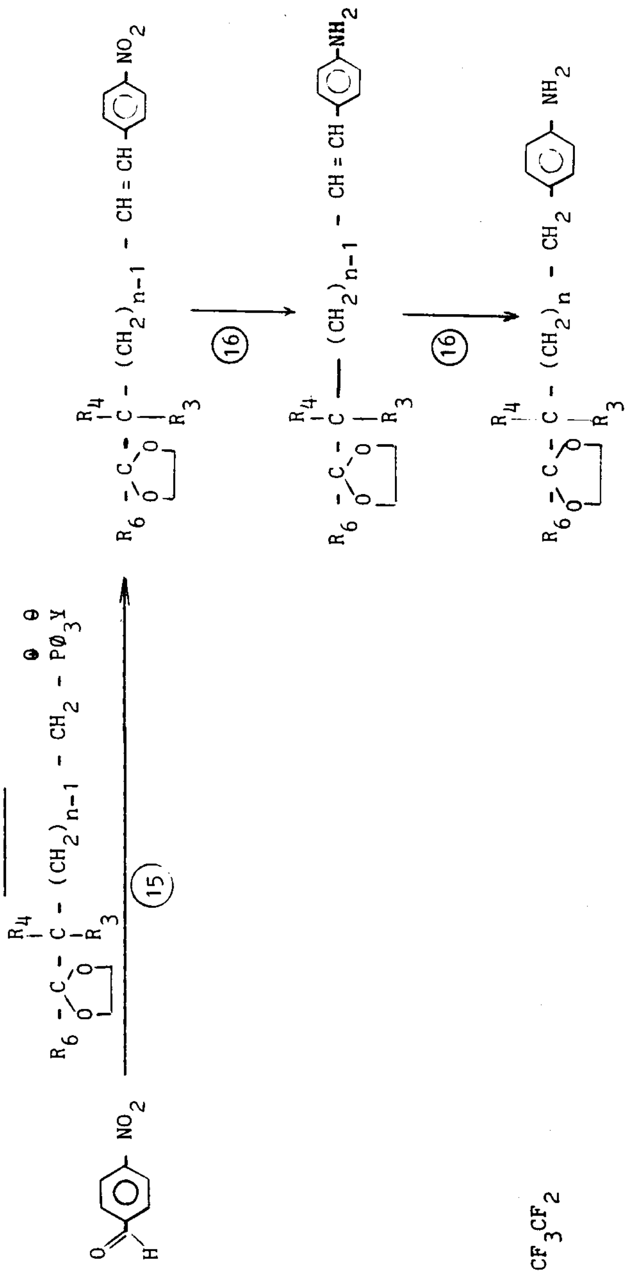


Schéma 9

$R_6 \neq CHF_2, CF_3, CF_3CF_2$

Y = halogène

$(R_3,R_4) \neq (H,H)$

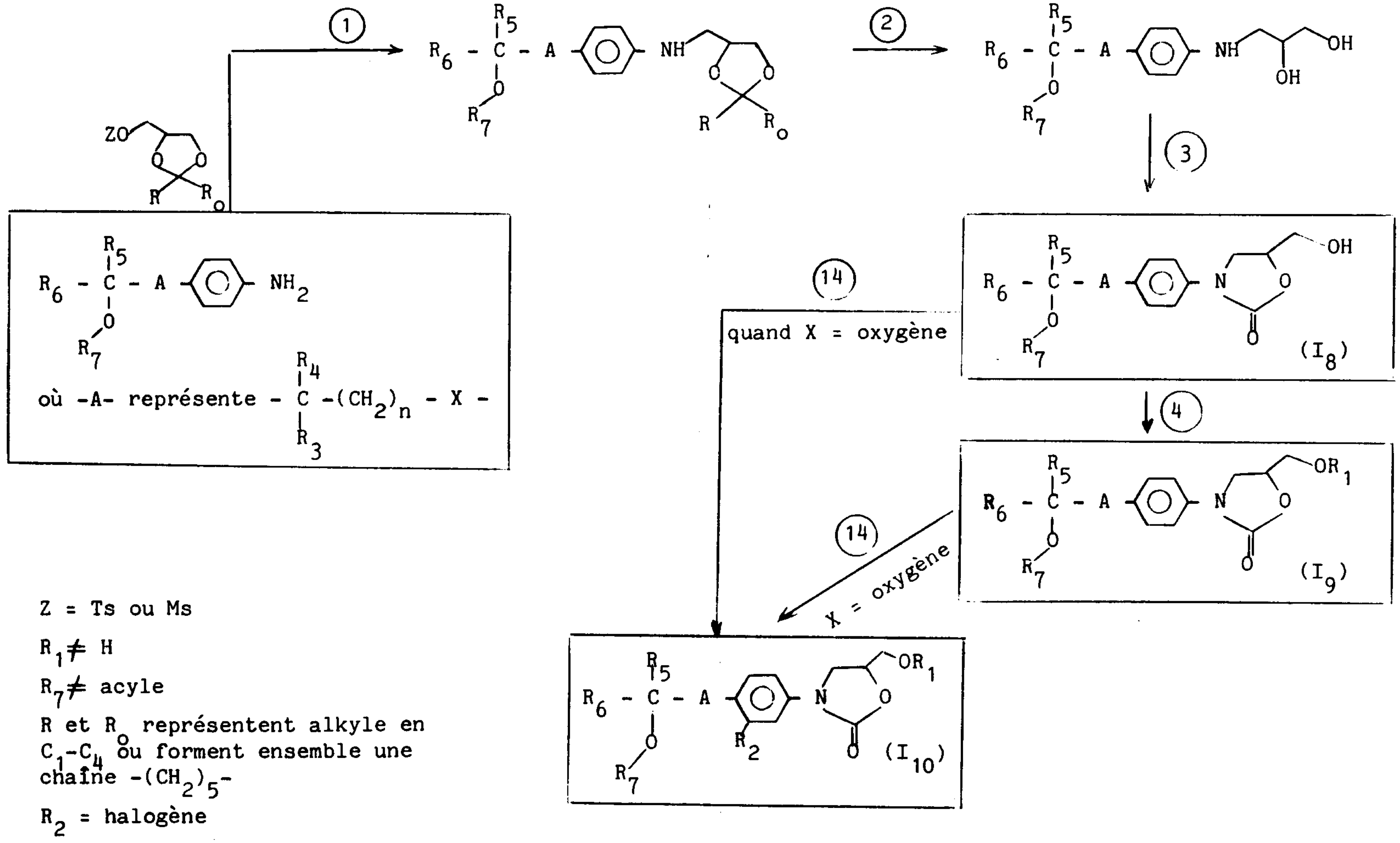


($I_8$) ($I_9$) ($I_{10}$)

Z = Ts ou Ms

$R_1 \neq H$

$R_7 \neq$ acyle

R et $R_o$ représentent alkyle en $C_1$-$C_4$ ou forment ensemble une chaîne $-(CH_2)_5-$

$R_2$ = halogène

Schéma 10

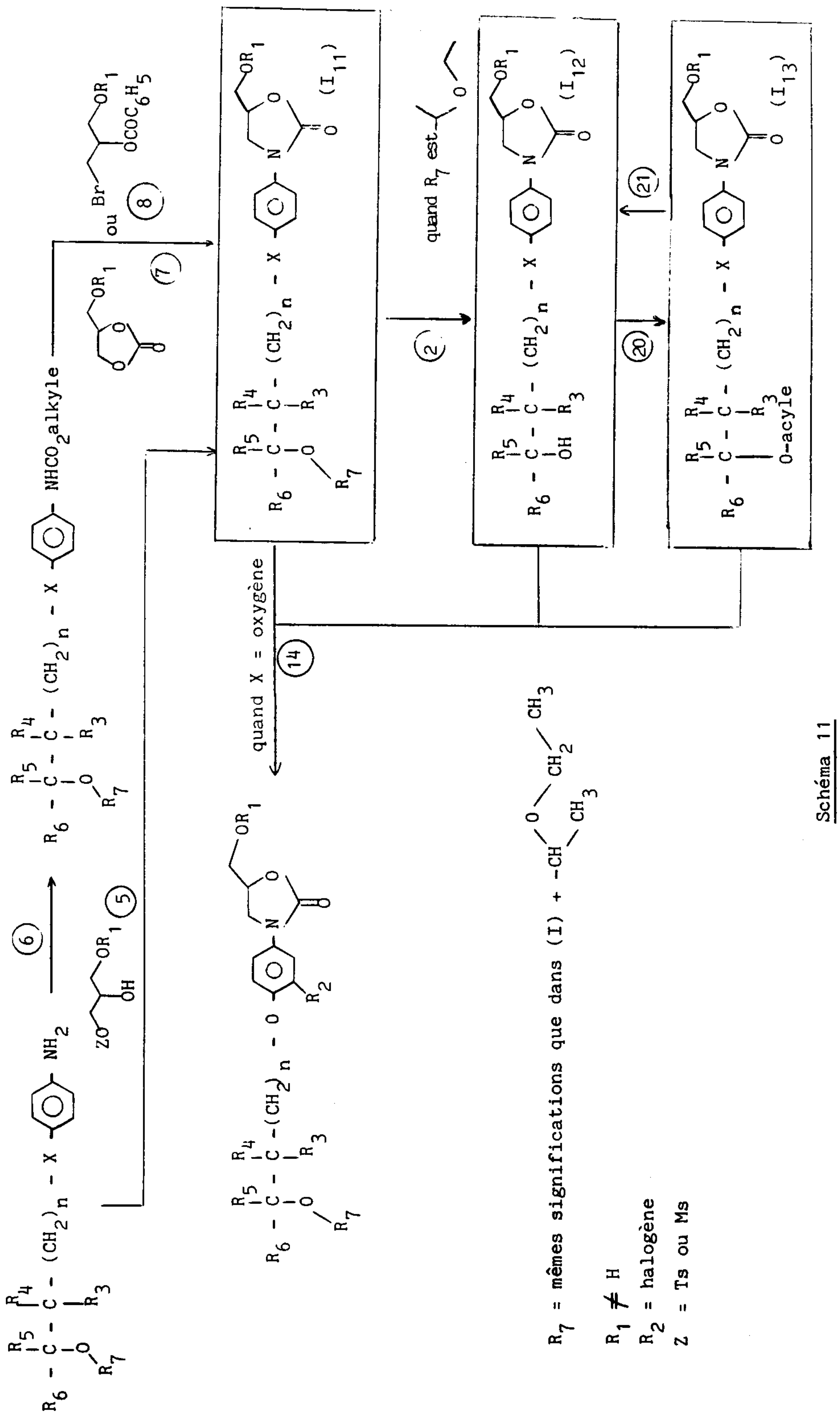
ou
quand X = oxygène
quand $R_7$ est
$R_7$ = mêmes significations que dans (I) +
$R_1$ ≠ H
$R_2$ = halogène
Z = Ts ou Ms

Schéma 11

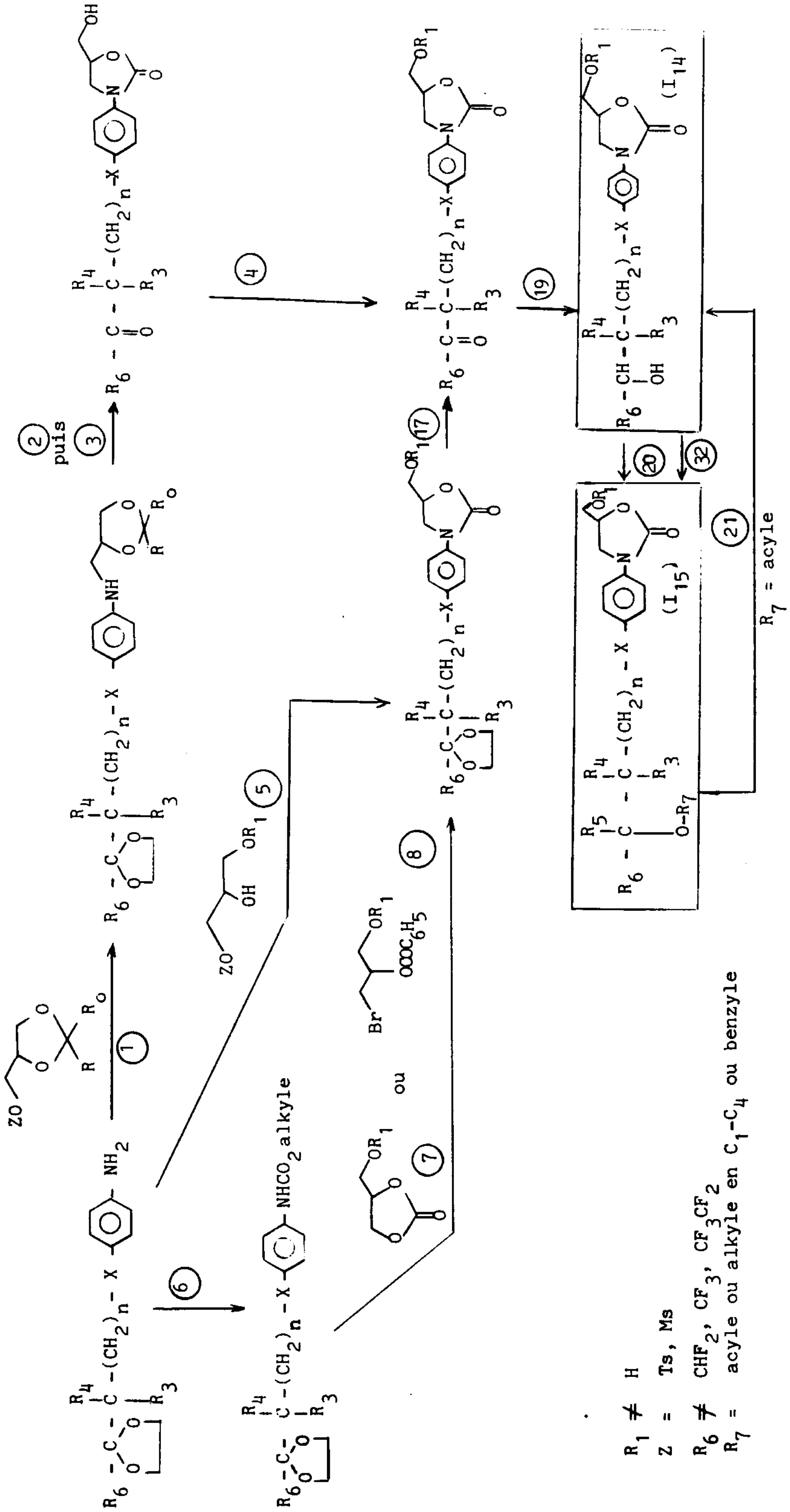


$R_1 \neq H$
$Z$ = Ts, Ms
$R_6 \neq CHF_2$, $CF_3$, $CF_3CF_2$
$R_7$ = acyle ou alkyle en $C_1$-$C_4$ ou benzyle

Schéma 12

Par ailleurs, les composés de formule :

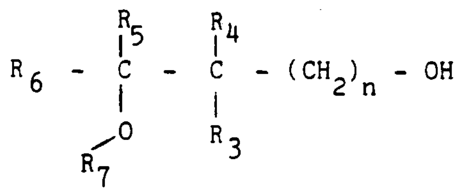

(schéma 6 ),

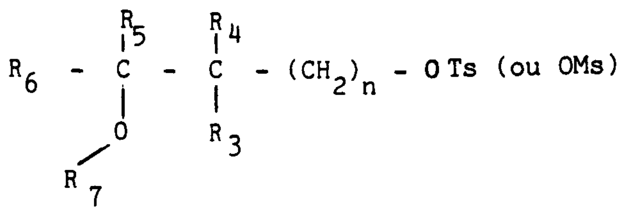

(schéma 3),

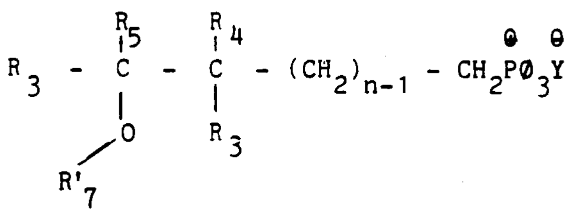

(schémas 4 et 7)

sont obtenus conformément au schéma 13 ci-après.

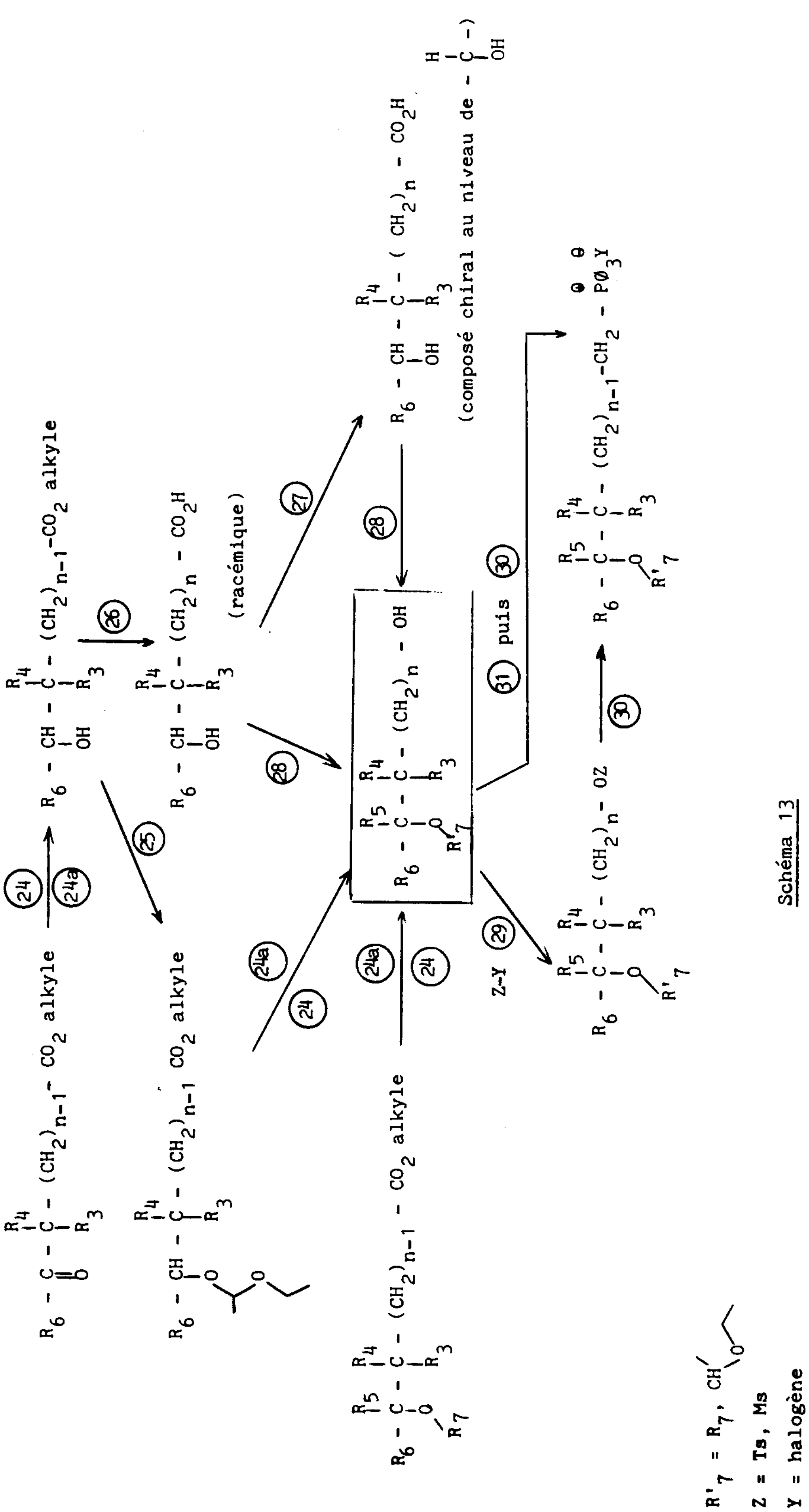


Schéma 13

D'autre part, les composés de formule :

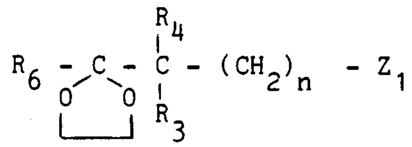 (schéma 5),

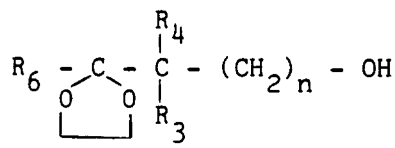 (schéma 8),

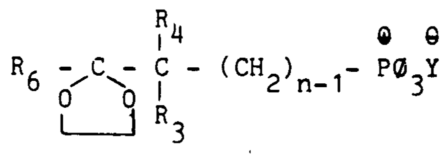 (schéma 9),

sont obtenue conformément au schéma 14 ci-après.

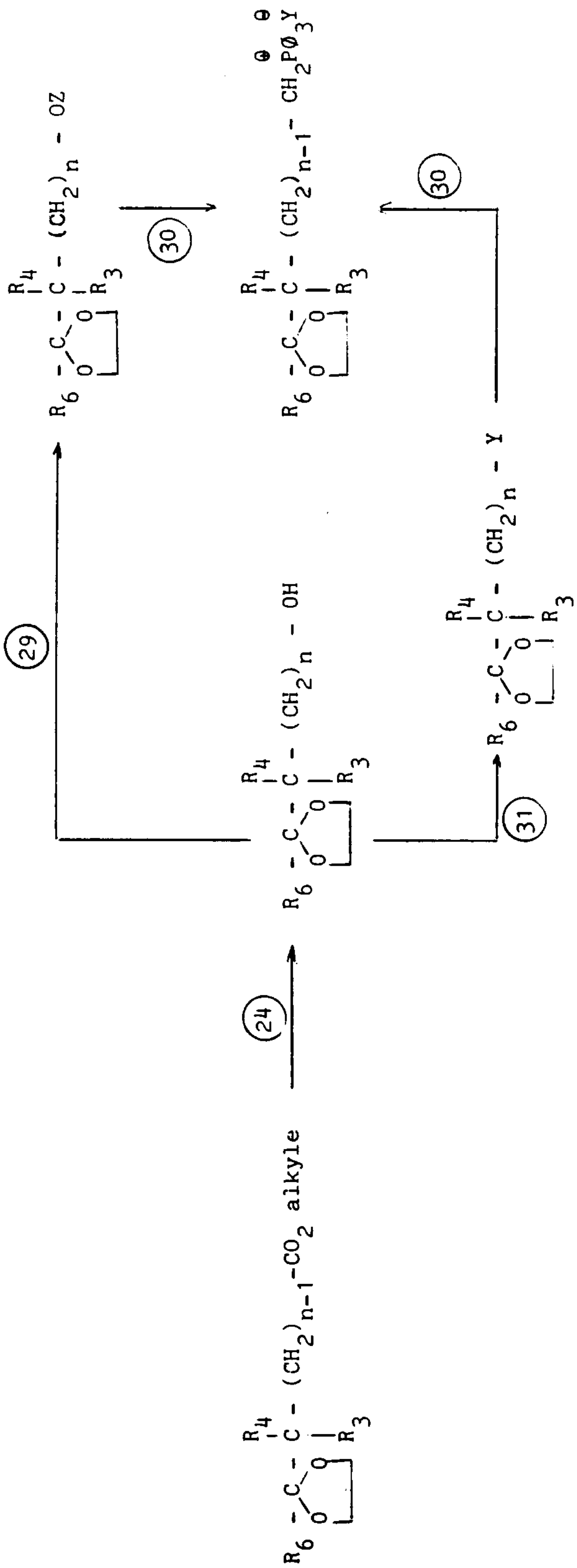


Z = Ts, Ms
Y = halogène

Schéma 14

Les indices ① à ㉜ apparaissant dans les schémas ci-dessus, ont les significations suivantes :

① Condensation soit dans un solvant aprotique anhydre comme le toluène, par chauffage, avec ou sans catalyseur comme le bromure d'hexacedyl tributyl phosphonium ou un halogénure d'ammonium quaternaire tel que le bromure de benzyl triéthylammonium, soit sans solvant en présence de triéthylamine

entre 130-150° C.

② Hydrolyse par un acide aqueux notamment l'acide chlorhydrique 6N en présence d'un solvant organique comme la méthyléthylcétone ou l'éther éthylique.

③ Condensation avec un carbonate d'alkyle en $C_1$-$C_4$ notamment le carbonate de diéthyle dans un solvant anhydre comme le toluène en présence d'alcoolate de métal alcalin comme le méthylate de sodium.

④ Alcoylation par un halogénure (bromure ou chlorure) d'alkyle en $C_1$-$C_4$ dans les conditions de transfert de phase notamment soude-chlorure de méthylène ou toluène, en présence d'un ammonium quaternaire comme le bromure ou l'hydrogénosulfate de tétrabutylammonium.

⑤ Condensation en présence de phosgène et d'une base notamment la diméthylaniline dans un solvant organique comme le chlorure de méthylène ou le dichloroéthane ; puis cyclisation par chauffage dans un solvant organique notamment alcoolique comme l'éthanol en présence d'une base notamment la potasse.

⑥ Condensation avec un chloroformiate d' alkyle, comme le chloroformiate d'éthyle, en présence d'une base notamment $NaHCO_3$ dans un mélange solvant eau-THF, à température ambiante.

⑦ Condensation à chaud (vers 150° C) en présence d'une base comme $K_2CO_3$. La réaction conserve la stéréochimie.

(7a) Condensation dans le toluène en présence de LiBr et de $nBu_3PO$.

⑧ Condensation en présence d'une base notamment NaH dans un solvant aprotique comme le THF à 55° C-60° C.

⑨ Débenzylation dans un solvant alcoolique, comme le méthanol ou l'éthanol en présence d'hydrogène et d'un catalyseur notamment le palladium sur carbone à 10 % humidifié ou non.

⑩ O.silylation de l'alcool dans un solvant organique aprotique comme le THF en présence d'une base notamment l'imidazole, et de terbutyldiméthylchlorosilane.

(10a) Réduction du dérivé nitré, par la poudre de fer en présence de chlorure d'ammonium.

⑪ Hydrolyse dans un solvant organique notamment le THF en présence de fluorure, notamment de tétrabutylammonium.

⑫ Oxydation en présence de chlorure d'oxalyle, de DMSO et d'une base, notamment, la triéthylamine dans un solvant organique aprotique comme le chlorure de méthylène.

⑬ O.alcoylation dans un solvant organique aprotique anhydre comme la méthyléthylcétone ou le DMF et en présence d'une base notamment $K_2CO_3$,

ou

O.alcoylation dans un solvant organique aprotique comme le DMF et/ou le THF et en présence d'un hydrure de métal alcalin comme l'hydrure de sodium.

⑭ Halogénation par action d'un halogène dans l'acide acétique.

⑮ Condensation en présence d'une base notamment $K_2CO_3$ et de formamide dans un solvant aprotique notamment le dioxanne, de préférence au reflux,

ou

Condensation en présence de LDA (lithium diisopropylamide) dans un mélange de solvants notamment DMSO/THF.

⑯ Hydrogénation sous pression atmosphérique d'hydrogène dans un solvant organique notamment l'acétate d'éthyle en présence d'un catalyseur comme le palladium sur carbone à 10 % humidifié ou non, ou $PtO_2$,

ou

Hydrogénation sous pression d'hydrogène notamment sous 5 atmosphères, en présence de palladium sur carbone à 10 % humidifié ou non, ou $PtO_2$, dans un solvant alcoolique, notamment l'éthanol,

ou

Hydrogénation sous pression d'hydrogène notamment sous 9 atmosphères, en présence de palladium sur carbone à 10 % humidifié ou non dans un solvant alcoolique, notamment l'éthanol.

⑰ Hydrolyse en présence de silice et de chlorure de fer hydraté dans un solvant organique notamment l'acétone ou la méthyléthylcétone.

⑱ Acétalisation en présence de silice greffée aminopropyle sous forme de chlorhydrate, par l'éthylène glycol, dans un solvant aprotique notamment le chlorure de méthylène et avec ou sans orthoformiate d'éthyle,

ou

Acétalisation par l'éthylène glycol au reflux d'un solvant aprotique notamment le toluène, en présence de l'acide paratoluène sulfonique, en éliminant l'eau formée.

⑲ Réduction par un borohydrure alcalin (Na par exemple) dans un solvant alcoolique (EtOH par exemple).

⑳ Acylation, par exemple :

- Action de l'anhydride d'un acide alkyl-COOH, dans un solvant organique comme $CH_2Cl_2$ en présence d'une base comme la pyridine, ou
- Action d'un acide alkyl-COOH en présence de triphénylphosphine et d'azodicarboxylate d'éthyle, dans un solvant organique comme le THF (inversion de la stéréochimie).

㉑ Hydrolyse basique dans un solvant organique notamment EtOH ou MeOH, avec une base comme NaOH ou KOH.

㉒ O.alcoylation dans un solvant organique aprotique notamment le DMF ou THF en présence d'hydrure de métal alcalin notamment le sodium.

㉓ Réduction du dérivé nitré par la poudre de fer en présence de chlorure d'ammonium.

㉔ Réduction dans un solvant organique aprotique comme le diméthoxyéthane ou $CH_2Cl_2$ en présence de borohydrure de sodium ou de lithium.

(24a) Réduction par $AlLiH_4$ dans un solvant aprotique comme le THF.

㉕ O.alcoylation par l'éthyl vinyl éther en présence de quantité catalytique de $POCl_3$, dans un solvant aprotique comme $CH_2Cl_2$.

㉖ Saponification par une base alcaline comme NaOH dans un solvant alcoolique comme EtOH.

㉗ Formation d'un sel chiral avec l'$\alpha$-méthylbenzylamine (R) ou (S), puis séparation des diastéréoisomères formés, puis libération de l'acide chiral par un acide notamment HCl.

㉘ Réduction par un borohydrure de métal alcalin (Na par exemple), en présence d'étherate de $BF_3$ dans un solvant aprotique, notamment THF, ou par l'hydrure de bis (méthoxyéthoxy) d'Al et de Na [-$(CH_3OCH_2CH_2O)_2AlH_2Na$] dans le toluène.

㉙ Condensation dans un solvant organique notamment la pyridine, THF ou $CH_2Cl_2$ en présence d'une base notamment la diméthylamino-4 pyridine ou $Et_3N$.

㉚ Conformément à Helv. Chim. Acta 59, 755 (1976).

㉛ Conformément à Can. J. Chem. 1968, 46, 86.

㉜ O.alcoylation par un halogénure ou sulfate d'alkyle en $C_1$-$C_4$, ou un halogénure de benzyle, de préférence soit dans un solvant organique aprotique tel que le DMF, et en présence d'un hydrure métallique notamment NaH, soit dans des conditions de transfert de phase en présence d'un catalyseur comme le bromure de tertiobutylammonium dans un mélange toluène/NaOH aqueux à 50 %.

Les préparations suivantes sont données à titre d'exemples pour illustrer l'invention :

Exemple 1 :

mélange racémique de diastéreoisomères d'[(hydroxy-3 butoxy)-4 phényl]-3 méthoxyméthyl-5 oxazolidinone-2 (numéro de code MD 370047).

A une solution de 27,5 g (0,112 mole) de tosyloxy-1 butanol-3 dans 250 ml de méthyléthylcétone, on ajoute 28,2 g (0,2 mole) de $K_2CO_3$ et de 22,8 g (0,102 mole) d'(hydroxy-4 phényl)-3 méthoxyméthyl-5 oxazolidinone-2 (numéro de code MD 780232). On chauffe au reflux pendant 4 h 30. Après filtration et concentration, le résidu est repris dans 200 ml de $CH_2Cl_2$, la phase organique est lavée à l'eau saturée de NaCl, séchée sur $Na_2SO_4$ et concentrée. Après purification par flash chromatographique (silice, éluant : $CH_2Cl_2$ : 98 ; $CH_3OH$ : 2), on obtient le produit attendu avec un rendement de 70 %, F : 58° C ; RMN$^1$H ($CDCl_3$) $\delta$ppm : 1,2 (3H) ; 1,8 (2H) ; 2,5 (1H éch.) ; 3,4 (3H) ; 3,6 (2H) ; 3,7-4,2 (5H) ; 4,7 (1H) ; 6,8 (2H) ; 7,4 (2H) ; IR (KBr)$\nu$cm$^{-1}$ : 3400, 1750, 1730.

De la même manière mais à partir de trifluoro-4,4,4 tosyloxy-1 butanol-3 et de l'(hydroxy-4)-phényl-3 méthoxyméthyl-5 oxazolidinone-2 (numéro de code MD 780232), on a obtenu le mélange des diastéréoisomères racémiques [(trifluoro-4,4,4 hydroxy-3 butoxy)-4 phényl]-3 méthoxyméthyl-5 oxazolidinone-2 :
(numéro de code MD 370167) : F : 89°C,RMN$^1$H ($CDCl_3$)$\delta$ppm : 2,05 (2H) ; 3,4 (3H) ; 3,5 (1H, éch.) ; 3,6 (2H) ; 3,7-4,3 (5H) ; 4,7 (1H) ; 6,8 (2H), 7,3 (2H). IR (KBr)$\nu$cm$^{-1}$ : 3400, 1750, 1785.

Exemple 2 :

[(hydroxy-3 (R) butoxy)-4 phényl]-3 méthoxyméthyl-5 (R) oxazolidinone-2 (numéro de code 370120)

Stade 1 :

(benzyloxy-4 phenyl)amino-3 propane diol-1,2 (R) : (numéro de code MD 200418).

Dans un autoclave, on ajoute 1,5 kg de benzyloxy-4 aniline (7,564 moles), 0,2014 de mésylate du dioxa-1,4 spiro [4,5] décane méthanol-2 (S) (8,048 moles) et 1,88 l de triéthylamine (13,5 moles). On chauffe les réactifs à 140 °C pendant 30 mn. Puis le milieu réactionnel est repris dans 7 l de méthyléthylcétone. La solution est lavée à l'eau et est utilisée pour l'étape suivante. A cette solution on ajoute 1,2 l d'acide chlorhydrique à 36 %. On chauffe le milieu réactionnel à 55 °C pendant 30 mn et on refroidit à 20 °C. On ajoute de la lessive de soude jusqu'à pH 9. La solution organique est lavée à l'eau et concentrée. Le produit est obtenu avec un rendement de 90 % ; F : 102 °C ; $[\alpha]_D^{20}$ = + 12,7 ° (C = 1, $CH_3OH$).

Stade 2 :

(benzyloxy-4 phényl)-3 méthoxyméthyl-5 (R) oxazolidinone-2 (numéro de code MD 200404).

a) A une suspension de 13 g (0,0475 mole) du composé MD 200418 dans 100 ml de toluène, on ajoute au reflux 6,2 ml (0,052 mole) de carbonate d'éthyle et 2 ml de méthylate de sodium 1M dans le méthanol. On distille jusqu'à ce que le reflux atteigne le point d'ébullition du toluène. Après refroidissement, on ajoute du $CH_2Cl_2$ et la solution organique est lavée à l'eau et séchée sur $Na_2SO_4$. Après concentration, on obtient 14 g de (benzyloxy-4 phényl)-3 hydroxyméthyl-5 (R) oxazolidinone-2 : (numéro de code MD 220201) : F : 157 °C ; $[\alpha]_D^{20}$ : - 41 ° (C = 1, $CH_2Cl_2$).

b) A 15 g (0,05 mole) du produit précédent (MD 220201), on ajoute 100 ml de toluène et 18,9 g de sulfate de méthyle, 1,8 g de tétrabutyl ammonium hydrogénosulfate, 10 ml d'eau et 10 g de NaOH. On chauffe les réactifs $\frac{1}{2}$ h. Le milieu réactionnel est extrait à l'éther isopropylique et le produit attendu est obtenu avec un rendement de 83 % ; F : 101 °C ; $[\alpha]_D^{20}$ : - 41,5 °(C = 1, $CH_2Cl_2$). Par un mode opératoire identique mais à partir des réactifs appropriés on a obtenu le (benzyloxy-4 phényl)-3 méthoxyméthyl-5 (S) oxazolidinone-2 (numéro de code 340190), F : 101 °C , $[\alpha]_D^{20}$ : + 41,9 ° (C = 1, $CH_2Cl_2$) ; ainsi que (benzyloxy-4 phényl)-3 éthoxyméthyl-5 (R) oxazolidinone-2 (numéro de code MD 230242), F : 78 ° C, $[\alpha]_D^{20}$ : - 35,9 ° (C = 1, $CH_2Cl_2$) ; IR (KBr) $\nu cm^{-1}$ : 1750, 1735.

Stade 3 :

(Hydroxy-4 phényl)-3 méthoxyméthyl-5 (R) oxazolidinone-2 (numéro de code MD 200405).

A une solution de 13 g (0,047 mole) de composé MD 200404 dans 80 ml d'éthanol et 40 ml de $CH_2Cl_2$ en présence de 2,6 g de Pd/C à 10 % humidifié à 50 %, on fait passer un courant d'hydrogène sous pression normale.

Lorsque la réaction est complète, la solution est filtrée et concentrée. Le produit attendu est obtenu avec un rendement de 100 %. F : 112 °C ; $[\alpha]_D^{20}$ : - 67 ° (C = 1, $CH_3OH$) ; IR (KBr) $\nu cm^{-1}$ : 3260, 1730.

Par un procédé identique mais à partir des réactifs correspondants, on obtient le dérivé (hydroxy-4 phényl)-3 méthoxyméthyl-5 (S) oxazolidinone-2 :

(numéro de code MD 200717) : F ; 114 °C, $[\alpha]_D^{20}$ : + 66 ° (C = 1, $CH_3OH$); ainsi que le dérivé (hydroxy-4 phényl)-3 éthoxyméthyl-5 (R) oxazolidinone-2 (numéro de code MD 230243), F : 92 ° C ; $[\alpha]_D^{20}$ : - 58,9 ° (C = 1, $CH_3OH$), IR (KBr) $\nu cm^{-1}$ : 3300, 1770.

Stade 4 :

[(hydroxy-3 (R) butoxy)-4 phényl]-3 méthoxyméthyl-5 (R) oxazolidinone-2 : (numéro de code MD 370120).

A 100 ml de méthyléthylcétone, on ajoute 14,6 g (0,059 mole) de tosyloxy-1 butanol-3 (R) (Helv. Chim. Acta, 67, 89, 1984), 14,8 g (0,1 mole) de $K_2CO_3$ et 18 g (0,053 mole) de composé MD 200405. On chauffe au reflux 5 h, après filtration, le milieu réactionnel est concentré, repris dans l'acétate d'éthyle, lavé à l'eau, séché sur $Na_2SO_4$ et concentré. Le produit est purifié par chromatographie flash (silice, éluant : $CH_2Cl_2$ : 95 ; $CH_3OH$ : 5) ; F : 76 °C $[\alpha]_D^{20}$ : - 50,7 ° (C = 1, $CH_2Cl_2$). Par un mode opératoire identique en faisant réagir le tosylate-1-butanol-3 (R) avec le MD 200717 on obtient l'[(hydroxy-3 (R) butoxy)-4 phényl]-3 méthoxyméthyl-5 (S) oxazolidinone-2 (numéro de code MD 370123) : F : 44 °C,$[\alpha]_D^{20}$ : + 33 ° (C = 1,

$CH_2Cl_2$).
De même, en faisant réagir le tosyloxy-1 butanol-3 (S) (J. Org. Chem. 47, 3850, 1982) avec le composé MD 200405 on obtient l'[(hydroxy-3 (S) butoxy)-4 phényl]-3 méthoxyméthyl-5 (R) oxazolidinone-2 (numéro de code MD 370122), et avec le composé MD 200717 on obtient l'[(hydroxy-3 (S) butoxy)-4 phényl]-3 méthoxyméthyl-5 (S) oxazolidinone-2 (numéro de code MD 370121).

Exemple 3 :

[(hydroxy-3(S) butoxy)-4 phényl]-3 méthoxyméthyl-5 (S) oxazolidinone-2 (numéro de code MD 370121).

Stade 1 :

[(Acétoxy-3 (S) butoxy)-4 phényl]-3 méthoxyméthyl-5 (S) oxazolidinone-2 (numéro de code MD 370162) : on dissout 6,5 g ($2,2.10^{-2}$ mole) du composé de nu-numéro de code 370123 (ex. 2, stade 4) et 17,3 g ($6,6.10^{-2}$ mole) de triphénylphosphine dans 65 ml de THF refroidi à 0 °C, on ajoute goutte à goutte, une solution de diéthylazodicarboxylate d'éthyle dans 20 ml de THF en 20 mn, 7,42 ml d'acide acétique dans 20 ml de THF. On laisse en agitation 2 heures. Après concentration, le produit est purifié par chromatographie flash (silice, éluant : AcOEt : 50 ; Heptane : 50) ; F < 50 °C; $[\alpha]_D^{20}$ = + 52,8 ° (C = 1, $CH_2Cl_2$) ; IR (film $\nu cm^{-1}$) : 1760, 1740. RMN $^1H$ ($CDCl_3$)δppm : 1,3 (3H) ; 2 (5H) ; 3,4 (3H) ; 3,6 (2H) ; 3,7-4,2 (4H) ; 4,7 (1H) ; 5,1 (1H) ; 6,8 (2H) ; 7,2 (2H).
De la même manière on obtient les composés à partir des matières premières correspondantes : [(Acétoxy-3 (S) butoxy)-4 phényl]-3 méthoxyméthyl-5 (R) oxazolidinone-2 (numéro de code MD 370161) : $[\alpha]_D^{20}$ = -18,6 ° (C = 1, $CH_2Cl_2$).
[(Acétoxy-3 (R) butoxy)-4 phényl]-3 méthoxyméthyl-5 (R) oxazolidinone-2 (numéro de code MD 370220) : $^1H$RMN ($CDCl_3$)δppm : 1,3 (3H) ; 2 (5H) ; 3,4 (3H) ; 3,6 (2H) ; 3,7-4,2 (4H) ; 4,7 (1H) ; 5,1 (1H) ; 6,8 (2H) ; 7,4 (2H) ; IR (KBr, $\nu cm^{-1}$) : 1730, 1740 ; $[\alpha]_D^{20}$ : - 53,1 ° (C = 1, $CH_2Cl_2$) ; F = 43 °C. [(Acétoxy-3 (R) butoxy)-4 phényl]-3 méthoxyméthyl-5 (S) oxazolidinone-2 (numéro de code MD 370219) : RMN$^1H$ ($CDCl_3$)-δppm : 1,3 (3H) ; 2 (5H) ; 3,4 (3H) ; 3,6 (2H) ; 3,7-4,2 (4H) ; 4,7 (1H) ; 5,1 (1H) ; 6,8 (2H) ; 7,4 (2H) ; $[\alpha]_D^{20}$ = + 19,3 ° (C = 1, $CH_2Cl_2$) ; huile.
[(acétoxy-3 (R) pentyl)-4 phényl]-3 méthoxyméthyl-5 (R) oxazolidinone-2 (numero de code MD 230323) : RMN$^1H$ ($CDCl_3$) δppm : 1,2 (3H) ; 1,6 (4H) ; 2 (3H) ; 2,6 (2H) ; 3,4 (3H) ; 3,6 (2H) ; 3,7-4,2 (2H) ; 4,8 (2H) ; 7,1 (2H) ; 7,4 (2H) ; IR (microcellule $\nu cm^{-1}$) : 1760 - 1730 ; $[\alpha]_D^{20}$ : - 31,5 ° (C = 1, $CH_2Cl_2$).

Stade 2 :

[(hydroxy-3 (S) butoxy)-4 phényl]-3 méthoxyméthyl-5 (S) oxazolidinone-2 (numéro de code MD 370121) : on dissout 6,1 g du composé de numéro de code MD 370162 dans 63 $cm^3$ de $CH_3OH$, on ajoute en 20 mn, 18 ml de soude 2N et on laisse sous agitation 3 heures, puis, on dilue le milieu réactionnel avec du chlorure de méthylène qui est lavé à l'eau, séché sur $Na_2SO_4$ et concentré, le produit attendu est obtenu avec un rendement de 83 % ; F = 76 °C ; $[\alpha]_D^{20}$ : + 50,4 ° (C = 1 ; $CH_2Cl_2$).
De la même manière mais à partir du composé de numéro de code MD 370161 on obtient le dérivé [-(hydroxy-3 (S) butoxy)-4 phényl]-4 méthoxyméthyl-5 (R) oxazolidinone-2 (numéro de code MD 370122) avec un rendement de 80 % ;
$[\alpha]_D^{20}$ = - 33,4 ° (C = 1, $CH_2Cl_2$) ; RMN$^1H$ ($CDCl_3$)δppm ; 1,2 (3H) ; 1,8 (2H);2,2 (1H éch.) ; 3,4 (3H) ; 3,6 (2H) ; 3,7-4,2 (5H) ; 4,7 (1H) ; 6,8 (2H) ; 7,4 (2H). RMN$^{13}C$ ($CDCl_3$)δppm : Cq : 155,6 ; 154,9 ; 131,6 ; CH : 120,2, 115, 71,3, 65,6 $CH_2$ : 72,7 ; 65,9 ; 47,6 ; 38,2 ; $CH_3$ : 59,6 ; 23,6. IR (KBr, $\nu cm^{-1}$) : 3380-3400 , 1755, 1730. F = 49 °C.
De même, à partir du composé MD 230323, on obtient le dérivé [(hydroxy-4 (R) pentyl-4 phényl]-3 méthoxyméthyl-5 (R) oxazolidinone (MD 230238) ; F : 53 ° C ; $[\alpha]_D^{20}$ : - 35,9 ° (C = 1, $CH_2Cl_2$) ; IR (KBr) $\nu cm^{-1}$ : 3400, 1740.

Exemple 4 :

[(hydroxy-3 (S) butoxy)-4 phényl]-3 méthoxyméthyl-5 (R) oxazolidinone-2 (numéro de code MD 370122)

Stade 1 :

(éthoxy-1 éthoxy)-2 butanoate d'éthyle (S) (numéro de code MD 370365).

A une solution de 15 g (0,113 mole) d'hydroxy-2 butanoate d'éthyle (S) dans 150 ml de $CH_2Cl_2$ refroidie à 0 ° C sous argon, on ajoute 16,3 g (0,227 mole) d'éthylvinyléther puis, 3 gouttes de $POCl_3$. On laisse remonter la température à 25 ° C et on ajoute une solution de bicarbonate de sodium. La solution organique est séchée sur $Na_2SO_4$ et concentrée. Le produit est purifié par chromatographie flash (silice, éluant : éther de pétrole : 90 ; éther isopropylique : 10) avec un rendement de 48 %. $[\alpha]_D^{20}$ = + 10,3 ° (C = 1, $CHCl_3$).

Stade 2 :

(éthoxy-1 éthoxy)-3 (S) butanol-1 (numéro de code 370366)

A une suspension de 1,36 g (0,0625 mole) de borohydrure de lithium dans 50 ml de DME (diméthoxy éthane) on ajoute 25,48 g (0,125 mole) du composé de numéro de code 370365 en solution dans le DME. Le milieu réactionnel est chauffé au reflux. Après 1 heure, on ajoute 0,5 équivalent de $LiBH_4$ et on laisse en agitation pendant une nuit. Après avoir ajouté de l'eau, le milieu réactionnel est extrait au $CH_2Cl_2$. La phase organique est lavée à l'eau saturée de NaCl, le produit attendu est isolé avec un rendement de 58 % après chromatographie flash (silice, acétate d'éthyle : 50 ; heptane : 50):$[\alpha]_D^{20}$ = + 57,6 ° (C = 1, $CHCl_3$).

Stade 3 :

[(éthoxy-1 éthoxy)-3 (S) butoxy]-4 phényl-3 méthoxyméthyl-5 (R) oxazolidinone-2 (numéro de code MD 370367).

A une solution de 6,6 g du composé MD 370366 (0,04 mole) dans 70 ml de THF, on ajoute à 0 ° C, 4,5 g (0,044 mole) de triéthylamine et 5,1 g (0,044 mole) de chlorure de mésyle. On laisse en agitation pendant 10 mn et le milieu réactionnel est versé sur l'eau. La phase aqueuse est extraite à l'ether isopropylique. La phase étherée est lavée à l'eau salée puis séchée sur $Na_2SO_4$ et concentrée. Le produit obtenu est utilisé sans autre purification pour l'étape suivante. A une solution de 7,6 g (0,034 mole) de composé MD 200405 (ex. 2, stade 3) dans 20 ml de diméthylformamide (DMF) et 200 ml de butanone, on ajoute 11,7 g (0,085 mole) de $K_2CO_3$, une petite quantité de KI et 9 g (0,0374 mole) du composé précédent. Le milieu réactionnel est chauffé 6 heures au reflux, puis versé sur une solution 0,5 N de soude.
Après extraction à l'acétate d'éthyle, la phase organique est lavée à l'eau saturée de sel, séchée sur $Na_2SO_4$ et concentrée. Le produit est obtenu avec un rendement de 92 % après chromatographie flash (silice, éluant : Heptane : 50 ; AcOEt : 50). $[\alpha]_D^{20}$ = - 12,1 ° (C = 1, $CH_2Cl_2$).

Stade 4 :

[(hydroxy-3 (S) butoxy)-4 phényl]-3 méthoxyméthyl-5 (R) oxazolidinone-2 (numéro de code MD 370122).

A une solution de 236 g (0,642 mole) du composé MD 370367 dans 250 ml de THF on ajoute à température ambiante 250 ml d'HCl1N. Après 15 mn d'agitation on ajoute de l'ammoniaque 10,8 N jusqu'à pH : 10. La phase aqueuse est extraite à l'acétate d'éthyle et la solution organique est lavée à l'eau salée. Après concentration, le produit obtenu est purifié par chromatographie flash (éluant : acétate d'éthyle : 33 ; éther isopropylique : 66). Le produit à les mêmes caractéristiques que celui obtenu à l'exemple 3.

Exemple 4a:

mélange racémique de diastéréoisomères d'acétoxy-3 butoxy-4 phényl-3 méthoxyméthyl-5 oxazolidinone-2 (numéro de code MD 370057).

Méthode 1 :

On laisse en agitation dans 1 ml de $CH_2Cl_2$, 100 mg du composé MD 370047 (décrit dans l'exemple 1) et 29 mg de chlorure d'acétyle en présence de 51 mg de triéthylamine, puis on ajoute du $CH_2Cl_2$, la solution organique est lavée à l'eau, séchée, concentrée. Le produit attendu est purifié par flash chromatographie (silice, éluant : Heptane : 40 ; Acétate d'éthyle : 60) avec un rendement de 33 %. RMN$^1$H ($CDCl_3$)-δppm : 1,3 (3H) ; 2 (5H) ; 3,4 (3H) ; 3,6 (2H) ; 3,7-4,2 (4H) ; 4,7 (1H) ; 5,1 (1H) ; 6,8 (2H) ; 7,4 (2H) ; IR (microcellule) νcm$^{-1}$ : 1740, 1760.

Méthode 2 :

A une solution refroidie à 0 °C de 6 g (0,02 mole) du composé MD 370047 dans 8,2 ml de pyridine, on ajoute 2,4 ml d'anhydride acétique et 3,5 ml de triéthylamine. On laisse en agitation 12 heures à température ambiante. Puis le milieu réactionnel est dilué avec $CH_2Cl_2$. La solution organique est lavée à l'eau jusqu'à pH neutre, séchée sur $Na_2SO_4$ et concentrée. Le produit attendu est purifié comme précédemment avec un rendement de 83 %.

Exemple 4b:

[(méthoxy-3 (S)-butoxy)-4 phényl]-3 méthoxyméthyl-5 (R) oxazolidinone-2 (numéro de code : MD 370375).

A une solution de 6 g (0,02 mole) du composé MD 370122 dans 60 ml de DMF, on ajoute 1,07 g de NaH à 50 % et on laisse à 40 °C pendant 30 mm, puis, on ajoute 1,8 ml d'iodure de méthyle (0,03 mole). Après 1 heure d'agitation, le milieu réactionnel est versé sur un mélange $H_2O$-$CH_2Cl_2$. La phase organique est séchée sur $Na_2SO_4$ et concentrée. Le produit est obtenu avec 63 % de rendement après chromatographie flash (silice, éluant : Acétate d'éthyle : 70 ; Heptane 30) : RMN$^1$H : ($CDCl_3$)δppm : 1,2 (3H) ; 1,9 (2H) ; 3,3 (3H) ; 3,4 (3H) ; 3,6 (2H) ; 3,7-4,2 (4H) ; 4,7 (1H) ; 6,9 (2H) ; 7,4 (2H) ; IR (microcellule) νcm$^{-1}$ : 1740-1760 ; $[\alpha]_D^{20}$ : - 18,7 ° (C = 1, $CH_2Cl_2$) ; huile.

De la même manière a été obtenu : [(méthoxy-3 (R) butoxy)-4 phényl]-3 méthoxyméthyl-5 (R) oxazolidinone-2 (numéro de code MD 370374) : F = 54 °C ; $[\alpha]_D^{20}$ = - 58,1 ° (C = 1, $CH_2Cl_2$) ; RMN$^1$H (δppm) $CDCl_3$ : 1,2 (3H) ; 1,9 (2H) ; 3,3 (3H) ; 3,4 (3H) ; 3,5-4,2 (7H) ; 4,7 (2H) ; 6,85 (2H).

Exemple 5 :

mélange de diastéréoisomères racémiques [(hydroxy-3 butoxy)-4 phényl]-3 hydroxyméthyl-5 oxazolidinone-2 (numéro de code MD 370210).

A une solution de 7,8 g du composé [(hydroxy-4) phényl]-3 hydroxyméthyl-5 oxazolidinone-2 (MD 760172) dans 75 ml de méthyléthylcétone, on ajoute 10,2 g (0,074 mole) de $K_2CO_3$, 0,1 g de KI et 10,84 g (0,044 mole) de tosylate d'hydroxy-3 butanol-1, on chauffe au reflux 12 heures. Après filtration le milieu réactionnel est concentré et le produit est obtenu après chromatographie (silice, éluant : $CH_2Cl_2$ : 92 ; $CH_3OH$ : 8) avec un rendement de 52 % , F = 95 °C ; RMN$^1$H ($DMSOd_6$)δppm : 1,2 (3H) ; 1,75 (2H) ; 3,5-4,2 (7H) ; 4,5 (1H éch.) ; 4,6 (1H) ; 5,2 (1H ech.) ; 6,9 (2H) ; 7,4 (2H).

De la même manière ont été obtenus à partir des réactifs correspondants :

[(hydroxy-3 (R) butoxy)-4 phényl]-3 hydroxyméthyl-5 (R) oxazolidinone-2 (numéro de code MD 370327) : RMN$^1$H ($DMSOd_6$)δppm : 1,1 (2H) ; 1,7 (2H) ; 3,5-4,2 (2H) ; 4,4 (1H éch.) ; 4,6 (1H) ; 5,2 (1H) ; 6,9 (2H) ; 7,4 (2H). IR (KBr) νcm$^{-1}$ : 3520, 3460 ; $[\alpha]_D^{20}$ : - 66,6 ° (C = 1, éthanol) ; F = 125 °C

[(hydroxy-3 (S) butoxy)-4 phényl]-3 hydroxyméthyl-5 (R) oxazolidinone-2 (numéro de code MD 370308) RMN$^1$H ($DMSOd_6$)δppm : 1,1 (2H) ; 1,7 (2H) ; 3,5-4,2 (7H) ; 4,5 (1H éch.) ; 4,6 (1H) ; 5,2 (1H éch.) ; 6,9 (2H) ; 7,4 (2H). IR (KBr) νcm$^{-1}$ : 3530, 3470, 1730. $[\alpha]_D^{20}$ : - 26,2 ° (C = 1, $CH_3OH$) ; F = 111 °C

[(benzyloxy-3 butoxy)-4 phényl]-3 hydroxyméthyl-5 oxazolidinone-2 (numéro de code MD 370380) : RMN$^1$H ($CDCl_3$)δppm : 1,2 (3H) ; 2 (2H) ; 3,4-4,3 (8H dont 1 éch.) ; 4,4-4,8 (3H) ; 6,8 (2H) ; 7,3 (5H) ; 7,4 (2H). IR (KBr) νcm$^{-1}$ : 3460, 1715. F = 78 °C.

[(benzyloxy-3 butoxy)-4 phényl]-3 hydroxyméthyl-5 (R) oxazolidinone-2 (numéro de code MD 370373) : RMN$^1$H ($CDCl_3$)δppm : 1,2 (3H) ; 2 (2H) ; 3,5-4,2 (8H dont 1 éch.) ; 4,4-4,8 (3H) ; 6,8 (2H) ; 7,3 (5H) ; 7,4 (2H) ; IR (KBr) νcm$^{-1}$ : 3460, 1715. $[\alpha]_D^{20}$ = - 33,2 ° (C = 1, $CH_2Cl_2$). F = 72 °C.

Mélange de diastéréoisomères de [(trifluoro-4,4,4 hydroxy-3 butoxy)-4 phényl]-3 hydroxyméthyl-5 (numéro de code MD 370262) : F = 130 °C, RMN$^1$H ($DMSOd_6$)δppm : 2 (2H) ; 3,5-4,2 (7H) ; 4,7 (1H) ; 5,2 (1H, éch.) ; 6,4 (1H, éch.) ; 6,9 (2H) ; 7,5 (2H) ; IR (KBr) νcm$^{-1}$ : 3300, 1720.

[(trifluoro-4,4,4 hydroxy-3 (R) butoxy)-4 phényl]-3 hydroxyméthyl-5 (R) oxazolidinone-2 (numéro de code

MD 230100) : RMN$^1$H ($DMSO_{d6}$)δppm : 2 (2H) ; 3,6-4,4 (7H) ; 4,7 (1H) ; 5,1 (1H éch.) ; 6,3 (1H éch.) ; 7 (2H) ; 7,5 (2H). IR (KBr) νcm$^{-1}$ : 3400, 3300, 1725. $[\alpha]_D^{20}$ = - 2,5° (C = 1, $CH_3OH$). F = 117°C.

Exemple 6 :

[(hydroxy-4 pentoxy)-4 phényl]-3 méthoxyméthyl-5 (R) oxazolidinone-2 de code MD 370509).

A une solution de 10,9 g (0,035 mole) du composé MD 370507 (ex. 9) dans 150 ml d'éthanol chauffée à 45°-50°C, on ajoute 1,347 g (0,035 mole) de borohydrure de sodium par petites portions. Puis le milieu réactionnel est concentré et le résidu est repris dans 200 ml d'eau. Après extraction au $CH_2Cl_2$, la phase organique est séchée sur $Na_2SO_4$, concentrée. Le produit est obtenu avec un rendement de 81 % après cristallisation dans un mélange acétate d'éthyle-hexane. F = 56°C; $[\alpha]_D^{20}$ = - 39,8° (C = 1, $CH_2Cl_2$) ; RMN$^1$H ($CDCl_3$)δppm : 1,2 (3H) ; 1,7 (4H) ; 2,2 (1H éch.) ; 3,4 (3H) ; 3,6 (2H) ; 3,7-4,2 (5H) ; 4,7 (1H) ; 6,9 (2H) ; 7,4 (2H). IR (KBr) νcm$^{-1}$ : 3350, 1745, 1730.

De la même manière ont été obtenus à partir des matières premières correspondantes :

[(hydroxy-3 butoxy)-4 phényl]-3 méthoxyméthyl-5-(R) oxazolidinone-2 (numéro de code MD 370284) : RMN$^1$H ($CDCl_3$)δppm : 1,2 (3H) ; 1,8 (2H) ; 2,6 (1H éch.) ; 3,4 (3H) ; 3,6 (2H) ; 3,7-4,2 (5H) ; 4,7 (1H) ; 6,8 (2H) ; 7,4 (2H). IR (KBr) νcm$^{-1}$ : 3400, 1745, 1730 ; $[\alpha]_D^{20}$ = - 41,5° (C = 1, $CH_2Cl_2$).

[(hydroxy-1 cyclopentyl-2 méthoxy)-4 phényl]-3 méthoxyméthyl-5 (R) oxazolidinone-2 (numéro de code MD 230201) ; F : 71°C ; $[\alpha]_D^{20}$ : - 40,3° (C = 1, $CH_2Cl_2$) ; IR (KBr) νcm$^{-1}$ : 3400-3300, 1755, 1730 ; RMN$^1$H ($CDCl_3$)δppm : 1,1-2,5 (7H) ; 2,8 (1H éch.) ; 3,4 (3H) ; 3,6 (2H) ; 4 (5H) ; 4,7 (1H) ; 6,9 (2H) ; 7,4 (2H).

Exemple 7 :

[(trifluoro-4,4,4 hydroxy-3 (R) butoxy)-4 phényl]-3 méthoxyméthyl-5 (R) oxazolidinone-2 (numéro de code MD 370503)

Stade 1 :

Trifluoro-4,4,4 hydroxy-3 butanoate d'éthyle (numéro de code MD 370270).

A une suspension de 91,2 g (2,4 mole) de borohydrure de sodium dans 6 l de $CH_2Cl_2$ refroidie à + 2°C on ajoute goutte à goutte 1473 g de trifluoro-4,4,4 acéto acétate d'éthyle en maintenant la température entre + 4 et + 7°C. Après l'introduction, on ajoute 100 ml d'éthanol absolu en maintenant la température à + 3°C. Le milieu réactionnel est lavé à l'eau bicarbonatée. La phase organique est concentrée et le produit est distillé à 78°C sous 10 mm Hg. F = 16°C.

Stade 2 :

Acide trifluoro-4,4,4 hydroxy-3 butanoïque (numéro de code MD 230096).

A 3 l d'éthanol absolu, on ajoute 228 g de NaOH. La solution éthanolique de soude obtenue est ajoutée à la solution du composé MD 370270 (1060 g, 5,7 mole) dans 1 l d'éthanol absolu en maintenant la température à 20°C. Après 3 heures de contact le sel de sodium est filtré et séché.

A une solution de 4,5 l d'acétate d'éthyle, 910 ml d'eau et 450 ml d'HCl 12N on ajoute le sel de sodium précédent et on laisse en agitation jusqu'à obtenir une solution.

Le milieu réactionnel est extrait à l'acétate d'éthyle et concentré.

Le produit est cristallisé dans l'heptane avec un rendement de 85,9 % : F = 78°C

Stade 3 :

Acide trifluoro-4,4,4 hydroxy-3 (R) butanoïque (numéro de code MD 230097).

A une solution de 7,8 l d'éthanol contenant 766,7 g (4,85 mole) d'acide racémique trifluoro-4,4,4 hydroxy-3 butanoïque, on ajoute 575,7 g d'α-méthyl-benzylamine (S). On chauffe le milieu réactionnel jusqu'à dissolution. Après une nuit à 20° C, on filtre le précipité et le sèche. Après recristallisations dans l'éthanol on obtient un pouvoir rotatoire constant $[\alpha]_D^{20}$ = - 8,8° et un point de fusion constant : F = 196°C. A un mélange de 200 ml d'eau, 150 ml d'HCl à 36 % et 1,2 l d'éther éthylique, on ajoute 434 g du produit précédent. La phase aqueuse est extraite à l'éther éthylique et les phases organiques sont séchées sur $Na_2SO_4$ et concentrées. Le produit est purifié par distillation sous 4 mm Hg à 110°C ; F = 40°C ; $[\alpha]_D^{20}$ : + 18,8° (C = 1, pyridine) ; Rdt = 66 %

| Analyse élémentaire: | | |
|---|---|---|
| cal % : | C : 30,39 ; | H : 3,19. |
| tr % : | C : 30,69 ; | H : 3,21 |

L'acide trifluoro-4,4,4 hydroxy-3 butanoïque de configuration (S) a été obtenu de la même façon mais en employant α-méthyl-benzylamine (R). F = 40 °C ; $[\alpha]_D^{20}$ : - 18,8 ° (C = 1, pyridine).

Stade 4 :

trifluoro-4,4,4 butanediol-1,3 (R) (numéro de code MD 230098).

A une suspension de 75,8 g (2 mole) de borohydrure de sodium dans 1,2 l de THF refroidie à - 2 °C on ajoute 243 g (1,537 mole) de MD 230097 en solution dans 300 ml de THF en 55 mm. Puis en 40 mm, 320 ml de $BF_3$ éthérate en maintenant la température entre 2 °C et 10 °C. Après avoir agité 35 mm, on ajoute en 35 mm de l'eau goutte à goutte, et on laisse remonter la température à 21 °C.
Après une nuit d'agitation, le milieu réactionnel est concentré et le résidu filtré. A l'huile refroidie, on ajoute 600 ml de soude à 10 %. La phase aqueuse est extraite à l'éther éthylique, puis, à l'acétate d'éthyle. Les phases organiques sont concentrées et le résidu est repris dans l'éther éthylique. La solution est acidifiée par HCl aqueux pour amener le pH à 1-2. Après filtration et concentration, le produit est purifié par chromatographie flash (silice, éluant : Heptane : 70 ; Acétate d'éthyle : 30) ; $Eb_4$ = 78 °C; $[\alpha]_D^{20}$ = + 28,9 ° (C = 1, $CHCl_3$). Par un même procédé, on obtient l'antipode optique (S) : $[\alpha]_D^{20}$ = - 28,9 ° (C = 1, $CHCl_3$).

Stade 5 :

Trifluoro-4,4,4 tosyloxy-1 butanol-3 (R) (numéro de code MD 230099).

A une solution de 120 g (0,833 mole) du composé MD 230098 dans 335 ml de pyridine, on ajoute 0,12 g de diméthylamino-4 pyridine et une solution de 198,4 g (1,041 mole) de chlorure de tosyle dans 200 ml de $CH_2Cl_2$. Après 1 heure 20 mn d'agitation, on ajoute 1,2 l de $CH_2Cl_2$ et 1,5 l d'eau. La phase organique est concentrée et le produit est purifié par chromatographie (silice, éluant : Heptane : 80 ; Acétate d'éthyle : 20) et est obtenu avec un rendement de 78 %. Il est utilisé directement pour l'étape suivante.

Stade 6 :

[(trifluoro-4,4,4 hydroxy-3 (R) butoxy)-4 phényl]-3 méthoxyméthyl-5 (R) oxazolidinone-2 (numéro de code MD 370503).

A une solution de 192,1 g (0,644 mole) du composé MD 200405 (ex. 2, stade 3) dans 400 ml de DMF on ajoute 161,5 g de $K_2CO_3$ et chauffe à 90 °C, puis la suspension du composé MD 230099 dans 200 ml de DMF. Après 1 heure, on refroidit le milieu réactionnel et ajoute 1,2 l de toluène par litre d'eau.
Après extraction de la phase aqueuse au toluène, les phases organiques sont concentrées à sec. Les produit est recristallisé dans un mélange éthanol-éther isopropylique. Rendement : 60,8 % ; F = 101 °C ; RMN$^1$H ($CDCl_3$)δppm : 2,05 (2H) ; 3,4 (3H) ; 3,6 (2H) ; 3,6-4,4 (6H dont 1 echangeable) ; 4,6 (1H) ; 6,8 (2H) ; 7,3 (2H).
RMN$^{13}$C ($DMSO_{d6}$) : Cq : 154,6 ; 154,4 ; 125,9 ($^1$JCF : 289,6 Hz) ; 131,8 ; CH : 119,8 ; 114,8 ; 71,3 ; 65,4 ($^2$JCF : 30,4 Hz) ; $CH_2$ : 72,5 ; 63,2 ; 46,6 ; 29,4 ; $CH_3$ : 58,7 ; IR (KBr) $\nu cm^{-1}$ : 3400, 1730, 1720 ; $[\alpha]_D^{20}$ = - 11,5 ° (C = 1, $CH_2Cl_2$).
De la même manière mais à partir du trifluoro-4,4,4 tosyloxy-1 butanol-3 (S) et du composé MD 200405 a été obtenu le [(trifluoro-4,4,4 hydroxy-3 (S) butoxy)-4 phényl]-3 méthoxyméthyl-5 (R) oxazolidinone-2 (numéro de code MD 370504). F : 121 °C; $[\alpha]_D^{20}$ : - 59,7 ° (C = 1, $CH_2Cl_2$) ; RMN$^1$H ($CDCl_3$)δppm : 2,1 (2H) ; 3,4 (4H dont 1 éch.) ; 3,6 (2H) ; 3,7-4,4 (5H) ; 4,7 (1H) ; 6,8 (2H) ; 7,4 (2H) ; IR (KBr) $\nu cm^{-1}$ : 3420, 1735.
A partir du composé racémique trifluoro-4,4,4 tosyloxy-1 butanol-3 (numéro de code MD 370272) et du composé MD 200405 a été obtenu dans les mêmes conditions le mélange de diastéréoisomères [(trifluoro-4,4,4 hydroxy-3 butoxy)-4 phényl]-3 méthoxyméthyl-5 (R) oxazolidinone-2 (numéro de code MD 230016).
RMN$^1$H ($CDCl_3$ + DMSO)δppm : 1,8-2,3 (2H) ; 3,4 (3H) ; 3,6 (2H) ; 3,8-4,4 (5H) ; 4,7 (1H) ; 5,3 (1H éch.) ; 6,9 (2H) ; 7,4 (2H) ; IR (KBr) $\nu cm^{-1}$ : 3400, 1755, 1735.
F = 103 °C ; $[\alpha]_D^{20}$ : - 35,2 ° (C = 1, $CH_2Cl_2$).
De la manière mais à partir des matières correspondantes ont été obtenus : [(Trifluoro-4,4,4 hydroxy-3 (S) butoxy)-4 phényl]-3 méthoxyméthyl-5 (S) oxazolidinone-2 (numéro de code MD 230154) : $[\alpha]^D_{20}$ = + 9,9 °

(C = 1, $CH_2Cl_2$) ; F = 100 ° C.
[(Trifluoro-4,4,4 hydroxy-3 (R) butoxy)-4 phényl]-3 méthoxyméthyl-5 (S) oxazolidinone-2 (numéro de code MD 230151) : $[\alpha]^{D}_{20}$ = + 59,2 ° (C = 1, $CH_2Cl_2$) ; F = 123 ° C.
[(trifluoro-4,4,4 hydroxy-3 (R) butoxy)-4 phényl]-3 éthoxy méthoxy-5 (R) oxazolidinone (numéro de code MD 230197) : F : 91 ° C ; $[\alpha]^{20}_{D}$ : - 11,4 ° (C = 1, $CH_3OH$) ; IR (KBr) $\nu cm^{-1}$ : 3400, 1750, 1735, RMN$^1$H ($CDCl_3$)-δppm : 1,1 (3H) ; 3,3-4,4 (9H) ; 4,7 (1H) ; 6,3 (1H éch.) ; 6,9 (2H) ; 7,4 (2H).

Exemple 8 :

[[(méthyl-2 dioxolane-1,3 yl-2)-2 éthoxy]-4 phényl]-3 méthoxyméthyl-5 (R) oxazolidinone-2 (numéro de code MD 370296).

A une solution de 50 ml de DMF, on ajoute 3 g (0,076 mole) de NaH à 60 % et en 15 mn, 16 g (0,076 mole) du composé MD 200405 (ex. 2, stade 3) a solution dans 75 ml de DMF. Puis en maintenant la température à 20 ° C, on ajoute 0,0836 mole de (mésyloxy-2 éthyl)-2 méthyl-2 dioxolane en solution dans 25 ml de DMF. Le milieu réactionnel est laissé 24 heures à température ambiante et versé sur l'eau glacée. La phase aqueuse est extraite au $CH_2Cl_2$ et la phase organique est séchée au sulfate de magnésium. Le produit est obtenu après purification sur colonne de silice (éluant : Heptane : 40 ; Acétate d'éthyle : 60) avec un rendement de 44 % ; F = 48 ° C ; $[\alpha]_D^{20}$ = - 32,8 ° (C = 1, $CH_2Cl_2$) ; RMN$^1$H ($CDCl_3$)δppm : 1,4 (3H) ; 2,2 (2H) ; 3,4 (3H) ; 3,6 (2H) ; 3,9 (4H) ; 3,7-4,3 (4H) ; 4,7 (1H) ; 6,8 (2H) ; 7,4 (2H) ; IR (KBr) $\nu cm^{-1}$ : 1740.
RMN$^{13}$C : Cq : 155,6 ; 154,4 ; 131,5 ; 108,7 ; CH : 120,2 ; 114,9 ; 71,2 ; $CH_2$ : 72,7 ; 64,6 ; 64,3 ; 47,5 ; 38,2 ; $CH_3$ : 59,6 ; 24,4.
De la même manière ont été obtenus :
[[(méthyl-2 dioxolane-1,3 yl-2)-3 propoxy]-4 phényl]-3 méthoxyméthyl-5 (R) oxazolidinone-2 (numéro de code MD 370506) : RMN$^1$H ($CDCl_3$)δppm : 1,35 (3H) ; 1,8 (4H) ; 3,4 (3H) ; 3,6 (2H) ; 3,7-4,2 (4H) ; 3,9 (4H) ; 4,7 (1H) ; 6,8 (2H) ; 7,4 (2H) ; IR (KBr) $\nu cm^{-1}$ ; 1750. F = 67 ° C
[(méthyl-2 dioxolane-1,3 yl-2)-2 éthoxy]-4 phényl]-3 hydroxyméthyl-5 (R) oxazolidinone-2 (numéro de code MD 230046) : RMN$^1$H ($CDCl_3$)δppm : 1,4 (3H) ; 2,15 (2H) ; 3 (1H éch.) ; 3,9 (4H) ; 3,6-4,2 (6H) ; 4,6 (1H) ; 6,2 (2H) ; 7,4 (2H). IR (KBr) $\nu cm^{-1}$ : 3480, 1710 : $[\alpha]_D^{20}$ = - 40,2 °(C = 1, $CH_2Cl_2$) F = 132 ° C rendement = 96 %.
(dioxa -1,3 spiro [4,4] nonane [1,4]-yl-6 méthoxy)-4 phényl]-3 méthoxy méthyl-5 (R) oxazolidinone-2 (numéro de code MD 230204) : $[\alpha]^{20}_{D}$ : -44,8 ° (C = 1, $CH_3OH$) ; RMN$^1$H ($CDCl_3$) δppm : 1,4-2,6 (7H) ; 3,4 (3H) ; 3,6 (2H) ; 3,8-4,2 (8H) ; 4,7 (1H) ; 6,9 (2H) ; 7,4 (2H) ; huile.

Exemple 9 :

[(oxo-3 butoxy)-4 phényl]-3 méthoxyméthyl-5 (R) oxazolidinone-2 (numéro de code MD 370268)

A une solution de 294 g (0,871 mole) du compoé MD 370296 (exemple 8) dans 2,5 l d'acétone, on ajoute 600 g de ($FeCl_3$, $6H_2O$, $SiO_2$)$_n$ en 10 mn. Après 4 heures d'agitation, le milieu réactionnel est filtré et séché sur $Na_2SO_4$ et concentré. Le produit est obtenu avec 74,1 % de rendement :
F = 49 ° C ; $[\alpha]_D^{20}$ = - 42,6 ° (C = 1, $CH_2Cl_2$) ; RMN$^1$H ($CDCl_3$)δppm : 2,2 (3H) ; 2,85 (2H) ; 3,4 (3H) ; 3,6 (2H) ; 3,8-4,4 (4H) ; 4,7 (1H) ; 6,8 (2H) ; 7,4 (2H) ; IR (KBr) $\nu cm^{-1}$ : 1750, 1710.

Par ce procédé ont également été obtenus :
[(oxo-4 pentoxy)-4 phényl]-3 méthoxyméthyl-5 (R) oxazolidinone-2 (numéro de code MD 370507) : RMN$^1$H ($CDCl_3$)δppm : 2 (2H) ; 2,15 (3H) ; 2,6 (2H) ; 3,4 (3H) ; 3,6 (2H) ; 3,9 (4H) ; 4,65 (1H) ; 6,8 (2H) ; 7,4 (2H) ; IR (KBr) $\nu cm^{-1}$ : 1760, 1710 ; $[\alpha]_D^{20}$ = - 40,3 ° (C = 1, $CH_2Cl_2$) ; F = 70 ° C.
[(oxo-3 butoxy)-4 phényl]-3 hydroxyméthyl-5 (R) oxazolidinone-2 (numéro de code MD 230047) : RMN$^1$H ($CDCl_3$)δppm : 2,2 (3H) ; 2,9 (2H) ; 3,3-4,3 (4H) ; 4,2 (2H) ; 4,7 (1H) ; 5,2 (1H éch.) ; 6,9 (2H) ; 7,5 (2H) ; IR (KBr) $\nu cm^{-1}$ : 3450, 1720 ; $[\alpha]_D^{20}$ = - 49,4 ° (C = 1, $CH_3OH$) ; F = 126 ° C.
[(oxo-1 cyclopentyl-2 méthoxy)-4 phényl]-3 méthoxy méthyl-5 (R) oxazolidinone-2 (numéro de code MD 230200) : F : 70 ° C ; $[\alpha]^{20}_{D}$ : - 51,2 ° (C = 1, $CH_3OH$) ; RMN$^1$H ($CDCl_3$)δppm : 1,8-2,1 (7H) ; 3,4 (3H) ; 3,6 (2H) ; 3,8-4,2 (4H) ; 4,7 (1H) ; 6,9 (2H) ; 7,4 (2H).

Exemple 10 :

[(oxo-3 diméthyl-2,2 butoxy)-4 phényl]-3 hydroxyméthyl-5 (R) oxazolidinone-2 (numéro de code MD 230109)

Stade 1 :

Diméthyl-2,2 (méthyl-2 dioxolan-1,3-yl-2)-2 éthanol (numéro de code MD 230103)

Une solution de 35 g (0,073 mole) d'ester éthylique de l'acide diméthyl-2,2 (méthyl-2 dioxolane-1,3 yl-2)-2 acétique dans 50 ml de THF est ajoutée à 0°C à une suspension de 7,23 g (0,19 mole) de $LialH_4$ dans 300 ml de THF en 15 mn. Puis le milieu réactionnel est hydrolysé par 20 ml d'eau. Après filtration et concentration, le produit est obtenu avec un rendement de 92 %. IR (KBr)νcm-1 : 3450, 2980, 2880. RMN$^1$H ($CDCl_3$) : 1 (6H) ; 1,2 (3H) ; 3,5 (2H) ; 4 (4H).

Stade 2 :

Méthyl-2 [(nitro-4 phénoxy)-2 diméthyl-1,1 éthyl]-2 dioxolane-1,3 (numéro de code MD 230105).

A une solution de 1,6 g (0,01 mole) de MD 230103 dans 13 ml de DMF, on ajoute 0,48 g (0,01 mole) de NaH à 50 %. Après 15 mn d'agitation, on ajoute une solution de 1,32 g (0,0084 mole) de parachloronitrobenzène et agite à température ambiante 30 mn.

Le milieu réactionnel est versé sur l'eau et extrait à l'éther isopropylique. Les phases organiques sont lavées à l'eau saturée de NaCl, séchées sur $Na_2SO_4$ et concentrées. Le produit est purifié par chromatographie flash (silice, éluant : heptane : 80 ; acétate d'éthyle : 20). Rendement : 68 % ; huile. RMN$^1$H ($CDCl_3$) δppm : 1,1 (6H) ; 1,3 (3H) ; 4 (6H) ; 6,9 (2H) ; 8,1 (1H).

Stade 3 :

Méthyl-2 [(amino-4 phénoxy)-2 diméthyl-1,1 éthyl]-2 dioxolane-1,3 (numéro de code MD 230106).

A une solution de 18,4 g (65,4 $10^{-3}$ mole) du composé 230105 dans 180 ml d'éthanol en présence de Pd/C à 10 % humidifié à 50 %, on fait arriver une courant d'hydrogène sous pression normale pendant 3 h 30. Après filtration et concentration, le produit est purifié par chromatographie flash (silice, éluant : acétate d'éthyle : 30 ; heptane : 70). RMN$^1$H ($CDCl_3$)δppm : 1,05 (6H) ; 1,3 (3H) ; 3,3 (2H éch.) ; 3,7 (2H) ; 3,9 (4H) ; 6,7 (4H). IR (microcellule) ν$cm^{-1}$ : 3460, 3450.

Stade 4 :

N-[[méthyl-2 dioxolane-1,3 yl-2)-2 diméthyl-1,1 éthoxy]-4 phényl] dioxa-1,4 spiro [4,5] décane méthanamine-2 (R) (numéro de code MD 230107)

8,8 g (0,035 mole) de MD 230 106, 12,6 g (0,036 mole) de tosylate de dioxa-1,4 [4,5] décane méthanol-2 (S) et 5,4 g (0,054 mole de triéthylamine sont chauffés en bombe à 130-140° C pendant 2 heures. Après refroidissement, le milieu réactionnel est repris dans l'acétate d'éthyle. La phase organique est lavée à l'eau saturée de sel, séchée et concentrée. Le produit est obtenu après chromatographie (silice, éluant : heptane : 80 ; acétate d'éthyle : 20) ; rendement : 63 % ; RMN$^1$H ($CDCl_3$) δppm : 1,1 (6H) ; 1,35 (3H) ; 1,6 (10H) ; 3,2 (2H) ; 3,6-4,5 (10 H dont 1 éch.) ; 6,65 (4H) ; IR (microcellule) ν$cm^{-1}$ : 3400 ; $[\alpha]_D^{20}$ : - 1,2° (C = 1, MeOH).

Stade 5 :

[(oxo-3 diméthyl-2,2 butoxy)-4 phényl]-amino-3 propane diol-1,2 (R) (numéro de code MD 230108).

A une solution de 0,7 g (1,7 $10^{-3}$ mole) du composé MD 230107 dans 3,5 ml de THF, on ajoute, goutte à goutte, 3,5 ml d'acide chlorhydrique 6N. Après 1 h, le milieu réactionnel est versé sur l'eau et extrait à l'acétate d'éthyle. La phase organique est lavée à l'eau, séchée sur $Na_2SO_4$ et concentrée. Rendement de 80 %. IR (microcellule) ν$cm^{-1}$ : 3400, 1710.

Stade 6 :

[(oxo-3 diméthyl-2,2 butoxy)-4 phényl]-3 hydroxyméthyl-5 (R) oxazolidinone-2 (numéro de code MD 230109) a été obtenu selon le mode opératoire du stade 2 a) de l'exemple 2 : F = 109°C; $[\alpha]_D^{20}$ = - 44,4° (C = 1, MeOH) ; RMN$^1$H ($CDCl_3$)δppm : 1,25 (6H) ; 2,2 (3H) ; 3,9 (6H) ; 4,7 (1H) ; 6,9 (2H) ; 7,4 (2H). IR (KBr) ν$cm^{-1}$ : 3450, 1745-1725.

Exemple 11 :

[(oxo-3 diméthyl-2,2 butoxy)-4 phényl]-3 méthoxyméthyl-5 (R) oxazolidinone-2 (numéro de code MD 230073)

A une suspension de 2,9g(0,009 mole) de MD 230109 dans 40 ml de toluène, on ajoute 3,8 g de soude à 50 %, 0,3 g de bromure de tétrabutylammonium et 3,6 g (0,0283 mole) de sulfate de méthyle. Après 10 mn d'agitation, le milieu réactionnel est versé sur l'eau. La phase organique est lavée, séchée sur $Na_2SO_4$ et concentrée et le produit est obtenu après chromatographie (silice, éluant : acétate d'éthyle : 50 ; heptane : 50); F : 75° C : $[\alpha]_D^{20}$ : - 52° (C = 1, MeOH) ; RMN$_1$H ($CDCl_3$) δppm : 1,25 (6H) ; 2,2 (3H) ; 3,6 (2H) ; 3,7-4,2 (2H) ; 3,9 (2H) ; 4,65 (1H) ; 6,8 (2H) ; 7,4 (2H) ; RMN$^{13}$C($CDCl_3$)δppm : Cq : 211,9 ; 155,8 ; 154,9 ; 132 ; 48,4 ; CH : 120,3 ; 115,1 ; 71,3 ; $CH_2$ : 74,9 ; 72,8, 47,8 ; $CH_3$ : 25,8 ; 22 ; IR (KBr) $\nu cm^{-1}$ : 1735, 1715.

Exemple 12:

[(oxo-4 pentyl)-4 phényl]-3 hydroxyméthyl-5 (R) oxazolidinone-2 (numéro de code MD 230116).

Stade 1 :

(p-nitrocinnamyl)-2 méthyl-2 dioxolane-1,3 (numéro de code MD 230111).

A 62,8 mmoles de LDA dans 226,4 ml de THF, ont a joute à 0°C une solution de 28,8 g (62,2 mmole) de bromure de (méthyl-2 dioxolane-yl-2 éthyl-2) triphénylphosphonium dans 60 ml de DMSO, goutte à goutte. Après 1 h à 0°C, on ajoute 7,8 g (51,6 mmoles) de p-nitro benzaldéhyde en solution dans 40 ml de THF. Le milieu réactionnel est hydrolisé par une solution saturée de $NH_4Cl$ et est extrait à l'éther éthylique. La phase organique est séchée sur $Na_2SO_4$ et concentrée. Après purification par chromatographie flash (silice, éluant : heptane : 70 ; acétate d'éthyle : 30) le produit est obtenu avec un rendement de 48 %. RMN$^1$H ($CDCl_3$)δppm : 1,3 (3H) ; 2,6 (2H) ; 4 (4H) ; 5,7-6,5 (2H) ; 7,4 (2H) ; 8,1 (2H).

Stade 2 :

(amino-4 cinnamyl)-2 méthyl-2 dioxolane-1,3 (numéro de code MD 230112).

A une solution de 8 g (32 mmoles) du composé MD 230111 dans 100 ml d'éthanol en présence de 0,8 g de Pd/C à 10 % dans un autoclave, on fait passer un courant d'hydrogène sous 5 atmosphères pendant 4 h. Après filtration, concentration, purification par chromatographie flash (silice, éluant : heptane : 50 ; acétate d'éthyle : 50) le produit est obtenu avec un rendement de 89 %. F : < 50°C ; RMN$^1$H ($CDCl_3$)δppm : 1,35 (3H) ; 2,4-2,8 (2H) ; 3,6 (2H éch.) ; 4 (4H) ; 5,5-6,3 (2H) ; 6,6 (2H) ; 7,2 (2H) ; IR (microcellule) $\nu cm^{-1}$ : 3460, 3440.

Stade 3 :

(amino-4 phényl propyl)-2 méthyl-2 dioxolane-1,3 (numéro de code MD 230113).

Une solution de 15,4 g (70,23 mmoles) du composé MD 230112 dans 100 ml d'éthanol et 1,5 g de Pd/C à 10% sont placés dans un autoclave ; on fait arriver un courant d'hydrogène sous 9 atmosphères pendant 1 h à 50° C. Après filtration et concentration, on obtient 15,5 g de produit attendu (liquide). RMN$^1$H ($CDCl_3$)δppm : 1,25 (3H) ; 1,6 (4H) ; 2,45 (2H) ; 3,5 (2H, éch.) ; 3,85 (4H) ; 6,55 (2H) ; 6,9 (2H). IR (microcellule , $\nu cm^{-1}$) : 3460, 3350.

Stade 4 :

[[Méthyl-2 dioxolane-1,3 yl-2)-3 propyl]-4 phényl]dioxa-1,4 spiro [4,5] décane méthanamine-2 (R) (numéro de code MD 230114).

A un mélange d'1 g (4,5 mmoles) du composé MD 230113 et 1,62 g (4,97 mmoles) de tosylate du dioxa-1,4 spiro [4,5] décane méthanol-2 (S), on ajoute 0,73 g (1 ml ; 7,23 mmoles) de triéthylamine et on chauffe à 140°C pendant 5 h. Le milieu réactionnel est repris dans l'eau et extrait à l'acétate d'éthyle. La phase organique est lavée à l'eau salée, puis, séchée sur $Na_2SO_4$. On obtient le produit sous forme liquide avec un rendement de 59 % après chromatographie flash (silice, éluant : heptane : 40 ; acétate d'éthyle : 60). RMN$^1$H ($CDCl_3$)δppm : 1,2 (3H) ; 1,5 (10H) ; 1,6 (4H) ; 2,4 (3H) ; 3,15 (3H) ; 3,8 (4H) ; 3,6-4,5 (3H). IR (microcellule) $\nu cm^{-1}$ : 3400 ; $[\alpha]_D^{20}$ = - 2,9° (C = 1, MeOH).

Stade 5 :

[(oxo-4 pentyl)-4 phényl]-amino propanediol-1,2 (R) (numéro de code MD 230115) a été obtenu selon un mode opératoire identique à celui du stade 5 de l'exemple 10 : RMN$^1$H ($CDCl_3$)δppm : 1,8 (2H) ; 2 (3H) ; 2,4 (4H) ; 2,7-3,3 (3H) ; 3,1 (3H) ; 3,6 (2H) ; 3,3 (1H) ; 6,5 (2H) ; 6,9 (2H).

Stade 6 :

[(oxo-4 pentyl)-4 phényl]-3 hydroxyméthyl-5 (R) oxazolidinone-2 (numéro de code MD 230116) : obtenu selon un mode opératolre identique à celui du stade 6 de l'exemple 10 : F = 110 °C ; $[\alpha]_D^{20}$ = - 50,7 ° (C = 1, MeOH) ; RMN$^1$H ($CDCl_3$)δppm : 1,8 (2H) ; 2,05 (3H) ; 2,2-2,7 (4H) ; 2,75 (1H) ; 3,65-4,10 (2H) ; 4,65 (1H) ; 7,1 (2H) ; 7,4 (2H) ; IR (KBr) $\nu cm^{-1}$ : 3460, 1720.

Exemple 13 :

[(oxo-4 pentyl)-4 phényl]-3 méthoxyméthyl-5 (R) oxazolidinone-2 (numéro de code MD 230083) : a été obtenu à partir du composé MD 230116 selon le mode opératoire de l'exemple 11. F : < 50 °C ; $[\alpha]_D^{20}$ = - 56,9 ° (C = 1, MeOH) ; RMN$^1$H ($CDCl_3$) : 1,9 (2H) ; 2,1 (3H) ; 2,45 (4H) ; 3,4 (3H) ; 3,6 (2H) ; 3,9 (2H) ; 4,7 (1H) ; 7,1 (2H) ; 7,4 (2H) ; IR (KBr) $\nu cm^{-1}$ : 1750, 1710.

Exemple 14 :

[(hydroxy-4 pentyl)-4 phényl]-3 méthoxyméthyl-5 (R) oxazolidinone-2 (numéro de code MD 230082).

A une solution de 1,83 g (6,28 mmoles) du composé MD 230083 (exemple 13) dans 30 ml d'éthanol on ajoute 0,24 g (6,28 mmoles) de $NaBH_4$. Après 10 mn de milieu réactionnel on ajoute de l'eau et extrait du chlorure de méthylène. La phase organique est lavée à l'eau salée et séchée sur $Na_2SO_4$ et concentrée. Le produit est obtenu après chromatographie flash sous forme d'huile : $[\alpha]_D^{20}$ = - 56,3 ° (C = 1, MeOH) ; RMN$^1$H($CDCl_3$)δppm: 1,15 (3H) ; 1,55 (5H) ; 2,6 (2H) ; 3,4 (3H) ; 3,6 (3H) ; 3,9 (2H) ; 4,65 (1H) ; 7,1 (2H) ; 7,4 (2H) ; IR (microcellule) $\nu cm^{-1}$ : 3500-3400, 1750.

EXEMPLE 15 :

(Hydroxy-4 (R) pentyl)-4 phényl]-3 méthoxy méthyl-5 (R) oxazolidinone-2 (numéro de code MD230238).

Stade 1 :

Terbutyl diméthyl silyloxy méthyl-4 nitro-1 benzène (numéro de code MD230245).

A une solution de 465,4 g (3,039 moles) de paranitrobenzylalcool dans 2,5 l de DMF, on ajoute 310 g (4,559 moles) d'imidazole puis 504 g (3,347 moles) de terbutyl diméthylchlorosilane. Après 1 h d'agitation à température ambiante, le milieu réactionnel est versé sur l'eau. La phase aqueuse est extraite du chlorure de méthylène. La phase organique est séchée sur $Na_2SO_4$ et concentrée ; huile ; RMN$^1$H ($CDCl_3$) δppm : 0,2 (6H) ; 1 (9H) ; 4,9 (2H) ; 7,6 (2H) ; 8,2 (2H) ; IR (microcellule)$\nu cm^{-1}$ : 1520, 1340, 1030, 840.

Stade 2 :

Terbutyl diméthylsilyloxy méthyl-4 aniline (numéro de code MD230246).

A 772 ml de chlorure d'ammonium 0,1 N, on ajoute 77,2 g (0,288 mole) du composé précédent MD230245 et 120,9 g de poudre de fer et on chauffe à reflux 2 h. Après refroidissement, on ajoute 20 ml d'ammoniaque concentrée, le milieu réactionnel est filtré et extrait par le toluène. La phase organique est lavée à l'eau, séchée au $Na_2SO_4$ et concentrée ; Eb 0,01 mm Hg: 88-93 °C ; RMN$^1$H ($CDCl_3$) δppm 0,2 (6H) ; 1,05 (9H) ; 3,6 (2H) ; 4,8 (2H) ; 6,75 (2H) ; 7,2 (2H). IR (microcellule) $\nu cm^{-1}$ : 3450, 3350.

Stade 3 :

[(Terbutyl diméthyl silyoxy méthyl)-4 phényl]-3 méthoxyméthyl-5 (R) oxazolidinone-2 (numéro de code MD230247).

A une solution de 43,8 g (0,168 mole) de MD 370488 (stade 3 de l'ex. 17) dans 200 ml de toluène, on ajoute 130 ml d'une solution toluénique de phosgène 1,93 Molaire puis, goutte à goutte, 37,8 g (0,252 mole) de diéthylaniline. Après refroidissement, on ajoute de l'eau glacée et la phase organique est décantée et séchée sur $Na_2SO_4$. Cette solution est alors additionnée à une solution de 40 g (0,168 mole) de MD230246 et de 20,5 g (0,168 mole) de diméthylaminopyridine dans 600 ml de toluène. Après 1/2 h d'agitation, le milieu réactionnel est versé sur l'eau et la phase organique est lavée avec une solution de bicarbonate de sodium puis par une solution saturée en NaCl. Après concentration, le produit obtenu (84,5 g) est mis en solution dans 800 ml d'éthanol auquel est ajouté 12,2 g (0,218 mole) de KOH en pastilles. Après 1/2 h d'agitation, le milieu réactionnel est versé sur l'eau et extrait au chlorure de méthylène. La phase organique est séchée sur $Na_2SO_4$ et concentrée. Le produit est obtenu après chromatographie (silice, éluant : acétate d'éthyle : 30 ; heptane : 70) avec un rendement de 63 % ; $[\alpha]_D^{20}$: - 46,2°(C = 1, $CH_3OH$) ; IR (KBr) $\nu cm^{-1}$ : 1755, 1735; RMN$^1$H ($CDCl_3$) δppm : 0 (6H) ; 1 (9H) ; 3,4 (3H) ; 3,6 (2H) ; 3,8-4,2 (2H) ; 4,7 (3H) ; 7,5 (4H) ; F < 50° C.

Stade 4 :

[(Hydroxyméthyl-4) phényl-]-3 méthoxyméthyl-5 (R) oxazolidinone-2 (numéro de code MD230248).

Une solution de 29,2 g (0,083 mole) de MD230247 et 7,8 g (0,025 mole) de fluorure de tétrabutylammonium trihydraté dans 200 ml de THF est agitée 12 h à température ordinaire et le milieu réactionnel est concentré. Le produit est obtenu après chromatographie (silice, éluant : acétate d'éthyle : 50, heptane : 50) ; F : 65 °C ; IR (KBr) $\nu cm^{-1}$ : 3400, 1750, 1720. ; RMN$^1$H ($CDCl_3$) δppm : 2,4 (1H éch.) ; 3,35 (3H) ; 3,6 (2H) ; 3,8-4,2 (2H) ; 4,6 (2H) ; 7,35 (4H).

Stade 5 :

(carboxaldéhydo-4 phényl)-3 méthoxyméthyl-5 (R) oxazolidinone-2 (numéro de code MD230256).

A une solution refroidie à - 60°C de 12,46 g (0,0982 mole) de chlorure d'oxalyle dans 80 ml de chlorure de méthylène, on introduit en 20 mn une solution de 12,76 g (0,1630 mole) de DMSO dans 80 ml de chlorure de méthylène. 40 mn plus tard, on ajoute une solution de 19,6 g (0,0818 mole) de MD230248 dans 80 ml de chlorure de méthylène puis 1,4 g (0,409 mole) de triéthylamine. Après retour à température ambiante, on ajoute 300 ml d'eau. La phase organique est lavée à l'eau, séchée et concentrée. Le produit a été obtenu après purification par chromatographie (silice, éluant : acétate d'éthyle : 70, heptane : 30) avec un rendement de 80 % ; F : 96 °C ; $[\alpha]_D^{20}$ : - 73,4°(C = 1, $CH_2Cl_2$) ; IR (KBr) $\nu cm^{-1}$ : 1740, 1690 ; RMN$^1$H ($CDCl_3$) δppm : 3,4 (3H) ; 3,7 (2H) ; 3,8-4,3 (2H) ; 4,8 (1H) ; 7,8 (4H) ; 9,8 (1H).

Stade 6 :

[(Hydroxy-4 (R) pentyl)-4 phényl]-3 méthoxyméthyl-5 (R) oxazolidinone-2 (numéro de code MD230238).

Une solution de 3,3 g (0,00712 mole) d'Iodure d'hydroxy-2 (R) propyl triphénylphosphonium (Helv. Chim. Acta, 59, 755-757, 1976),de 1,34 g (0,00569 mole) de MD230256 et 2,9 g (0,0213 mole) de $K_2CO_3$ dans 10 ml de dioxanne et 1,5 ml de formamide est chauffée au reflux 20 h. Après filtration et concentration, le produit insaturé obtenu est purifié en le dissolvant dans 30 ml de DMF et on ajoute 0,58 g d'imidazole et 0,94 g (0,00625 mole) de terbutyl diméthylchlorosilane. Après 24 h d'agitation, le milieu réactionnel est versé sur l'eau. Le produit silylé est extrait au chlorure de méthylène et purifié par chromatographie (silice, éluant : acétate d'éthyle : 50, heptane : 50) avec un rendement de 36 %. 0,84 g du produit résultant est mis en solution dans 15 ml de THF en présence de 0,65 g de fluorure de tétrabutylammonium pendant 12 h. Après concentration et purification par chromatographie (silice, éluant : acétate d'éthyle : 70, heptane : 30), 0,53 g (0,0018 mole) du produit insaturé purifié en solution dans 10 ml de méthanol en présence de palladium sur carbone à 10 % (humide à 50 %) est hydrogéné sous pression normale. Le produit attendu est obtenu avec un rendement de 55 % après chromatographie (silice, acétate d'éthyle : 60, heptane : 40) ; $[\alpha]_D^{20}$ : -45,8°(C = 1, $CH_2Cl_2$) ; IR (KBr) $\nu cm^{-1}$ : 3400, 1735 ; F : 47°C ; RMN$^1$H($CDCl_3$)δppm : 1,2 (3H) ; 1,5 (4H) ; 1,8 (1H éch.) ; 2,6 (2H) ; 3,4 (3H) ; 3,6 (2H) ; 3,7-4,2 (3H) ; 4,7

(1H) ; 7,2 (2H) ; 7,4 (2H).

De la même façon a été obtenu : [(hydroxy-4 (S) pentyl)-4 phényl]-3 méthoxyméthyl-5 (R) oxazolidinone-2 (numéro de code MD230239) ; F : 53 °C ; $[\alpha]_D^{20}$ : - 35,9° (C = 1, $CH_2Cl_2$) ; IR (KBr) $\nu cm^{-1}$ : 3400, 1740 ; RMN[1]H ($CDCl_3$) δppm : 1,1 (3H) ; 1,6 (5H dont 1 éch.) ; 3,4 (3H) ; 3,6 (2H) ; 3,7-4,2 (3H) ; 4,7 (1H) ; 7,1 (2H) ; 7,4 (2H).

Il est à noter que le produit insaturé susmentionné est l'[(hydroxy-4 pentylène-1)-4 phényl]-3 méthoxyméthyl-5 (R) oxazolidinone-2 (numéro de code 230319) $[\alpha]_D^{20}$ : - 38,8° (C = 1, $CH_2Cl_2$) ; IR (microcellule) $\nu cm^{-1}$ : 3500-3400, 1750; RMN[1]H ($CDCl_3$) δppm : 1,2 (3H) ; 2,05 (1H) ; 2,4 (2H) ; 3,4 (3H) ; 3,6 (2H) ; 3,7-4,2 (3H) ; 4,7 (1H) ; 5,4-6,5 (2H) ; 7,4 (4H).

EXEMPLE 16

[(Méthyl-2 dioxolane-1,3 yl-2 propyl)-4 phényl]-3 méthoxyméthyl-5 (R) oxazolidinone-2 (numéro de code MD230084).

A une solution de 1,83 g (6,28 mmoles) du composé MD230083 (ex. 13) dans 25 ml de toluène, on ajoute 0,382 g (6,28 mmoles) d'éthylène glycol et on chauffe au reflux 12 h en présence d'acide p-toluène sulfonique et en éliminant l'eau. Le milieu de réaction est concentré. Le résidu est repris dans $CH_2Cl_2$. La phase organique est lavée au $NaHCO_3$ puis à l'eau, séchée et concentrée. Le produit est purifié par HPLC (silice, éluant : éther isopropylique : 65 ; heptane : 25 ; méthanol : 10) ; F : 81°C ; $[\alpha]_D^{20}$ : -49° (C = 1, MeOH) ; IR (KBr) $\nu cm^{-1}$ : 1740 ; RMN[1]H ($CDCl_3$) δppm : 1,25 (3H) ; 1,65 (4H) ; 2,55 (2H) ; 3,4 (3H) ; 3,6 (2H) ; 3,9 (6H) ; 4,65 (1H) ; 7,1 (2H) ; 7,4 (2H).

EXEMPLE 17 :

Stade 1 :

Diméthyl-2,2 méthoxyméthyl-4 (S) dioxolane (numéro de code MD370486).

A 910 ml d'eau, on ajoute 910 g de NaOH en pastilles puis à température ambiante 5 l de chlorure de méthylène, 44,4 g (0,195 mole) de chlorure de benzyltriéthylammonium, 8558,6 g (6,5 moles) de diméthyl-2,2 hydroxyméthyl-3 (S) dioxolane et 1229,5 g (9,75 moles) de sulfate de diméthyle. Le milieu réactionnel est agité 12 h et versé sur l'eau. La phase organique est concentrée. Le produit est distillé : Eb : 45 °C sous 10 mm Hg ; $[\alpha]_D^{20}$ : +7,9°(C=4, $CH_3OH$) ; IR (microcellule) $\nu cm^{-1}$ : 2995, 2940, 2820, 1380, 1370, 840 ; RMN[1]H ($CDCl_3$) δppm : 1,2 (3H) ; 1,4 (3H) ; 3,35 (3H) ; 3,4-4,4 (3H) ; 4 (2H).

Stade 2 :

Méthoxy-3 propane diol-1,2 (R) (numéro de code MD370487).

On chauffe une solution de 950,3 g (6,5 moles) de MD370486 dans 4510 ml d'eau à 60 °C et on ajoute 3,2 ml d'acide chlorhydrique concentré. Puis on ajoute 9 ml de triéthylamine et le milieu réactionnel est concentré et distillé avec un rendement de 84 % ; Eb : 66 °C sous 1 mm Hg ; $[\alpha]_D^{20}$ : - 6,4°(C=4, $CH_3OH$) ; IR (microcellule) $\nu cm^{-1}$ : 3300,3500, 2960, 2945, 2910 ; RMN[1]H (DMSO $d_6$) δppm : 3,2-3,7 (8H) ; 4,5 (2H éch.).

Stade 3 :

Tosylate de méthoxy-3 propane-diol -1,2 (S) (numéro de code MD370488).

Une solution de 371,4 g (3,5 moles) de MD370487 dans 100 ml de toluène est refroidie à 13 °C et on ajoute 565 ml de pyridine puis petit à petit une solution de 700,6 g (3,675 moles) de chlorure de paratoluène sulfonique dans 775 ml de toluène. Le milieu réactionnel est agité pendant 12 h et versé sur l'eau. La phase organique est lavée à l'acide chlorhydrique 2N et concentrée. Le produit est obtenu avec un rendement de 58 % après chromatographie (silice, éluant : $CH_2Cl_2$ : 50 ; éther de pétrole : 50) ; $[\alpha]_D^{20}$ : + 5,3°(C = 4, $CH_3OH$) ; IR (microcellule) $\nu cm^{-1}$ : 3500, 1335, 1185, 1170 ; RMN[1]H ($CDCl_3$) δppm : 2,4 (3H) ; 3,1 (1H éch.) ; 3,2-3,6 (5H) ; 3,8-4,2 (3H).

Stade 4 :

[[(Méthyl-2-dioxolane-1,3 yl-2)-3 propyl]-4 phényl]-3 méthoxyméthyl-5 (R) oxazolidinone-2 (numéro de code MD230084).

A 8,9 g (0,0887 mole) de phosgène dans 120 ml de dichloroéthane, on ajoute 5,4 g (0,059 mole) de MD370488 puis 13,3 g (0,0887 mole) de diméthyl aniline en solution dans 20 ml de dichloroéthane. Le milieu réactionnel est agité 1 h 30 à 50 °C. Après refroidissement, le milieu réactionnel est lavé à l'eau glacée et séché sur sulfate de sodium. Cette solution est additionnée à une solution de 13 g (0,059 mole) de MD230113 (ex. 12, stade 3) et 7,2 g (0,059 mole) de diméthylamino-4 pyridine dans 200 ml de dichloroéthane. Le milieu réactionnel est alors chauffé au reflux 30 mn, refroidi et versé sur l'eau. La phase organique est lavée avec une solution de bicarbonate de soude, séchée et concentrée. Le produit est obtenu après chromatographie (silice, éluant : éther isopropylique : 65 ; heptane : 25 ; $CH_3OH$ : 10) avec un rendement de 47 %. Il a les mêmes caractéristiques physique que celles du composé obtenu à l'exemple 16.

EXEMPLE 18:

[Bromo-3 (trifluoro-4,4,4 hydroxy-3 (R) butoxy)-4 phényl]-3 méthoxy méthyl-5 (R) oxazolidinone-2 (numéro de code MD230237)

A la solution de 2 g (0,00572 mole) de MD370503 (ex. 7) dans 8 ml d'acide acétique, on ajoute 1,83 g (0,01145 mole) de brome en solution dans 10 ml d'acide acétique. Le milieu réactionnel est agité 2 h et versé sur de l'eau glacée. La phase aqueuse est extraite au chlorure de méthylène et l'acétate d'éthyle. La phase organique est séchée sur $Na_2SO_4$ et concentrée. Le produit est purifié par chromatographie (silice, éluant : $CH_2Cl_2$ : 98 ; MeOH:2) ; F : 87° C ; $[\alpha]_D^{20}$ : - 14,8° (C = 1, $CH_2Cl_2$) ; IR (KBr) $\nu cm^{-1}$ : 3360, 3400, 1760, 1725 ; RMN$^1$H ($CDCl_3$) δppm : 2,1 (2H) ; 3,4 (3H) ; 3,6 (2H) ; 3,7-4,9 (7H) ; 6,8 (1H) ; 7,5 (1H) ; 7,6 (1H).

**Exemple 19 :**

[[(hydroxy-1-cyclohexyl-1)-2 éthoxy]-4 phényl]-3 méthoxyméthyl-5 (R) oxazolidinone-2
(numéro de code MD 360331)

Stade 1

(mésyloxy-2 éthyl)-1 cyclohexanol-1 (MD 360342)

Obtenu à partir de l'(hydroxy-2 éthyl)-1 cyclohexanol-1(Org. Prep. Proc. 16, 321, 1984) selon le mode opératoire du stade 5 de l'exemple 7 dans lequel on utilise du chlorure de mésyle à la place du chlorure de tosyle. Liquide ;

| Analyse élémentaire : | | |
|---|---|---|
| Cal. % | C : 48,62 | H : 8,16 |
| Tr. % | C : 47,71 | H : 8,43 |

Stade 2

[[(hydroxy-1-cyclohexyl-1)-2 éthoxy]-4 phényl]-3 méthoxyméthyl-5 (R) oxazolidinone-2 (numéro de code 360331)

Obtenue selon le mode opératoire du stade 6 de l'exemple 7, avec utilisation de MD 360342 et MD 200405. Rendement = 75 % ; F = 87° C ; $[\alpha]_D^{20}$ : - 47,6° (C = 1, $CH_3OH$).

**Exemple 20 :**

[(méthyl-3 hydroxy-3 butoxy)-4 phényl]-3 méthoxyméthyl-5 (R) oxazolidinone-2
(numéro de code MD 360335)

Stade 1

méthyl-3 mésyloxy-1 butanol-3 (MD 360359)

Obtenu selon le mode opératoire du stade 5 de l'exemple 7, avec utilisation du chlorure de mésyle à la place du chlorure de tosyle. Liquide ; IR (microcellule) $\nu cm^{-1}$ : 3520-3400, 1350-1170, 950 ; RMN$^1$H

($CDCl_3$) δppm : 1,3 (6H) ; 2 (2H) ; 2,6 (1H éch.) ; 3 (3H) ; 4,4 (2H).

Stade 2

[(méthyl-3 hydroxy-3 butoxy)-4 phényl]-3 méthoxyméthyl-5(R) oxazolidinone-2 (MD 360335)

Obtenue selon le mode opératoire du stade 6 de l'exemple 7, avec utilisation de MD 360359 et MD 200405.

F : 52° C ; $[\alpha]_D^{20}$ : - 40,4° (C = 1, $CH_2Cl_2$) ; IR (KBr) νcm⁻¹ : 3480, 1745 ; RMN¹H ($CDCl_3$) δppm : 1,4 (6H) ; 2 (2H) ; 2,6 (1H éch.) ; 3,4 (3H) ; 3,6 (2H) ; 3,8-4,4 (4H) ; 4,7 (1H) ; 6,9 (2 H) ; 7,4 (2H).

De la même manière, à partir du MD 200405 et du tosylate du pentafluoro-5,5,5,4,4 hydroxy-3 pentanol (MD 360410), on a obtenu le : [(pentafluoro-5,5,5,4,4 hydroxy-3 pentoxy)-4 phényl]-3 méthoxyméthyl-5 (R) oxazolidinone-2 (MD 360328) : RMN¹H ($CDCl_3$) δppm : 2,15 (2H) ; 3 (1H éch.) ; 3,4 (3H) ; 3,6 (2H) ; 3,7-4,3 (5H) ; 4,6 (1H) ; 6,85 (2H).

Le composé MD 360410 a été obtenu selon le mode opératoire du stade 5 de l'exemple 7, à partir du pentafluoro pentane diol-1,3 (J. Fluorine Chem. 42, 17, 1989). Les données RMN¹H de MD 360410 sont comme suit : ($CDCl_3$) δppm : 1,7-2,5 (2H) ; 2,45 (3H) ; 2,8 (1H éch.) ; 3,85-4,6 (3H) ; 7,35 (2H) ; 7,8 (2H).

**Exemple 21 :**

[(trifluoro-4,4,4 hydroxy-3 butoxy)-4 phényl]-3 méthoxyméthyl-5 (R) oxazolidinone-2
(numéro de code MD 230016)

Stade 1

nitro-4 [ trifluoro-4,4-4 (éthoxy-1 éthoxy)-3 butoxy]-1 benzène (MD 360349)

A une suspension de 2,4 g (0,049 mole) de NaH à 50 % dans 50 ml de THF, on ajoute lentement une solution de 10,6 g (0,049 mole) d'(éthoxy-1 éthoxy)-3 trifluoro-4,4,4 butanol-1 dans 15 ml de THF. Après cessation du dégagement gazeux, on ajoute une solution de 6,5 g (0,041 mole) de chloro-4 nitrobenzène dans 65 ml de DMF. Après 30 mn le milieu réactionnel est versé sur l'eau glacée. Le milieu réactionnel est extrait à l'éther. La phase organique est lavée à l'eau, séchée sur $Na_2SO_4$ et concentrée. Le produit attendu est obtenu avec un rendement de 78 % après recristallisation dans l'isopropanol. F : 81° C ; IR (KBr) νcm⁻¹ : 1610-1590 ; 1510-1500 ; 1340-1250 ; 1165-1110 ; RMN¹H ($CDCl_3$) δppm : 1,15 (3H) ; 1,25 (3H) ; 2,2 (3H) ; 3,6 (2H) ; 4,2 (3H) ; 4,8 (1H) ; 6,95 (2H) ; 8,2 (2H).

De la même manière, ont été obtenus les composés suivants :

- nitro-4 (trifluoro-4,4,4 hydroxy-3 (R) butoxy)-1 benzène (MD 360363) à partir du trifluoro-4,4,4 hydroxy-3 (R) butanol-1 : F : 80° C ; Rendement : 10 % ; IR (KBr) νcm⁻¹ = 3500, 1300-1360 ; RMN¹H ($CDCl_3$) δppm : 1,9-2,5 (2H) ; 3,7 (1H) ; 4-4,6 (3H) ; 6,9 (2H) ; 8 (2H) ;
- nitro-4 (hydroxy-3 butoxy)-1 benzène (MD 360364) à partir de l'hydroxy-3 butanol-1 : RMN¹H ($CDCl_3$) δ ppm : 1,25 (3H) ; 1,65 (1H éch.) ; 2 (2H) ; 3,8 (1H) ; 6,9 (2H) ; 8,2 (2H) ;
- nitro-4 (hydroxy-4 pentoxy)-1 benzène (MD 360377) à partir de l'hydroxy-4 pentanol-1 : IR (microcellule) νcm⁻¹ : 3400, 1610-1590, 1550 ; RMN¹H ($CDCl_3$) δppm : 1,25 (3H) ; 1,4-2,2 (8H) ; 3,75 (1H) ; 4,05 (2H) ; 6,9 (2H) ; 8,15 (2H).

Stade 2

[trifluoro-4,4,4 (éthoxy-1 éthoxy)-3 butoxy]-4 aniline (MD 370350)

A été obtenue par hydrogénation du composé MD 360349 avec un rendement de 52 % (mode opératoire identique au stade 2 de l'exemple 15). IR (microcellule) νcm⁻¹ : 3440, 3360, 1510-1670, 1300-1000 ; RMN¹H ($CDCl_3$) δ ppm : 0,8-1,4 (6H) ; 2,05 (2H) ; 3-3,8 (5H) ; 3,8-4,4 (3H) ; 6,45-6,85 (4H).

De la même manière, on a obtenu à partir du composé MD 360364 : (hydroxy-3 butoxy)-4 aniline (MD 360365) : F : 90° C ; IR (KBr) νcm⁻¹ : 3350-3240, 3200, 1510, 1240 ; RMN¹H ($CDCl_3$) δppm : 1,25 (3H) ; 1,85 (2H) ; 3,1 (3H éch.) ; 4 (3H) ; 6,45-6,9 (4H).

Stade 3

[trifluoro-4,4,4 (éthoxy-1 éthoxy)-3 butoxy]-4 N-éthoxycarbonyl aniline (MD 360351)

Obtenue selon le mode opératoire du stade 2 de l'exemple 23. Rendement : 85 % ; huile ; IR (microcellule) νcm⁻¹ : 3320, 1710-1730 ; RMN¹H ($CDCl_3$) δppm : 0,8-1,4 (9H) ; 2,05 (2H) ; 3,45 (2H) ; 3,8-4,45 (5H) ; 4,8 (1H); 6,5 (1H éch.) ; 6,8 (2H) ; 7,25 (2H).

De la même manière, on a obtenu à partir de MD 360365 : (hydroxy-3 butoxy)-4 N-éthoxycarbonyl aniline (MD 360366) : F : 86° C ; IR (KBr) $\nu cm^{-1}$ : 3430-3300, 1705 ; RMN$^1$H ($CDCl_3$) $\delta$ppm : 1,2 (6H) ; 1,85 (2H) ; 2,3 (1H éch.) ; 3,8-4,4 (4H) ; 6,8 (3H dont 1 éch.) ; 7,25 (2H).

Stade 4

[ trifluoro-4,4,4 (éthoxy-1 éthoxy)-3 butoxy)-4 phényl]-3 méthoxyméthyl-5 (R) oxazolidinone-2 (MD 360352)

Obtenue suivant le mode opératoire du stade 3 de l'exemple 23. Huile ; RMN$^1$H ($CDCl_3$) $\delta$ppm : 0,8-1,4 (6H) ; 2,1 (2H) ; 3,4 (3H) ; 3,6 (2H) ; 3,8-4,4 (5H) ; 4,6-5 (2H) ; 6,8 (2H) ; 7,4 (2H).

De la même manière, à partir de MD 360366, on a obtenu MD 370284 ayant les mêmes caractéristiques physiques que celles indiquées à l'exemple 6.

Stade 5

[(trifluoro-4,4,4 hydroxy-3 butoxy)-4 phényl]-3 méthoxyméthyl-5 (R) oxazolidinone-2 (MD 230016)

Obtenue par traitement de MD 360352, selon le mode opératoire du stade 4 de l'exemple 4. Le produit est obtenu avec un rendement de 40 %. Ce composé possède les mêmes caractéristiques physiques que celles données à l'exemple 7.

**Exemple 22 :**

[(trifluoro-5,5,5 hydroxy-4 pentyl)-4 phényl]-3 méthoxyméthyl-5 (R) oxazolidinone-2
(numéro de code MD 360207)

Stade 1

trifluoro-4,4,4 iodo-1 butanol-3 (MD 360253)

A une solution de 2,04 g (0,0068 mole) de MD 370272 (trifluoro-4,4,4 tosyloxy-1 butanol-3) dans 20 ml d'acétone, on ajoute 2,56 g (0,0171 mole) de KI et chauffe à reflux une nuit. Après filtration et concentration, le produit est obtenu après chromatographie (silice, éluant : heptane 80, acétate d'éthyle 20). IR (microcellule) $\nu cm^{-1}$ : 3400 ; RMN$^1$H ($CDCl_3$) $\delta$ppm : 1,9-2,5 (2H) ; 2,4 (1H éch.) ; 3,35 (2H) ; 4,2 (1H).

Stade 2

iodure de trifluoro-4,4,4 hydroxy-3 butyl triphénylphosphonium (MD 360254)

On chauffe une nuit 35,6 g (0,14 mole) de MD 360253 et 36,8 g (0,14 mole) de triphénylphosphine dans le dioxane à reflux. Le produit est filtré et séché. Rendement : 72 % ; F : 159° C.

Stade 3

trifluoro-2,2,2 para-nitrocinnamyl-1 éthanol (MD 360255)

On chauffe à reflux une solution de 13 g (0,086 mole) de paranitrobenzaldéhyde, 55,57 g (0,108 mole) de MD 360254 et 44,78 g (0,324 mole) de $K_2CO_3$ pendant 4 heures. Le milieu réactionnel est versé dans l'eau et extrait à l'acétate d'éthyle. La phase organique est séchée sur $Na_2SO_4$ et concentrée. Le produit est obtenu après chromatographie (silice, éluant : $CH_2Cl_2$). Rendement : 73 % ; F : 71° C ; IR (KBr) $\nu cm^{-1}$ : 3480, 1595-1510, 1340 ; RMN$^1$H ($CDCl_3$) $\delta$ppm ; 2,6 (2H) ; 2,85 (1H éch.) ; 4,15 (1H) ; 6,5 (2H) ; 7,4 (2H) ; 8,1 (2H).

Stade 4

trifluoro-1,1,1 (amino-4 phényl)-5 pentanol-2 (MD 360256)

Obtenu par hydrogénation de MD 360255 selon le mode opératoire du stade 2 de l'exemple 12. Rendement : 78 % ; F : 90° C ; IR (KBr) $\nu cm^{-1}$ : 3420-3330, 3150, 1620, 1515, 1280, 1260, 1180, 1120 ; RMN$^1$H ($CDCl_3$) $\delta$ppm : 1,6 (4H) ; 2,5 (2H) ; 3,6 (3H) ; 4,6 (1H éch.) ; 6,6 (2H) ; 6,9 (2H).

Stade 5

[(trifluoro-5,5,5 hydroxy-4 pentyl)-4 phényl]-3 méthoxyméthyl-5 (R) oxazolidinone-2 (MD 360207)

Obtenue par le mode opératoire du stade 4 de l'exemple 17 : huile ; IR (microcellule) $\nu cm^{-1}$ : 3410, 1735 ; RMN[1]H ($CDCl_3$) δppm : 1 (4H) ; 2,7 (2H) ; 3,4 (3H) ; 3,6 (3H dont 1 éch.) ; 4 (1H) ; 4,7 (1H) ; 7,1 (2H) ; 7,4 (2H).

**Exemple 23 :**

(benzyloxy-4 phényl)-3 méthoxyméthyl-5 (R) oxazolidinone-2 (MD 200404)

Stade 1

méthoxyméthyl-4 dioxolane-1,3 one-2 (S) (numéro de code MD 360287)

On chauffe un mélange de 14 g (0,132 mole) de méthoxy-3 propane diol-1,2 (S) et de 31,16 g (0,264 mole) de carbonate de diéthyle en présence de 0,108 g d'hydrure de sodium à 50 % jusqu'à distillation de l'alcool formé. Lorsque la réaction est complète, le produit attendu est distillé $E_{b0,3}$ : 117° C ; Rendement : 93 % ; $[\alpha]_D^{20}$ : - 32,2° (C = 1, $CH_2Cl_2$) ; IR (microcellule) $\nu co$ : 1790 $cm^{-1}$ ; RMH[1]H ($CDCl_3$) δppm : 3,4 (3H) ; 3,6 (2H) 4,3-4,9 (3H).

Stade 2

N-éthoxycarbonyl benzyloxy-4 aniline (MD 360343)

A une solution de 10 g ($10^{-3}$ mole) de benzyloxy-4 aniline dans 90 ml de THF et 10 ml d'eau, on ajoute 6,3 g de bicarbonate de sodium, puis 5,28 ml ($55.10^{-3}$ mole) de chloroformiate d'éthyle. Après 18 heures d'agitation, le milieu réactionnel est filtré et concentré. Le résidu est repris dans l'acétate d'éthyle. La solution organique est lavée à l'eau, séchée sur $Na_2SO_4$ et concentrée. Le produit est obtenu avec un rendement de 91 % ; F : 98° C.; IR(KBr) $\nu cm^{-1}$ : 3320, 1700, 1510-1530, 1230 ; RMN[1]H ($CDCl_3$) δppm : 1,2 (3H) ; 4,2 (2H) ; 5 (2H) ; 6,7 (1H); 6,9 (2H) ; 7,2 (2H).

Stade 3

(benzyloxy-4 phényl)-3 méthoxyméthyl-5 (R) oxazolidinone-2 (MD 200404)

On chauffe à 160° C sous agitation 1 g ($3,6.10^{-3}$ mole) de MD 360343, 0,099 g ($0,72.10^{-3}$ mole) de $K_2CO_3$ et 0,586 g ($4,5.10^{-3}$ mole) de MD 360287 (obtenu au stade 1) pendant 3 heures. Après refroidissement, le milieu réactionnel est repris dans le chlorure de méthylène, lavé à l'eau, séché sur $Na_2SO_4$ et concentré. Le produit est recristallisé dans l'isopropanol. Rendement : 71 %. Mêmes caractéristiques physiques que celles données à l'exemple 2.

**Exemple 24 :**

[[(phénylméthyl-2 dioxolane-1,3 yl-2)-3 propyl]-4 phényl]-3 méthoxyméthyl-5 (R) oxazolidinone-2 (numéro de code 360334)

Stade 1

[(phénylméthyl)-2 dioxolane-1,3 yl-2]-2 éthanol
(numéro de code 360370)

Obtenu selon la méthode décrite au stade 2 de l'exemple 4, à partir de l'ester éthylique de l'acide [-(phénylméthyl)-2 dioxolane-1,3 yl-2] acétique (Synthesis 451, 1982) : IR (microcellule) $\nu cm^{-1}$ : 3440-3400 ; RMN[1]H ($CDCl_3$) δppm : 1,9 (2H) ; 2,8 (3H dont 1 éch.) ; 3,5 - 4 (6H ; 7,2 (5H).

Stade 2

(phénylméthyl)-2 (bromo-2 éthyl)-2 dioxolane-1,3
(numéro de code 360371)

A une solution de 37,8 g (0,181 mole) de 360370 dans 200 ml de $CH_2Cl_2$, on ajoute 120,4 g (0,363 mole) de $CBr_4$, puis petit à petit 95,2 g (0,363 mole) de triphénylphosphine, puis le milieu réactionnel est

agité à température ambiante pendant 1/2 heure . Après filtration, la phase organique est concentrée. Rendement : 81 % ; IR (microcellule) $\nu cm^{-1}$ : 3020, 2960, 2880, 1605 ; RMN$^1$H ($CDCl_3$) $\delta$ppm : 2,2 (2H) ; 2,8 (2H) ; 3,4 (2H) ; 3,8 (4H) ; 7,2 (5H).

Stade 3

Bromure de [[(phénylméthyl)-2 dioxolane-1,3 yl-2]-2 éthyl] triphénylphosphonium
(numéro de code 360372)

A une solution de 33 g (0,1217 mole) de 360371 dans 200 ml de dioxane, on ajoute 31 g (0,1217 mole) de triphénylphosphine et chauffe le mélange 20 heures. Après refroidissement, le précipité est filtré et lavé au dioxane et à l'éther éthylique. Rendement : 81 % ; F = 225° C ; RMN$^1$H ($CDCl_3$) $\delta$ppm : 1,6-2,2 (2H) ; 3 (2H) ; 3,2-4,2 (6H) ; 7,2 (5H) ; 7,5-7,9 (15H).

De la même manière ont été obtenus :

- le bromure de [(phényl-2 dioxolane-1,3-yl-2)-2 éthyl] triphénylphosphonium [F : 228° C ; RMN$^1$H ($CDCl_3$) $\delta$ppm : 2-2,5 (2H) ; 3,4-4,4 (6H) ; 7,4 (5H) ; 7,6-8 (15 H)] à partir du phényl-2 (bromo-2 éthyl)-2 dioxolane-1,3 (Tétrahedron Letters, 1987, 28, 1397).
- le bromure de [cyclohexyl-2 dioxolane-1,3-yl-2)-2 éthyl] triphénylphosphonium, à partir du cyclohexyl-2 (bromo-2 éthyl)-2 dioxolane-1,3 (ex. 27) ; Liq., IR (microcellule) $\nu cm^{-1}$ : 2920-2850, 1440-1110 ; RMN$^1$H ($CDCl_3$) $\delta$ppm : 0,9-2,3 (13H) ; 3-4,2 (6H) ; 7,6-8 (15H).

Stade 4

(Para-nitrocinnamyl)-2 (phénylméthyl)-2 dioxolane-1,3
(numéro de code 360373)

Obtenu selon le mode opératoire du stade 3 de l'exemple 22 : liquide ; IR (microcellule) $\nu cm^{-1}$ : 1595, 1510, 1340 ; RMN$^1$H ($CDCl_3$) $\delta$ppm : 2,6 (2H) ; 3 (2H) ; 3,9 (4H) ; 5,8-6,8 (2H) ; 7,3 (5H) ; 7,4 (2H) ; 8,2 (2H).

De la même façon ont été obtenus :

- (para-nitrocinnamyl)-2 phényl-2 dioxolane-1,3
  (numéro de code 360384)
  IR (microcellule) $\nu cm^{-1}$ : 1595, 1510, 1340 ; RMN$^1$H ($CDCl_3$) $\delta$ppm : 2,8-3 (2H) ; 3,6-4,2 (4H) ; 5,6-6,8 (2H) ; 7,15-7,65 (7H) ; 8,1 (2H) ; F = 82° C ;
- (para-nitrocinnamyl)-2 cyclohexyl-2 dioxolane-1,3
  (numéro de code 360 416)
  IR (microcellule) $\nu cm^{-1}$ : 2920-2850, 1595-1510, 1390 ; RMN$^1$H ($CDCl_3$) $\delta$ppm : 0,8-2,1 (11H) ; 2,5-2,8 (2H) ; 4 (4H) ; 5,7-6,7 (2H) ; 7,45 (2H) ; 8,2 (2H).

Stade 5

[(amino-4 phényl)-3 propyl]-2 (phénylméthyl)-2 dioxolane-1,3
(numéro de code 360374)

Obtenu selon le mode opératoire du stade 4 de l'exemple 22 :
F : 55° C ; IR (KBr) $\nu cm^{-1}$ :
3450-3360, 1620-1510 ; RMN$^1$H ($CDCl_3$) $\delta$ ppm : 1,65 (4H) ; 2,45 (2H) ; 2,85 (2H) ; 3,45 (2H éch.) ; 3,45-4 (4H) ; 6,5 (2H) ; 6,9 (2H) ; 7,2 (5H).

De la même manière ont été obtenus :

- [(amino-4 phényl)-3 propyl]-2 phényl-2 dioxolane-1,3
  (numéro de code 360385)
  F = 68° C ; IR (KBr) $\nu cm^{-1}$ : 3440-3360, 1630-1610, 1515; RMN$^1$H ($CDCl_3$) $\delta$ppm : 1,4-2,2 (4H) ; 2,4 (2H) ; 3,45 (2H) ; 3,6-4,15 (4H) ; 6,5 (2H) ; 6,9 (2H) ; 7,2-7,6 (5H) ;
- [(amino-4 phényl)-3 propyl]-2 cyclohexyl-2 dioxolane-1,3
  (numéro de code 360417)
  IR (microcellule) $\nu cm^{-1}$ : 3440-3360, 1625 ; RMN$^1$H ($CDCl_3$) $\delta$ppm : 0,8-2 (11H) ; 2,4 (2H) ; 3,45 (2H éch.) ; 3,8 (4H) ; 6,5 (2H) ; 6,9 (2H).

Stade 6

[[(éthoxycarbonylamino)-4 phényl]-3 propyl]-2 (phénylméthyl)-2 dioxolane-1,3
(numéro de code 360375)

Obtenu selon le mode opératoire du stade 2 de l'exemple 23. RMN$^1$H ($CDCl_3$) δppm : 1,25 (3H) ; 1,65 (4H) ; 2,5 (2H) ; 2,85 (2H) ; 3,7 (4H) ; 4,2 (2H) ; 6,9 (1H éch.) ; 7-7,4 (9H).

De la même manière ont été obtenus :

- [[(éthoxycarbonylamino)-4-phényl]-3 propyl]-2 phényl-2 dioxolane-1,3 (numéro de code 360386) RMN$^1$H ($CDCl_3$) δppm : 1,25 (3H) ; 1,4-2,2 (4H) ; 2,5 (2H) ; 3,5-4 (4H) ; 4,2 (2H) ; 6,6-7,6 (10H dont 1 éch.) ; F = 66° C.
- [[(éthoxycarbonylamino)-4 phényl]-3 propyl]-2 cyclohexyl-2 dioxolane-1,3 (numéro de code 360420) F = 70° C ; IR (KBr) $\nu cm^{-1}$ : 3360, 1705

Stade 7

[[(phénylméthyl-2 dioxolane-1,3 yl-2)-3 propyl]-4 phényl]-3 méthoxyméthyl-5 (R) oxazolidinone-2 (numéro de code 360334)

Obtenu selon le mode opératoire du stade 3 de l'exemple 23. RMN$^1$H ($CDCl_3$) δppm : 1,6 (4H) ; 2,5 (2H) ; 2,85 (2H) ; 3,4 (3H) ; 3,45-4,2 (8H) ; 4,65 (1H) ; 6,9-7,6 (9H) ; $[\alpha]_D^{20}$ : -33,2° (C = 1, $CH_2Cl_2$)

De la même manière ont été obtenus :

- [[(phényl-2 dioxolane-1,3 yl-2)-3 propyl]-4 phényl]-3 méthoxyméthyl-5 (R) oxazolidinone-2 (numéro de code 360332) RMN$^1$H ($CDCl_3$) δppm : 1,4-2,1 (4H) ; 2,55 (2H) ; 3,4 (3H) ; 3,6 (2H) ; 3,6-4,2 (6H) ; 4,65 (1H) ; 6,95-7,95 (IR (KBr) $\nu cm^{-1}$): 1750 ; $[\alpha]_D^{20}$ = - 31,9° (C = 1, $CH_2Cl_2$)
- [[(cyclohexyl-2 dioxolane-1,3 yl-2)-3 propyl]-4 phényl]-3 méthoxyméthyl-5 (R) oxazolidinone-2 (MD 360354) : IR (KBr) $\nu cm^{-1}$ : 1740-1730 ; F : 86° C.

**Exemple 25 :**

[(phényl-5 oxo-4 pentyl)-4 phényl]-3 méthoxyméthyl-5 (R) oxazolidinone-2 (MD 360394)

Obtenu à partir de MD 360334 (exemple 24) soumis au mode opératoire de l'exemple 9. Rendement : 58 % ; F : 105° C ; $[\alpha]_D^{20}$ : - 35,9° (C = 1, $CH_2Cl_2$) ; RMN$^1$H ($CDCl_3$) δppm : 1,9 (2H) ; 2,5 (4H) ; 3,4 (3H) ; 3,6 (2H) ; 3,65 (2H) ; 3,95 (2H) ; 4,7 (1H) ; 7,1 (2H) ; 7,25 (5H) ; 7,45 (2H) ; IR (KBr) $\nu cm^{-1}$ : 1740, 1710.

De la même manière on obtient, à partir des dioxolanes correspondants :

- [(phényl-4 oxo-4 butyl)-4 phényl]-3 méthoxyméthyl-5 (R) oxazolidinone-2 (MD 360401) ; IR (microcellule) $\nu cm^{-1}$ : 1750, 1735 ; RMN$^1$H ($CDCl_3$) δppm : 2,05 (2H ) ; 2,5-3 (4H) ; 3,4 (3H) ; 3,6 (2H) ; 3,8-4,2 (2H) ; 4,65 (1H) ; 7-7,6 (7H) ; 7,9 (2H) ; F = 83° C : $[\alpha]_D^{20}$ = - 36,3° (C = 1, $CH_2Cl_2$).
- [(cyclohexyl-4 oxo-4 butyl)-4 phényl]-3 méthoxyméthyl-5 (R) oxazolidinone-2 (MD 360399) F = 86° C : $[\alpha]_D^{20}$ = - 35,4° (C = 1, $CH_2Cl_2$)

**Exemple 26 :**

[(phényl-5 hydroxy-4 pentyl)-4 phényl]-3 méthoxyméthyl-5 (R) oxazolidinone-2 (MD 360395)

Obtenue à partir de MD 360394 soumis au mode opératoire de l'exemple 14. RMN$^1$H ($CDCl_3$) δppm : 1,6-1,9 (5H dont 1 éch.) ; 2,7 (4H) ; 3,4 (3H) ; 3,6 (2H) ; 3,7-4,2 (3H) ; 4,7 (1H) ; 7-7,5 (9H) ; F = 72° C.

De la même façon on obtient à partir des cétones correspondantes :

- [(phényl-4 hydroxy-4 butyl)-4 phényl]-3 méthoxyméthyl-5 (R) oxazolidinone-2 (MD 360402) : IR (microcellule) $\nu cm^{-1}$ : 3450, 1750 ; RMN$^1$H ($CDCl_3$) δppm : 1,6-1,8 (4H) ; 2,6 (2H) ; 3,4 (3H) ; 3,6 (2H) ; 3,7-4,2 (2H) ; 4,4-4,8 (2H) ; 7-7,4 (9H).
- [(cyclohexyl-4 hydroxy-4 butyl)-4 phényl]-3 méthoxyméthyl-5 (R) oxazolidinone-2

**Exemple 27 :**

cyclohexyl-2 (bromo-2 éthyl)-2 dioxolane-1,3 (numéro de code 360414)

Stade 1

bromo-3 cyclohexyl-1 propanone

Dans une solution de cyclohexyl-1 one-1 propène-2 (0,255 mole) dans 200 ml de $CH_2Cl_2$, refroidie à 10-15° C, on fait barboter HBr gazeux. Quand la réaction est achevée, on lave le milieu réactionnel avec

une solution aqueuse saturée de $NaHCO_3$, sèche sur $Na_2SO_4$ et concentre pour obtenir le produit attendu sous la forme d'une huile.

Stade 2

cyclohexyl-2 (bromo-2 éthyl)-2 dioxolane-1,3

On porte au reflux, avec élimination de l'eau formée, une solution du composé obtenu au stade précédent (0,223 mole) dans 600 ml de benzène, cette solution comprenant en outre 0,58 mole d'éthylène glycol et 2,5 g d'acide para-toluène sulfonique. Après 3 heures 30 mn de réaction, on verse dans une solution saturée de NaCl, sèche la phase organique sur $Na_2SO_4$, concentre et purifie par chromatographie (silice, éluant : heptane 60 - $CH_2Cl_2$ 40).

**Exemple 28 :**

[(trifluoro-4,4,4 méthoxy-3 (R) butoxy)-4 phényl]-3 méthoxyméthyl-5 (R) oxazolidinone-2 (numéro de code MD 360316)

A une solution de $2,29.10^{-2}$ mole de MD 370503 (exemple 7) dans 80 ml de toluène, on ajoute $2,29.10^{-3}$ mole de bromure de tertiobutylammonium, $6,8.10^{-2}$ mole de sulfate de méthyle et 7,3 ml de NaOH aqueuse à 50 %. Le milieu réactionnel est agité une heure et versé sur de l'acétate d'éthyle. La phase organique est lavée à l'eau, séchée sur $Na_2SO_4$ et concentrée. Le produit est obtenu après chromatographie (silice, éluant : acétate d'éthyle 40/heptane 60) avec un rendement de 86 %. $[\alpha]_D^{20}$ : + 1,2° (C = 1, $CH_2Cl_2$) ; IR (microcellule) $\nu cm^{-1}$ : 1750 ; RMN$^1$H ($CDCl_3$) $\delta$ppm : 1,8-2,3 (2H) ; 3,4 (3H) ; 3,5 (3H) ; 3,6 (2H) ; 3,8-4,3 (5H) ; 4,7 (1H) ; 6,9 (2H) ; 7,45 (2H).

De la même manière a été obtenue la [(trifluoro-4,4,4 benzyloxy-3 (R) butoxy)-4 phényl]-3 méthoxyméthyl-5 (R) oxazolidinone-2 (numéro de code MD 360317) : $[\alpha]_D^{20}$ : + 49° (C = 1, $CH_2Cl_2$) ; IR (microcellule) $\nu cm^{-1}$ : 1750 . RMN$^1$H ($CDCl_3$) $\delta$ppm : 1,8-2,2 (2H) ; 3,3 (3H) ; 3,6 (2H) ; 3,7-4,1 (5H) ; 4,3-4,9 (3H) ; 6,7 (2H) ; 7,1 (5H) ; 7,3 (2H).

**Exemple 29 :**

Mélange racémique de [(trifluoro-4,4,4 hydroxy-3 butoxy)-4 phényl]-3 méthoxyméthyl-5 oxazolidinone-2 (numéro de code 370167)

Obtenu selon le mode opératoire de l'exemple 5. F = 89° C ; RMN$^1$H ($CDCl_3$) $\delta$ppm : 2,05 (2H) ; 3,4 (3H) ; 3,5 (1H) ; 3,6 (2H) ; 4 (5H) ; 4,7 (1H) ; 6,8 (2H) ; 7,3 (2H).

Les dérivés de formule (I) ont été étudiés chez l'animal de laboratoire et ont montré des activités pharmacologiques notamment dans le domaine psychotrope, en particulier comme anxiolytiques et antidépresseurs potentiels.

L'activité antidépressive a été mise en évidence par le test de potentialisation du 5-HTP chez le rat selon le protocole décrit par M. JALFRE, B. BUCHER, A. COSTON, G. MOCQUET et R.D. PORSOLT : Arch. Int. Pharmacodyn. (1982), 259, 194-221. On détermine chez le rat la dose de produit qui, administrée par voie orale, provoque chez 50 % des animaux ($DE_{50}$) l'apparition de tremblements généralisés ou des stéréotypies (pianotage, mouvements de tête) consécutifs à l'administration par voie intrapéritonéale 1 h après le premier traitement d'hydroxy-5 tryptophane (5-HTP). Les résultats obtenus avec certains composés selon l'invention dans le test précédent sont rassemblés, à titre d'exemple dans le tableau ci-après qui donne par ailleurs la toxicité aiguë ($DL_{50}$) de certains des composés testés et qui est estimée chez la souris selon la méthode de J.T. LITCHFIELD et F. WILCOXON (J. Pharmacol. Exp. Ther., (1949), 96, 99.

TABLEAU

| COMPOSE TESTE NUMERO DE CODE | $DE_{50}$ MG/KG | $DL_{50}$P.O.MG/KG |
|---|---|---|
| MD230197 | 2,4 | |
| MD230201 | 1,6 | |
| MD230238 | 1,9 | |
| MD370503 | 0,14 | 2500 |
| MD370120 | 0,83 | 1400 |
| MD370122 | 0,68 | |
| MD370504 | 1,8 | |
| MD370047 | 1,9 | |
| MD370167 | 1,1 | |
| MD230082 | 1,1 | |
| TOLOXATONE | 30 | |

Les résultats qui précèdent montrent que les composés objet de la présente demande peuvent être utilisés pour la préparation de médicaments psychotropes et notamment d'anxiolytiques et d'antidépresseurs potentiels, ces médicaments trouvant leur emploi en thérapeutique, notamment pour le traitement des états dépressifs endogène et exogène.

Ces médicaments peuvent être administrés à l'homme ou à tout animal à sang chaud, sous diverses formes pharmaceutiques bien connues dans la technique et notamment sous la forme de compositions formulées en vue de leur administration par voie orale, injectable ou rectale.

Pour l'administration par voie orale, lesdites compositions peuvent prendre la forme de comprimés, dragées ou gélules préparées par les techniques habituelles utilisant des supports et excipients connus tels que des agents liants, des charges, des lubrifiants et des agents de désintégration ; elles peuvent également prendre la forme de solutions, sirop ou suspensions.

Pour l'administration sous forme de soluté injectable, les compositions selon l'invention peuvent prendre la forme de solutions, suspensions ou émulsions injectables comprenant un véhicule liquide huileux ou aqueux acceptable.

Pour l'administration par voie rectale, les compositions peuvent prendre la forme de suppositoire comprenant les bases habituelles pour suppositoires.

La dose thérapeutique active des principes actifs, c'est-à-dire des composés (I) et leurs sels pharmaceutiquement acceptables, dépend notamment de la voie d'administration, du poids corporel du patient et de la puissance thérapeutique des principes actifs mis en oeuvre.

Généralement, par voie orale, les doses administrées pourront atteindre 10 mg/kg/jour de principe actif (en une ou plusieurs prises) ; par voie injectable, elles pourront atteindre 1 mg/kg/jour (en une ou plusieurs prises) ; par voie rectale, elles pourront atteindre 5 mg/kg/jour de principe actif (en un ou plusieurs suppositoires).

**Revendications**

**Revendications pour les Etats contractants suivants : AT, BE, CH, DE, DK, FR, GB, IT, LI, LU, NL, SE**

**1.** Dérivés répondant à la formule :

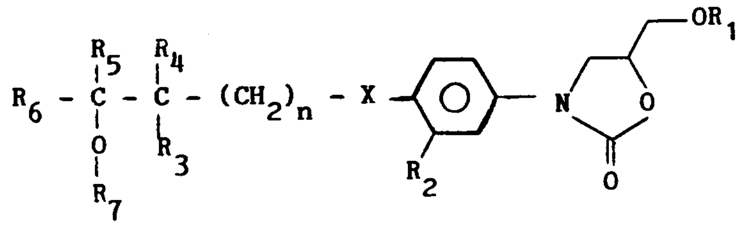

(I)

où :

- $R_1$ représente H ou alkyle en $C_1$-$C_4$ ;

- X représente soit un atome d'oxygène auquel cas $R_2$ = H ou halogène, soit un groupe méthylène ou un groupe -CH=CH-, auquel cas $R_2$ = H ;
- n prend la valeur 1 ou 2 quand X est un atome d'oxygène ou un groupe méthylène et la valeur 0 ou 1 quand X représente le groupe -CH=CH- ;
- $R_3$ et $R_4$ représentent chacun indépendamment l'un de l'autre, H, alkyle en $C_1$-$C_4$ ou cycloalkyle en $C_4$-$C_7$;
- $R_5$ représente H et $R_6$ un groupe $CF_3$ ou cycloalkyle en $C_4$-$C_7$ ou bien $R_5$ est un groupe alkyle en $C_1$-$C_4$ et $R_6$ un groupe alkyle en $C_1$-$C_4$ ou cycloalkyle en $C_4$-$C_7$;
- $R_4$ et $R_6$ peuvent en outre former ensemble une chaîne $-(CH_2)_3-$ ou $-(CH_2)_4-$ ;
- $R_5$ et $R_6$ peuvent en outre former ensemble une chaîne $-(CH_2)_4-$ ou $-(CH_2)_5-$ ; et
- $R_7$ représente H, alkyle en $C_1$-$C_4$ ou benzyle,

ces dérivés se présentant sous la forme de diastéréoisomères ou d'énantiomères ou sous forme cis ou trans ou encore sous la forme d'un mélange de toutes ces formes, y compris les formes racémiques.

**2.** Dérivés selon la revendication 1, pour lesquels $R_1$ = H ou $CH_3$ ; $R_2$ = H ; X = oxygène ou $CH_2$ ; n = 1 ou 2 ; $R_3$ et $R_4$ représentent H ou $CH_3$ ; $R_5$ = H ; $R_6$ représente $CF_3$ ; et $R_7$ représente H ou $CH_3$.

**3.** Dérivés selon la revendication 1, pour lesquels $R_1$ = H ou $CH_3$ ; $R_2$ = H ; X = oxygène ou $CH_2$ ; n = 1 ou 2 ; $R_3$ et $R_4$ représentent H ou $CH_3$ ; $R_5$ et $R_6$ = $CH_3$ ; et $R_7$ = H ou $CH_3$.

**4.** Dérivés selon la revendication 1, pour lesquels $R_1$ = $CH_3$ ; $R_2$ = H ; X = oxygène ; n = 1 ou 2 ; $R_3$ = $R_4$ = $R_5$ = H ; $R_6$ = $CF_3$ ; et $R_7$ = H.

**5.** Dérivés selon la revendication 1, pour lesquels $R_1$ = $CH_3$ ; $R_2$ = H ; X = méthylène ; n = 1 ou 2 ; $R_3$ = $R_4$ = $R_5$ = H ; $R_6$ = $CF_3$ ; et $R_7$ = H.

**6.** Dérivés selon la revendication 1, pour lesquels $R_1$ = $CH_3$ ; $R_2$ = H ; X représente CH = CH ; n = 0 ou 1 ; $R_3$ = $R_4$ = $R_5$ = H ; $R_6$ = $CF_3$ et $R_7$ = H.

**7.** Dérivé selon la revendication 1, pour lequel $R_1$ = $CH_3$, $R_2$ = $R_3$ = $R_4$ = $R_5$ = $R_7$ = H, n = 1, $R_6$ = $CF_3$ et X = oxygène.

**8.** Dérivé selon la revendication 7, pour lequel les deux atomes de carbone asymétrique sont de configuration (R).

**9.** Composition pharmaceutique, caractérisée en ce qu'elle comprend un excipient physiologiquement acceptable en association avec au moins un dérivé (I) selon l'une quelconque des revendications 1 à 8.

**10.** Utilisation des dérivés selon les revendications 1 à 8, pour la préparation de médicaments psychotropes.

**11.** Procédé de préparation des dérivés de formule (I) selon la revendication 1, caractérisé en ce qu'il comprend :

a) l'O.alcoylation des composés de formule :

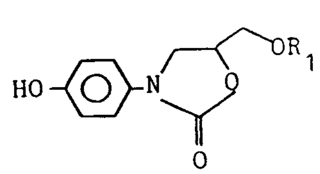

où $R_1$ a la même signification que dans la formule (I), par un composé de formule :

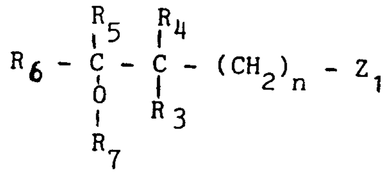

où $Z_1$ = OTs, OMs ou halogène ; $R_3$ = $R_4$ = H : et n, $R_5$, $R_6$ et $R_7$ ont la même signification que dans la formule (I) ;

b) la condensation du composé de formule :

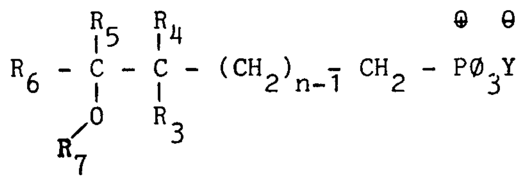

où Y = halogène et $R_3$ à $R_7$ et n ont la même signification que dans la formule (I), sur les composés de formule :

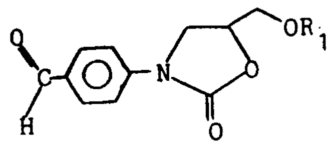

où $R_1$ = alkyle en $C_1$-$C_4$ ;

c) l'hydrogénation de la double liaison des composés de formule :

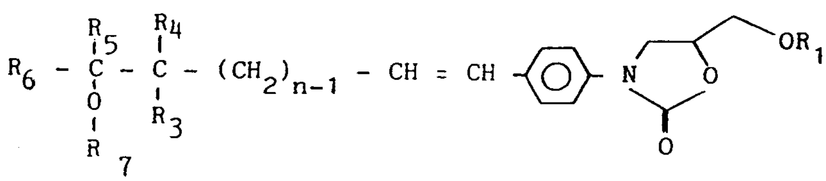

où n et $R_3$ à $R_7$ ont la même signification que dans la formule (I) ;

d) la réduction en

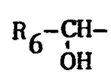 de 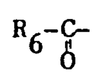

des composés de formule :

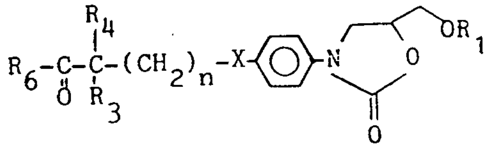

où X, n, $R_1$, $R_3$, $R_4$ et $R_6$ ont la même signification que dans la formule (I), $R_6$ ne pouvant toutefois représenter $CF_3$,

e) la condensation d'un carbonate d'alkyle en $C_1$-$C_4$ sur les composés de formule :

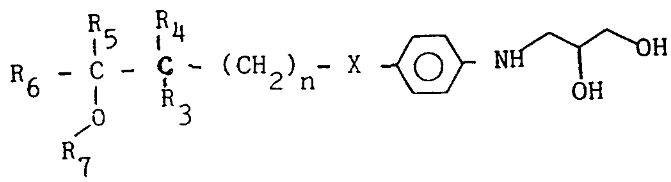

où n, X et $R_3$ à $R_7$ ont la même signification que dans la formule (I);

f) l'alcoylation par un halogénure d'alkyle en $C_1$-$C_4$ des composés de formule :

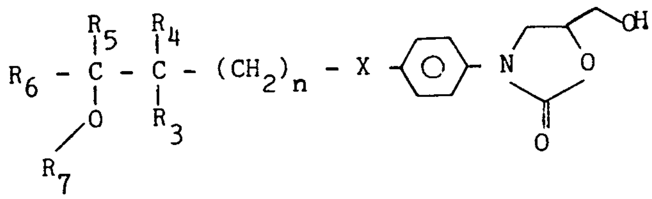

où n, X et $R_3$ à $R_7$ ont la même signification que dans la formule (I);

g) la condensation sur les composés de formule :

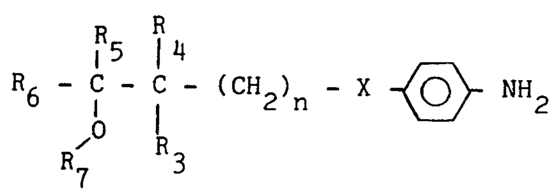

où n, X et $R_3$ à $R_7$ ont la même signification que dans la formule (I), d'un composé de formule :

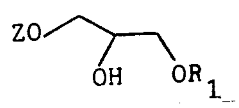

où $R_1$ = alkyle en $C_1$-$C_4$ et Z = Ts ou Ms ;

h) la condensation sur les composés de formule :

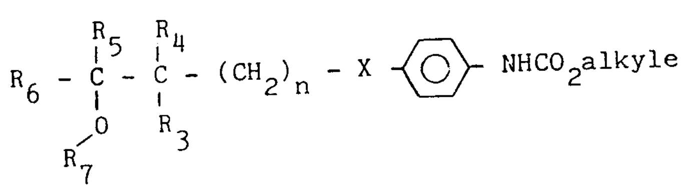

où n, X et $R_3$ à $R_7$ ont la même signification que dans la formule (I), d'un composé de formule :

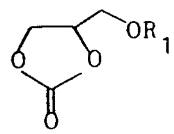

ou

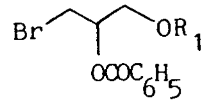

où $R_1$ = alkyle en $C_1$-$C_4$ ;

i) l'hydrolyse par un acide aqueux du composé de formule :

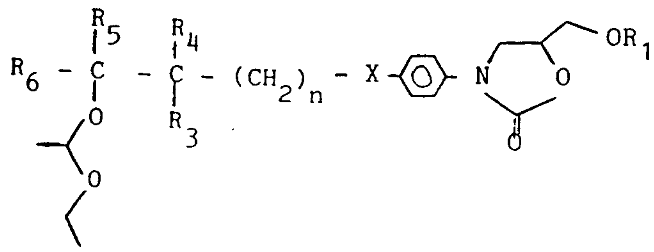

où $R_1$ = alkyle en $C_1$-$C_4$ et n, X et $R_3$ à $R_6$ ont la même signification que dans la formule (I) ;

j) la désacylation par hydrolyse basique des composés de formule :

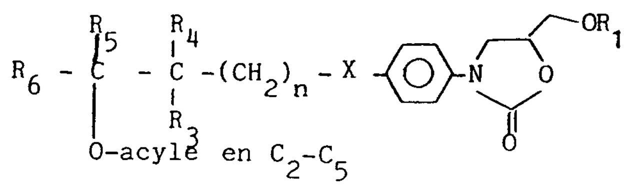

où les divers paramètres ont le même signification que dans la formule (I) ; ou

k) l'O.alcoylation par un halogénure ou sulfate d'alkyle en $C_1$-$C_4$ ou un halogénure de benzyle, des composés de formule :

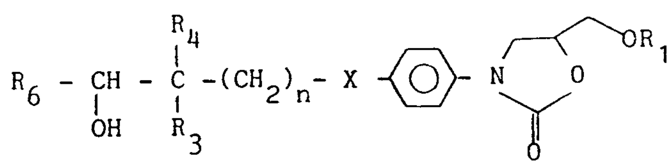

où $R_1$ = alkyle en $C_1$-$C_4$ et X, n, $R_3$, $R_4$ et $R_6$ ont la même signification que dans la formule I ;

les réactions a) à k) ci-dessus étant éventuellement suivies de l'action d'un halogène sur les composés obtenus par ces réactions.

**Revendications pour les Etats contractants suivants : ES, GR**

**1.** Procédé de préparation des dérivés répondant à la formule :

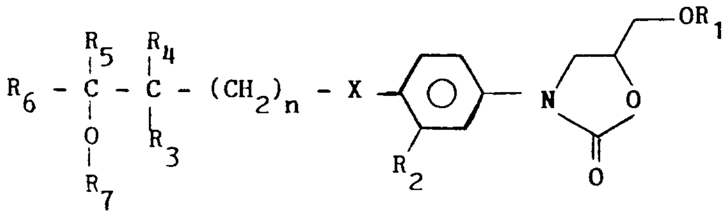 (I)

où :

- $R_1$ représente H ou alkyle en $C_1$-$C_4$ ;
- X représente soit un atome d'oxygène auquel cas $R_2$ = H ou halogène, soit un groupe méthylène ou un groupe -CH=CH-, auquel cas $R_2$ = H ;
- n prend la valeur 1 ou 2 quand X est un atome d'oxygène ou un groupe méthylène et la valeur 0 ou 1 quand X représente le groupe -CH=CH- ;
- $R_3$ et $R_4$ représentent chacun indépendamment l'un de l'autre, H, alkyle en $C_1$-$C_4$ ou cycloalkyle en $C_4$-$C_7$;
- $R_5$ représente H et $R_6$ un groupe $CF_3$ ou cycloalkyle en $C_4$-$C_7$ ou bien $R_5$ est un groupe alkyle en $C_1$-$C_4$ et $R_6$ un groupe alkyle en $C_1$-$C_4$ ou cycloalkyle en $C_4$-$C_7$;
- $R_4$ et $R_6$ peuvent en outre former ensemble une chaîne $-(CH_2)_3-$ ou $-(CH_2)_4-$ ;
- $R_5$ et $R_6$ peuvent en outre former ensemble une chaîne $-(CH_2)_4-$ ou $-(CH_2)_5-$ ; et
- $R_7$ représente H, alkyle en $C_1$-$C_4$ ou benzyle,

ces dérivés se présentant sous la forme de diastéréoisomères ou d'énantiomères ou sous forme cis ou trans ou encore sous la forme d'un mélange de toutes ces formes, y compris les formes racémiques, caractérisé en ce qu'il comprend :

a) l'O.alcoylation des composés de formule :

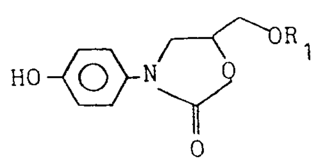

où $R_1$ a la même signification que dans la formule (I), par un composé de formule :

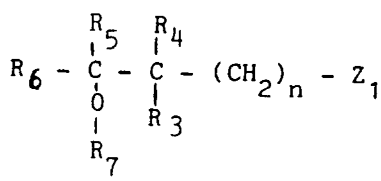

où $Z_1$ = OTs, OMs ou halogène ; $R_3$ = $R_4$ = H ; et n, $R_5$, $R_6$ et $R_7$ ont la même signification que dans la formule (I) ;

b) la condensation du composé de formule :

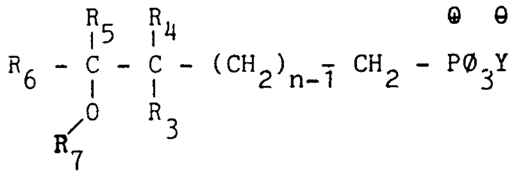

où Y = halogène et $R_3$ à $R_7$ et n ont la même signification que dans la formule (I), sur les composés de formule :

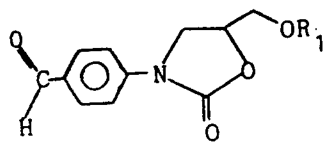

où $R_1$ = alkyle en $C_1$-$C_4$;

c) l'hydrogénation de la double liaison des composés de formule :

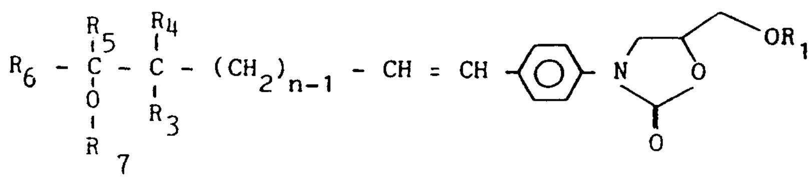

où n et $R_3$ à $R_7$ ont la même signification que dans la formule (I) ;

d) la réduction en

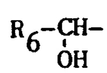 de 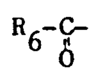

des composés de formule :

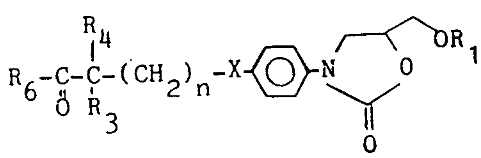

où X, n, $R_1$, $R_3$, $R_4$ et $R_6$ ont la même signification que dans la formule (I), $R_6$ ne pouvant toutefois représenter $CF_3$;

e) la condensation d'un carbonate d'alkyle en $C_1$-$C_4$ sur les composés de formule :

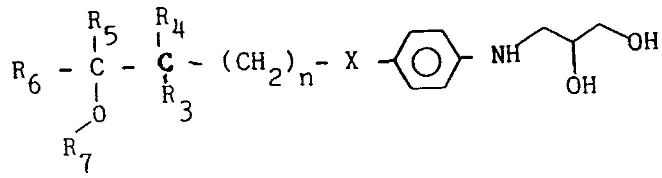

où n, X et $R_3$ à $R_7$ ont la même signification que dans la formule (I);

f) l'alcoylation par un halogénure d'alkyle en $C_1$-$C_4$ des composés de formule :

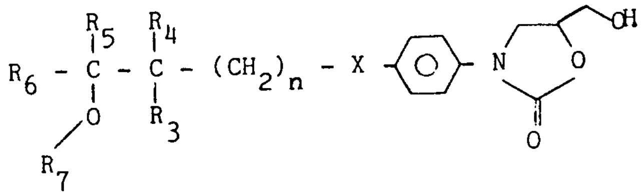

où n, X et $R_3$ à $R_7$ ont la même signification que dans la formule (I);

g) la condensation sur les composés de formule :

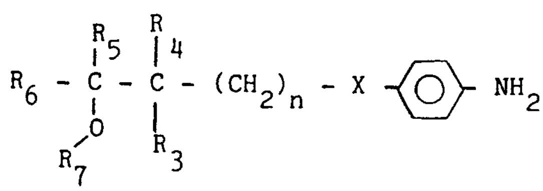

où n, X et $R_3$ à $R_7$ ont la même signification que dans la formule (I), d'un composé de formule :

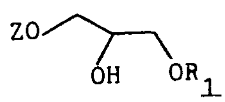

où $R_1$ = alkyle en $C_1$-$C_4$ et Z = Ts ou Ms ;

h) la condensation sur les composés de formule :

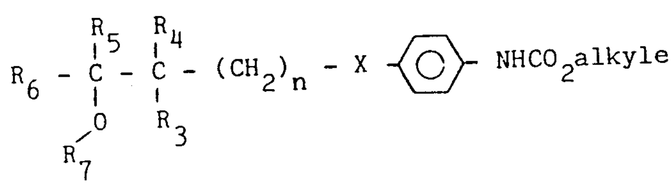

où n, X et $R_3$ à $R_7$ ont la même signification que dans la formule (I), d'un composé de formule :

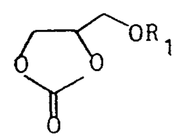 ou 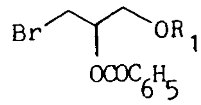

où $R_1$ = alkyle en $C_1$-$C_4$ ;

i) l'hydrolyse par un acide aqueux du composé de formule :

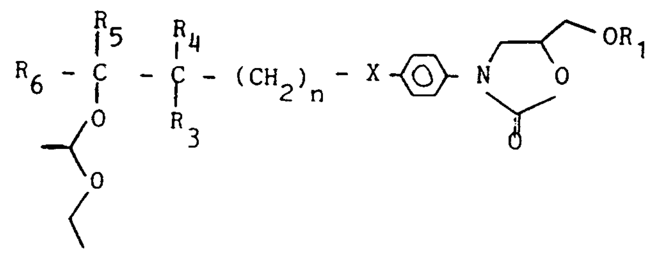

où $R_1$ = alkyle en $C_1$-$C_4$ et n, X et $R_3$ à $R_6$ ont la même signification que dans la formule (I) ;

j) la désacylation par hydrolyse basique des composés de formule :

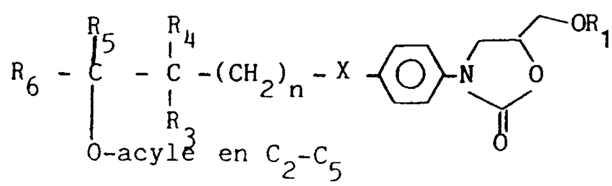

où les divers paramètres ont le même signification que dans la formule (I) ; ou

k) l'O.alcoylation par un halogénure ou sulfate d'alkyle en $C_1$-$C_4$ ou un halogénure de benzyle, des composés de formule :

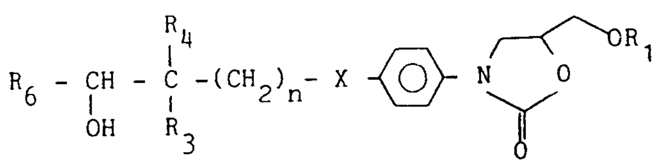

où $R_1$ = alkyle en $C_1$-$C_4$ et X, n, $R_3$, $R_4$ et $R_6$ ont la même signification que dans la formule I ;

les réactions a) à k) ci-dessus étant éventuellement suivies de l'action d'un halogène sur les composés obtenus par ces réactions.

2. Procédé selon la revendication 1, pour la préparation des dérivés de formule (I) pour lesquels $R_1$ = H ou $CH_3$ ; $R_2$ = H ; X = oxygène ou $CH_2$ ; n = 1 ou 2 ; $R_3$ et $R_4$ représentent H ou $CH_3$ ; $R_5$ = H ; $R_6$ représente $CF_3$ ; et $R_7$ représente H ou $CH_3$.

3. Procédé selon la revendication 1, pour la préparation des dérivés de formule (I) pour lequels $R_1$ = H ou $CH_3$ ; $R_2$ = H ; X = oxygène ou $CH_2$ ; n = 1 ou 2 ; $R_3$ et $R_4$ représentent H ou $CH_3$ ; $R_5$ et $R_6$ = $CH_3$ ; et $R_7$ = H ou $CH_3$.

4. Procédé selon la revendication 1, pour la préparation des dérivés de formule (I) pour lesquels $R_1$ = $CH_3$ ; $R_2$ = H ; X = oxygène ; n = 1 ou 2 ; $R_3$ = $R_4$ = $R_5$ = H ; $R_6$ = $CF_3$ ; et $R_7$ = H.

5. Procédé selon la revendication 1, pour la préparation des dérivés de formule (I) pour lesquels $R_1$ = $CH_3$ ; $R_2$ = H ; X = méthylène ; n = 1 ou 2 ; $R_3$ = $R_4$ = $R_5$ = H ; $R_6$ = $CF_3$ ; et $R_7$ = H.

6. Procédé selon la revendication 1, pour la préparation des dérivés de formule (I) pour lesquels $R_1$ = $CH_3$ ; $R_2$ = H ; X représente CH = CH ; n = 0 ou 1 ; $R_3$ = $R_4$ = $R_5$ = H ; $R_6$ = $CF_3$ et $R_7$ = H.

7. Procédé selon la revendication 1, pour la préparation du dérivé de formule (I) pour lequel $R_1$ = $CH_3$, $R_2$ = $R_3$ = $R_4$ = $R_5$ = $R_7$ = H, n = 1, $R_6$ = $CF_3$ et X = oxygène.

8. Procédé selon la revendication 7, pour la préparation du dérivé pour lequel les deux atomes de carbone asymétrique sont de configuration (R).

**Claims**

**Claims for the following Contracting States : AT, BE, CH, DE, DK, FR, GB, IT, LI, LU, NL, SE**

1. The derivatives of the formula :

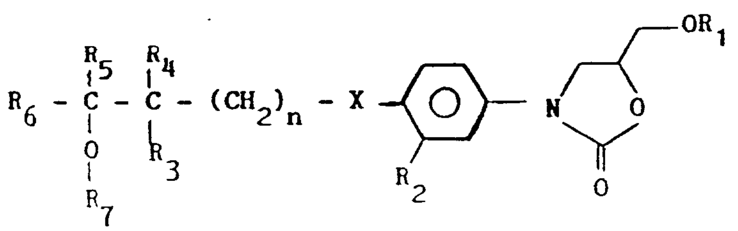

(I)

wherein :

- $R_1$ is H or $C_1$-$C_4$ alkyl ;
- X is either an oxygen atom, in which case $R_2$ = H or halogen, or a methylene group or a -CH=CH- group, in which case $R_2$ = H ;
- n is 1 or 2 when X is an oxygen atom or a methylene group and 0 or 1 when X is a -CH=CH- group ;
- each of $R_3$ and $R_4$ is independently H, $C_1$-$C_4$ alkyl,or $C_4$-$C_7$ cycloalkyl ;
- $R_5$ is H and $R_6$ is a $CF_3$ or $C_4$-$C_7$ cycloalkyl group or $R_5$ is a $C_1$-$C_4$ alkyl group and $R_6$ is a $C_1$-$C_4$ alkyl or $C_4$-$C_7$ cycloalkyl group ;
- $R_4$ and $R_6$ may further form together a $-(CH_2)_3-$ or $-(CH_2)_4-$ chain ;
- $R_5$ and $R_6$ may further form together a $-(CH_2)_4-$ or $-(CH_2)_5-$ chain ; and
- $R_7$ is H, $C_1$-$C_4$ alkyl or benzyl,

these derivatives being under the form of diastereoisomers or enantiomers or under the cis- or trans-form or under the form of a mixture of all these forms, including the racemic forms.

2. The derivatives according to Claim 1, wherein $R_1$ = H or $CH_3$ ; $R_2$ = H ; X = oxygen or $CH_2$ ; n = 1 or 2 ; $R_3$ and $R_4$ are H or $CH_3$ ; $R_5$ is H ; $R_6$ is $CF_3$ ; and $R_7$ is H or $CH_3$.

3. The derivatives according to claim 1, wherein $R_1$ = H or $CH_3$ ; $R_2$ = H ; X = oxygen or $CH_2$ ; n = 1 or 2 ; $R_3$ and $R_4$ are H or $CH_3$ ; $R_5$ and $R_6$ = $CH_3$ ; and $R_7$ = H or $CH_3$.

4. The derivatives according to claim 1, wherein $R_1$ = $CH_3$ ; $R_2$ = H ; X = oxygen ; n = 1 or 2 ; $R_3$ = $R_4$ = $R_5$ = H ; $R_6$ = $CF_3$ ; and $R_7$ = H.

5. The derivatives according to claim 1, wherein $R_1$ = $CH_3$ ; $R_2$ = H ; X = methylene ; n = 1 or 2 ; $R_3$ = $R_4$ = $R_5$ = H ; $R_6$ = $CF_3$ ; and $R_7$ = H.

6. The derivatives according to claim 1, wherein $R_1$ = $CH_3$ ; $R_2$ = H ; X = represents CH=CH ; n = 0 or 1 ; $R_3$ = $R_4$ = $R_5$ = H ; $R_6$ = $CF_3$ ; and $R_7$ = H.

7. The derivatives according to claim 1, wherein $R_1 = CH_3$ ; $R_2 = R_3 = R_4 = R_5 = R_7 = H$ ; n = 1 ; $R_6 = CF_3$ ; and X = oxygen.

8. The derivative according to claim 7, wherein the two asymmetric carbon atoms have (R) configuration.

9. A pharmaceutical composition, characterized in that it comprises a physiologically acceptable excipient in association with at least one derivative (I) according to any one of claims 1 to 8.

10. The use of the derivatives according to claims 1 to 8 for the preparation of psychotropic drugs.

11. A process for the preparation of derivatives of formula (I) according to claim 1, characterized in that it comprises :

a) O-alkylating compounds of formula :

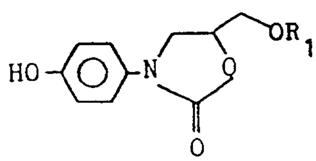

wherein $R_1$ has the same meaning as in formula (I), with a compound of formula :

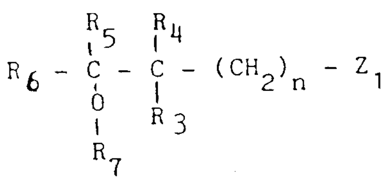

wherein :

- $Z_1$ is OTs, OMs or halogen ;
- $R_3 = R_4 = H$ ; and
- n, $R_5$, $R_6$ and $R_7$ have the same meaning as in formula (I) ;

b) condensing compound of formula :

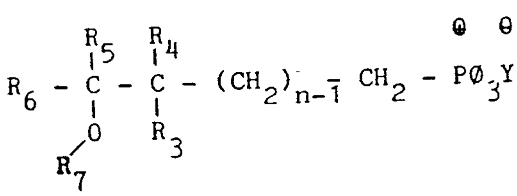

wherein :

- Y = halogen ; and
- $R_3$ to $R_7$ and n have the same meaning as in formula (I),

with compounds of formula :

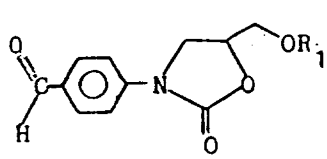

wherein $R_1$ = $C_1$-$C_4$ alkyl ;

c) hydrogenating the double bond of compounds of formula :

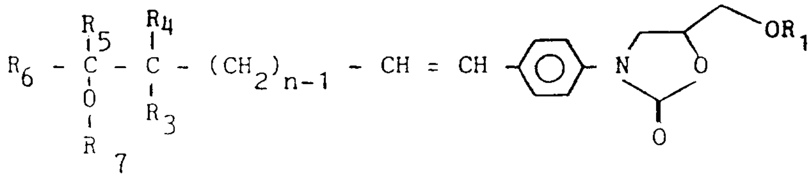

wherein n and $R_3$ to $R_7$ have the same meaning as in formula (I) ;

d) reducing to

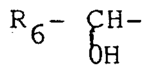 the 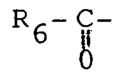

group of compounds of formula :

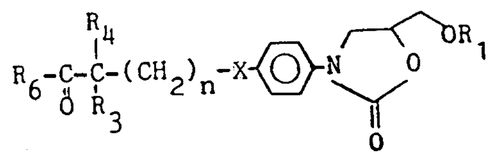

wherein X, n, $R_1$, $R_3$, $R_4$ and $R_6$ have the same meaning as in formula (I) except that $R_6$ cannot be $CF_3$ ;

e) condensing a $C_1$-$C_4$ alkyl carbonate with compounds of formula :

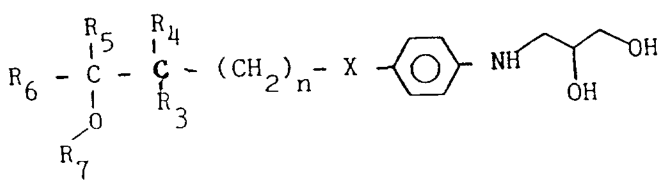

wherein n, X, and $R_3$ to $R_7$ have the same meaning as in formula (I) ;

f) alkylating with a $C_1$-$C_4$ alkyl halide compounds of formula :

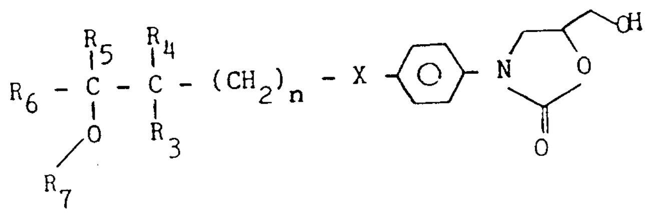

wherein n, X and $R_3$ to $R_7$ have the same meaning as in formula (I) ;

g) condensing with compounds of formula :

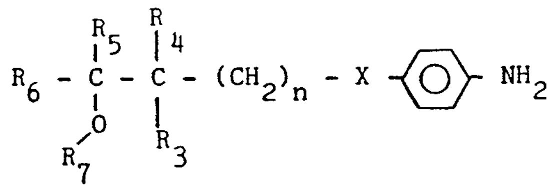

wherein n, X and $R_3$ to $R_7$ have the same meaning as in formula (I), a compound of formula :

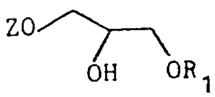

wherein $R_1$ = $C_1$-$C_4$ alkyl ; and Z = Ts or Ms ;

h) condensing with compounds of formula :

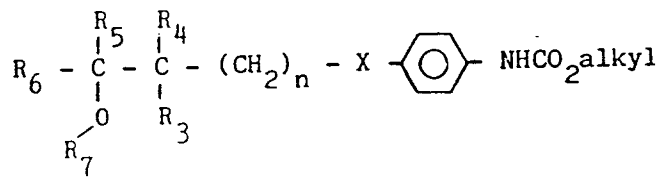

wherein n, X and $R_3$ to $R_7$ have the same meaning as in formula (I), a compound of formula :

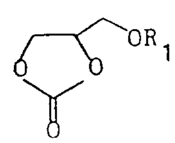

or

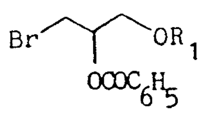

wherein $R_1$ = $C_1$-$C_4$ alkyl ;

i) hydrolyzing with an aqueous acid a compound of formula :

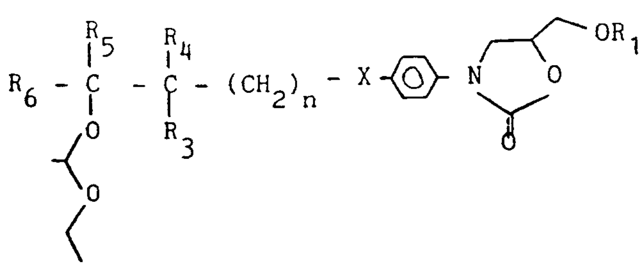

wherein $R_1$ = $C_1$- $C_4$ alkyl ; and n, X and $R_3$ to $R_6$ have the same meaning as in formula (I) ;

j) desacylating by basic hydrolysis compounds of formula :

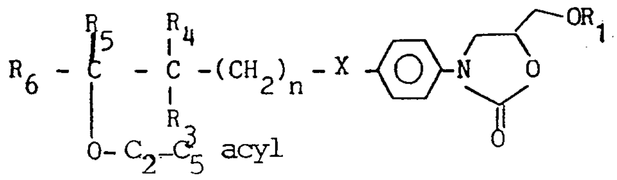

wherein the various parameters have the same meaning as in formula (I) ; or

k) O - alkylating with a $C_1$-$C_4$ alkyl sulphate or halide or with a benzyl halogenide, compounds of formula :

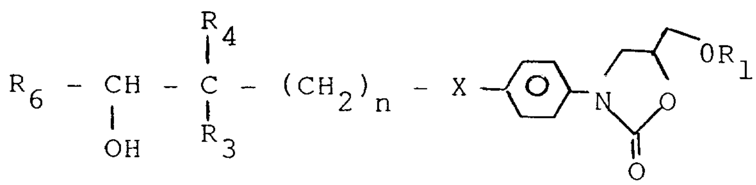

wherein $R_1$ = $C_1$-$C_4$ alkyl and X, n, $R_3$ $R_4$, and $R_6$ have the same meaning as in formula (I) ;

the above-mentioned reactions a) to k) being optionally followed by the action of an halogen on the compounds obtained by these reactions.

**Claims for the following Contracting States : ES, GR**

**1.** Process for the preparation of derivatives of the formula :

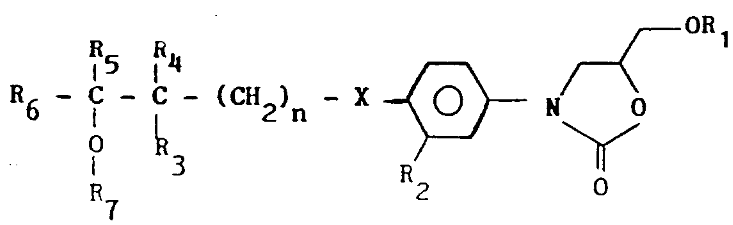

(I)

wherein :

- $R_1$ is H or $C_1$-$C_4$ alkyl ;
- X is either an oxygen atom, in which case $R_2$ = H or halogen, or a methylene group or a -CH=CH- group, in which case $R_2$ = H ;
- n is 1 or 2 when X is an oxygen atom or a methylene group and 0 or 1 when X is a -CH=CH- group ;
- each of $R_3$ and $R_4$ is independently H, $C_1$-$C_4$ alkyl or $C_4$-$C_7$ cycloalkyl ;
- $R_5$ is H and $R_6$ is a $CF_3$ or $C_4$-$C_7$ cycloalkyl group or $R_5$ is a $C_1$-$C_4$ alkyl group and $R_6$ is a $C_1$-$C_4$ alkyl or $C_4$-$C_7$ cycloalkyl group ;
- $R_4$ and $R_6$ may further form together a $-(CH_2)_3-$ or $-(CH_2)_4-$ chain ;
- $R_5$ and $R_6$ may further form together a $-(CH_2)_4-$ or $-(CH_2)_5-$ chain ; and
- $R_7$ is H, $C_1$-$C_4$ alkyl or benzyl,

these derivatives being under the form of diastereoisomers or enantiomers or under the cis- or trans-form or under the form of a mixture of all these forms, including the racemic forms, characterized in that it comprises :

a) O-alkylating compounds of formula :

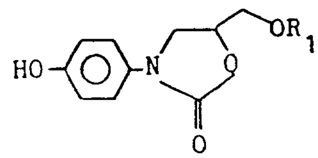

wherein $R_1$ has the same meaning as in formula (I), with a compound of formula :

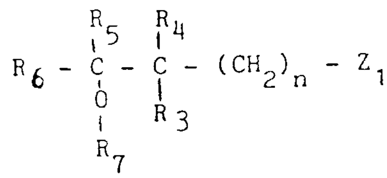

wherein :

- $Z_1$ is OTs, OMs or halogen ;
- $R_3 = R_4 = H$ ; and
- n, $R_5$, $R_6$ and $R_7$ have the same meaning as in formula (I) ;

b) condensing compound of formula :

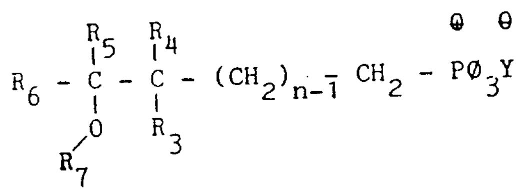

wherein :

- Y = halogen ; and
- $R_3$ to $R_7$ and n have the same meaning as in formula (I),

with compounds of formula :

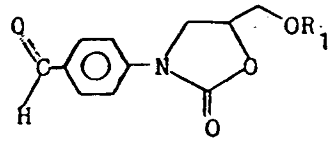

wherein $R_1 = C_1\text{-}C_4$ alkyl ;

c) hydrogenating the double bond of compounds of formula :

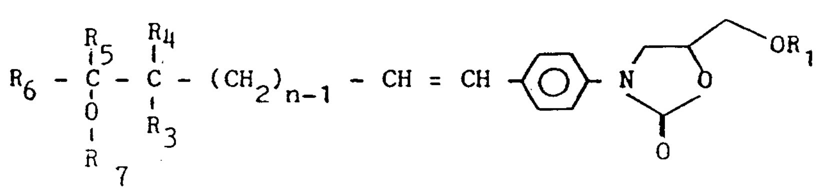

wherein n and $R_3$ to $R_7$ have the same meaning as in formula (I) ;
d) reducing to

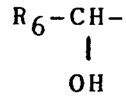 the 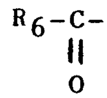

group of compounds of formula :

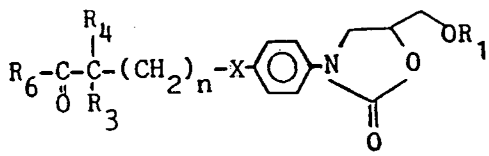

wherein X, n, $R_1$, $R_3$, $R_4$ and $R_6$ have the same meaning as in formula (I) , except that $R_6$ cannot be $CF_3$ ;
e) condensing a $C_1$-$C_4$ alkyl carbonate with compounds of formula :

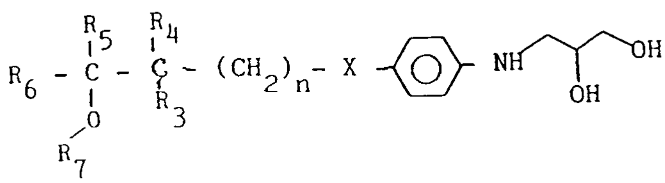

wherein n, X, and $R_3$ to $R_7$ have the same meaning as in formula (I);
f) alkylating with a $C_1$-$C_4$ alkyl halide compounds of formula :

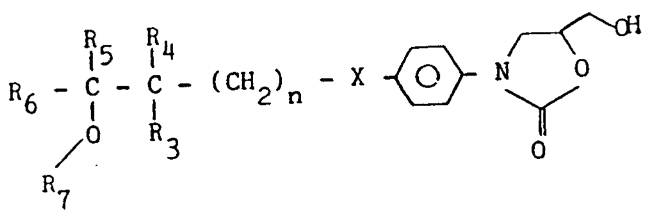

wherein n, X and $R_3$ to $R_7$ have the same meaning as in formula (I);
g) condensing with compounds of formula :

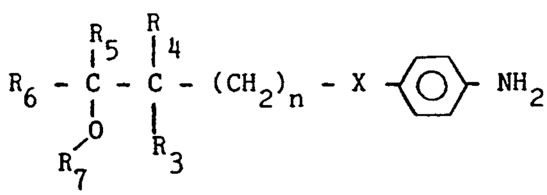

wherein n, X and $R_3$ to $R_7$ have the same meaning as in formula (I),
a compound of formula :

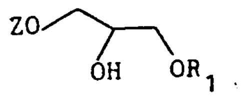

wherein $R_1$ = $C_1$-$C_4$ alkyl ; and Z = Ts or Ms ;

h) condensing with compounds of formula :

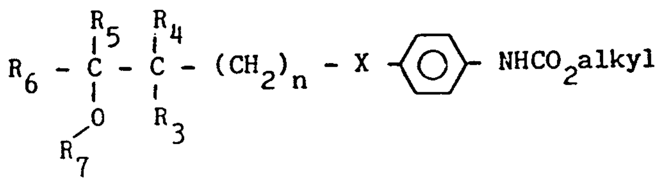

wherein n, X and $R_3$ to $R_7$ have the same meaning as in formula (I), a compound of formula :

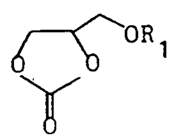

or

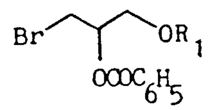

wherein $R_1$ = $C_1$-$C_4$ alkyl ;

i) hydrolyzing with an aqueous acid a compound of formula :

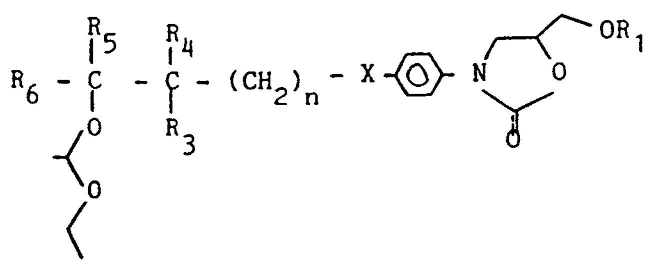

wherein $R_1$ = $C_1$-$C_4$ alkyl ; and n, X and $R_3$ to $R_6$ have the same meaning as in formula (I) ;

j) desacylating by basic hydrolysis compounds of formula :

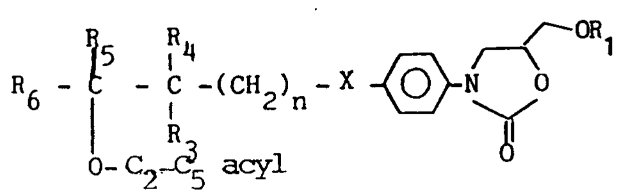

wherein the various parameters have the same meaning as in formula (I) ; or

k) O-alkylating with a $C_1$-$C_4$ alkyl sulphate or halide or with a benzyl halogenide, compounds of formula :

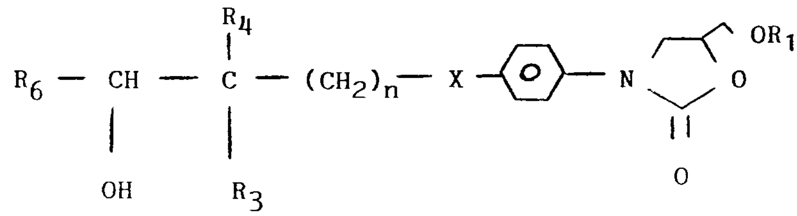

wherein $R_1$ = $C_1$-$C_4$ alkyl and X, n, $R_3$, $R_4$ and $R_6$ have the same meaning as in formula (I) ; the above-mentioned reactions a) to k) being optionally followed by the action of an halogen on the compounds obtained by these reactions.

**2.** Process according to Claim 1, for the preparation of the derivatives of formula (I) wherein $R_1$ = H or $CH_3$ ; $R_2$ = H ; X = oxygen or $CH_2$ ; n = 1 or 2 ; $R_3$ and $R_4$ are H or $CH_3$; $R_5$ is H ; $R_6$ is $CF_3$ ; and $R_7$ is H or $CH_3$.

**3.** Process according to claim 1, for the preparation of the derivatives of formula (I) wherein $R_1$ = H or $CH_3$ ; $R_2$ = H ; X = oxygen or $CH_2$ ; n = 1 or 2 ; $R_3$ and $R_4$ are H or $CH_3$; $R_5$ and $R_6$ = $CH_3$ ; and $R_7$ = H or $CH_3$.

**4.** Process according to claim 1, for the preparation of the derivatives of formula (I) wherein $R_1$ = $CH_3$ ; $R_2$ = H ; X = oxygen; n = 1 or 2 ; $R_3$ = $R_4$ = $R_5$ = H ; $R_6$ = $CF_3$ ; and $R_7$ = H.

**5.** Process according to claim 1, for the preparation of the derivatives of formula (I) wherein $R_1$ = $CH_3$ ; $R_2$ = H ; X = methylene ; n = 1 or 2 ; $R_3$ = $R_4$ = $R_5$ = H ; $R_6$ = $CF_3$ ; and $R_7$ = H.

**6.** Process according to claim 1, for the preparation of the derivatives of formula (I) wherein $R_1$ = $CH_3$ ; $R_2$ = H ; X represents CH = CH ; n = 0 or 1 ; $R_3$ = $R_4$ = $R_5$ = H ; $R_6$ = $CF_3$ and $R_7$ = H.

**7.** Process according to claim 1, for the preparation of the derivatives of formula (I) wherein $R_1$ = $CH_3$ ; $R_2$ = $R_3$ = $R_4$ = $R_5$ = $R_7$ = H ; n = 1 ; $R_6$ = $CF_3$ ; and X = oxygen.

**8.** Process according to claim 7, for the preparation of the derivative wherein the two asymmetric carbon atoms have (R) configuration.

**Patentansprüche**

**Patentansprüche für folgende Vertragsstaaten : AT, BE, CH, DE, DK, FR, GB, IT, LI, LU, NL, SE**

**1.** Derivate gemäß der Formel:

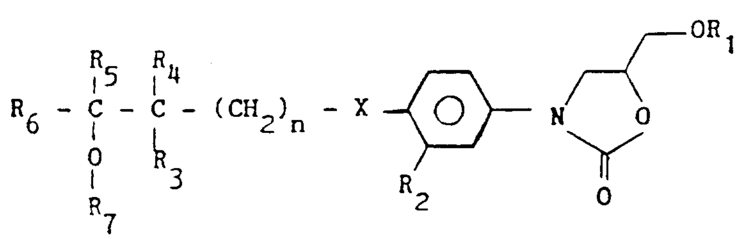

(I)

worin:

- $R_1$ H oder $C_1$-$C_4$-Alkyl bedeutet;
- X entweder ein Sauerstoffatom bedeutet, wobei in diesem Fall $R_2$ H oder Halogen ist, oder eine Methylengruppe oder eine -CH=CH- Gruppe bedeutet, wobei in diesem Fall $R_2$ H ist;
- n den Wert 1 oder 2 annimmt, wenn X ein Sauerstoffatom oder eine Methylengruppe ist, und den Wert 0 oder 1 annimmt, wenn X die -CH=CH- Gruppe bedeutet;
- $R_3$ und $R_4$ jeweils unabhängig voneinander H, $C_1$-$C_4$-Alkyl oder $C_4$-$C_7$-Cycloalkyl bedeuten;

- $R_5$ H bedeutet und $R_6$ eine $CF_3$-Gruppe oder $C_4$-$C_7$-Cycloalkylgruppe bedeutet, oder $R_5$ eine $C_1$-$C_4$-Alkylgruppe ist und $R_6$ eine $C_1$-$C_4$-Alkylgruppe oder $C_4$-$C_7$-Cycloalkylgruppe ist;
- $R_4$ und $R_6$ darüber hinaus zusammen eine $-(CH_2)_3-$ oder $-(CH_2)_4$-Kette bilden können;
- $R_5$ und $R_6$ darüber hinaus zusammen eine $-(CH_2)_4-$ oder $-(CH_2)_5-$ Kette bilden können; und
- $R_7$ H, $C_1$-$C_4$-Alkyl oder Benzyl bedeutet,

wobei diese Derivate in Form von Diastereomeren oder Enantiomeren oder in cis- oder trans-Form oder auch in Form eines Gemisches aller dieser Formen einschließlich der racemischen Formen vorliegen.

**2.** Derivate nach Anspruch 1, worin $R_1$ = H oder $CH_3$; $R_2$ = H; X = Sauerstoff oder $CH_2$; n = 1 oder 2; $R_3$ und $R_4$ H oder $CH_3$ bedeuten; $R_5$ = H; $R_6$ $CF_3$ bedeutet; und $R_7$ H oder $CH_3$ bedeutet.

**3.** Derivate nach Anspruch 1, worin $R_1$ = H oder $CH_3$; $R_2$ = H; X = Sauerstoff oder $CH_2$; n = 1 oder 2; $R_3$ und $R_4$ H oder $CH_3$ bedeuten; $R_5$ und $R_6$ = $CH_3$; und $R_7$ = H oder $CH_3$.

**4.** Derivate nach Anspruch 1, worin $R_1$ = $CH_3$; $R_2$ = H; X = Sauerstoff; n = 1 oder 2; $R_3$ = $R_4$ = $R_5$ = H; $R_6$ = $CF_3$; und $R_7$ = H.

**5.** Derivate nach Anspruch 1, worin $R_1$ = $CH_3$; $R_2$ = H; X = Methylen; n = 1 oder 2; $R_3$ = $R_4$ = $R_5$ = H; $R_6$ = $CF_3$; und $R_7$ = H.

**6.** Derivate nach Anspruch 1, worin $R_1$ = $CH_3$; $R_2$ = H; X CH=CH bedeutet; n = 0 oder 1; $R_3$ = $R_4$ = $R_5$ = H; $R_6$ = $CF_3$ und $R_7$ = H.

**7.** Derivat nach Anspruch 1, worin $R_1$ = $CH_3$; $R_2$ = $R_3$ = $R_4$ = $R_5$ = $R_7$ = H; n = 1, $R_6$ = $CF_3$ und X = Sauerstoff.

**8.** Derivat nach Anspruch 7, worin die beiden asymmetrischen Kohlenstoffatome die (R)-Konfiguration haben.

**9.** Pharmazeutische Zusammensetzung, **dadurch gekennzeichnet**, daß sie ein physiologisch annehmbares Excipiens in Verbindung mit mindestens einem Derivat (I) nach einem der Ansprüche 1 bis 8 umfaßt.

**10.** Verwendung von Derivaten nach den Ansprüchen 1 bis 8 zur Herstellung von psychotropen Arzneimitteln.

**11.** Verfahren zur Herstellung von Derivaten der Formel (I) nach Anspruch 1, **dadurch gekennzeichnet**, daß es umfaßt:

a) die O-Alkylierung von Verbindungen der Formel

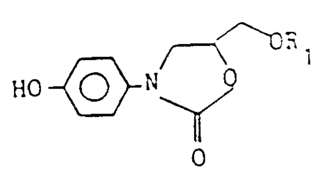

worin $R_1$ die gleiche Bedeutung wie in Formel (I) hat, durch eine Verbindung der Formel:

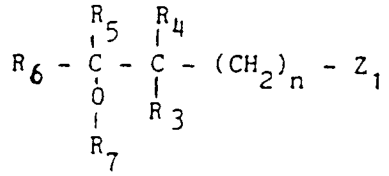

worin $Z_1$ = OTs, OMs oder Halogen; $R_3$ = $R_4$ = H; und n, $R_5$, $R_6$ und $R_7$ die gleiche Bedeutung

wie in Formel (I) haben;

b) die Kondensation der Verbindung der Formel:

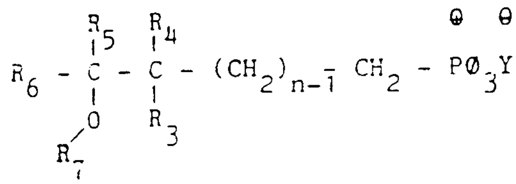

worin Y = Halogen und $R_3$ bis $R_7$ und n die gleiche Bedeutung wie in Formel (I) haben, mit den Verbindungen der Formel:

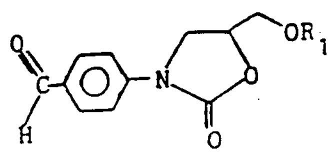

worin $R_1$ = $C_1$-$C_4$-Alkyl;

c) die Hydrierung der Doppelbindung von Verbindungen der Formel

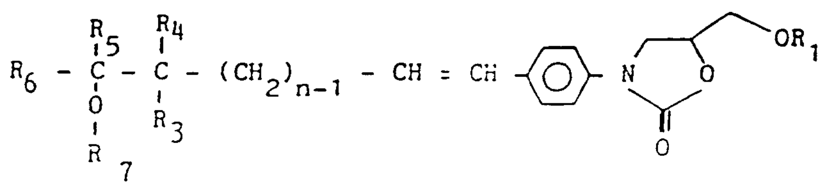

worin n und $R_3$ bis $R_7$ die gleiche Bedeutung wie in der Formel (I) haben;

d) die Reduktion zu

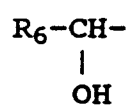 von 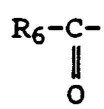

von Verbindungen der Formel:

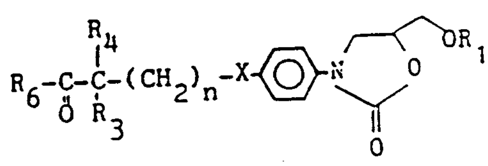

worin X, n, $R_1$, $R_3$, $R_4$ und $R_6$ die gleiche Bedeutung wie in der Formel (I) haben, $R_6$ kann aber nicht die Bedeutung $CF_3$ haben ;

e) die Kondensation eines $C_1$-$C_4$-Alkylcarbonats mit den Verbindungen der Formel:

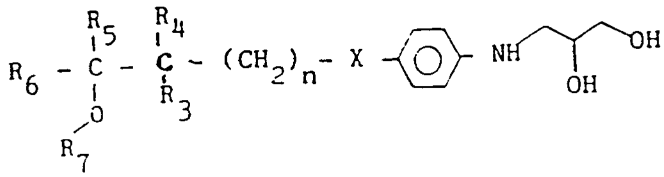

worin n, X und $R_3$ bis $R_7$ die gleiche Bedeutung wie in der Formel (I) haben;
f) die Alkylierung von Verbindungen der Formel:

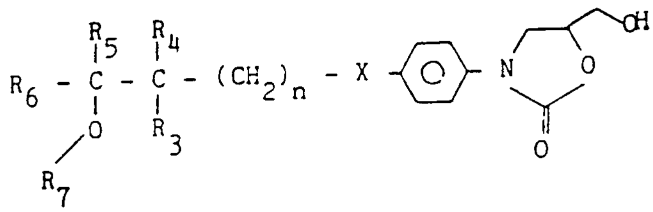

worin n, X und $R_3$ bis $R_7$ die gleiche Bedeutung wie in der Formel (I) haben, mit einem $C_1$-$C_4$-Alkylhalogenid;
g) die Kondensation von Verbindungen der Formel:

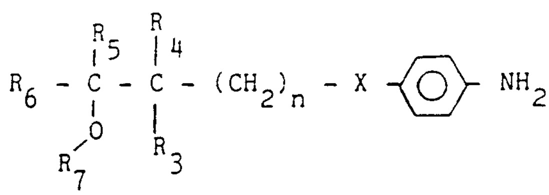

worin n, X und $R_3$ bis $R_7$ die gleiche Bedeutung wie in der Formel (I) haben, mit einer Verbindung der Formel:

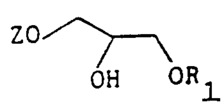

worin $R_1$ = $C_1$-$C_4$-Alkyl und Z = Ts oder Ms;
h) die Kondensation von Verbindungen der Formel:

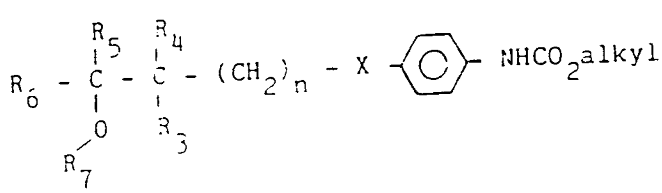

worin n, X und $R_3$ bis $R_7$ die gleiche Bedeutung wie in der Formel (I) haben, mit einer Verbindung der Formel:

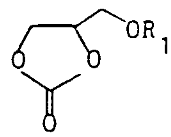 oder 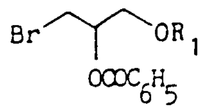

worin $R_1$ = $C_1$-$C_4$-Alkyl;

i) die Hydrolyse der Verbindung der Formel:

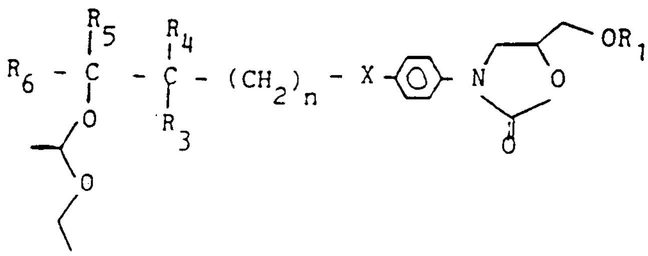

worin $R_1$ = $C_1$-$C_4$-Alkyl und n, X und $R_3$ bis $R_6$ die gleiche Bedeutung wie in der Formel (I) haben, mit einer wäßrigen Säure;

j) die Desacylierung durch basische Hydrolyse von Verbindungen der Formel:

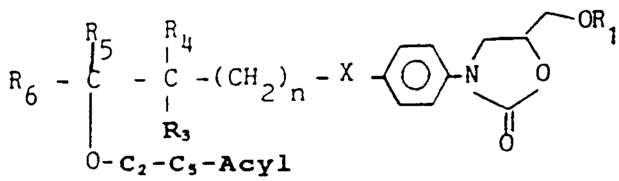

worin die verschiedenen Parameter die gleiche Bedeutung wie in der Formel (I) haben; oder

k) die O-Alkylierung von Verbindungen der Formel

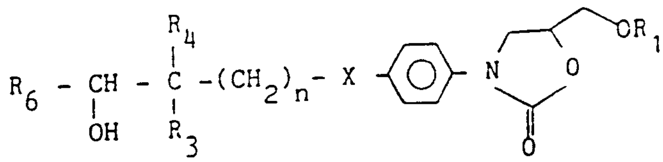

worin $R_1$ = $C_1$-$C_4$-Alkyl und X, n, $R_3$, $R_4$ und $R_6$ die gleiche Bedeutung wie in der Formel I haben, mit einem $C_1$-$C_4$-Alkylhalogenid oder -Sulfat oder einem Benzylhalogenid;

wobei auf die vorstehenden Reaktionen a) bis k) gegebenenfalls die Einwirkung eines Halogens auf die durch diese Reaktionen erhaltenen Verbindungen folgt.

**Patentansprüche für folgende Vertragsstaaten : ES, GR**

1. Verfahren zur Herstellung von Derivaten gemäß der Formel:

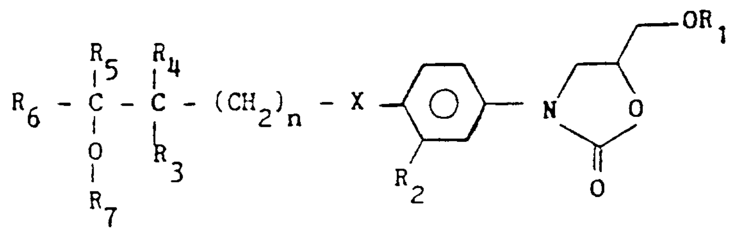

(I)

worin:

- $R_1$ H oder $C_1$-$C_4$-Alkyl bedeutet;
- X entweder ein Sauerstoffatom bedeutet, wobei in diesem Fall $R_2$ H oder Halogen ist, oder eine Methylengruppe oder eine -CH=CH- Gruppe bedeutet, wobei in diesem Fall $R_2$ H ist;
- n den Wert 1 oder 2 annimmt, wenn X ein Sauerstoffatom oder eine Methylengruppe ist, und den Wert 0 oder 1 annimmt, wenn X die -CH=CH- Gruppe bedeutet;
- $R_3$ und $R_4$ jeweils unabhängig voneinander H, $C_1$-$C_4$-Alkyl oder $C_4$-$C_7$-Cycloalkyl bedeuten;
- $R_5$ H bedeutet und $R_6$ eine $CF_3$-Gruppe oder $C_4$-$C_7$-Cycloalkylgruppe bedeutet, oder $R_5$ eine $C_1$-$C_4$-Alkylgruppe ist und $R_6$ eine $C_1$-$C_4$-Alkylgruppe oder $C_4$-$C_7$-Cycloalkylgruppe ist;
- $R_4$ und $R_6$ darüber hinaus zusammen eine $-(CH_2)_3-$ oder $-(CH_2)_4-$Kette bilden können;
- $R_5$ und $R_6$ darüber hinaus zusammen eine $-(CH_2)_4-$ oder $-(CH_2)_5-$ Kette bilden können; und
- $R_7$ H, $C_1$-$C_4$-Alkyl oder Benzyl bedeutet,

wobei diese Derivate in Form von Diastereomeren oder Enantiomeren oder in cis- oder trans-Form oder auch in Form eines Gemisches aller dieser Formen einschließlich der racemischen Formen vorliegen, **dadurch gekennzeichnet**, daß es umfaßt:

a) die O-Alkylierung von Verbindungen der Formel

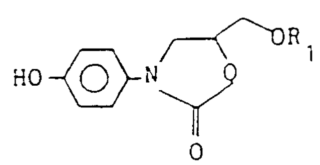

worin $R_1$ die gleiche Bedeutung wie in Formel (I) hat, durch eine Verbindung der Formel:

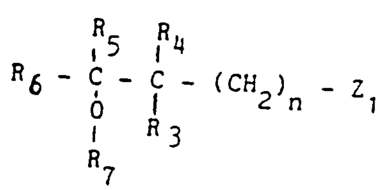

worin $Z_1$ = OTs, OMs oder Halogen; $R_3$ = $R_4$ = H; und n, $R_5$, $R_6$ und $R_7$ die gleiche Bedeutung wie in Formel (I) haben;

b) die Kondensation der Verbindung der Formel:

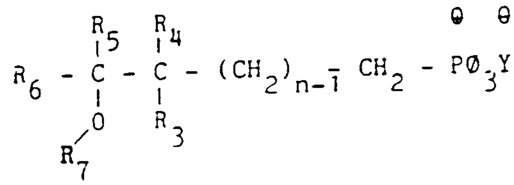

worin Y = Halogen und $R_3$ bis $R_7$ und n die gleiche Bedeutung wie in Formel (I) haben, mit den Verbindungen der Formel:

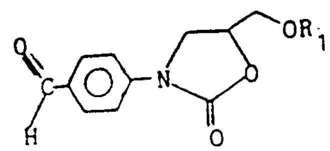

worin $R_1$ = $C_1$-$C_4$-Alkyl;

c) die Hydrierung der Doppelbindung von Verbindungen der Formel

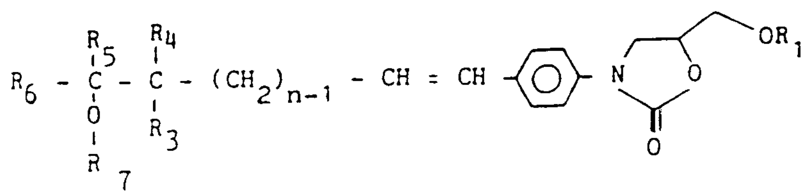

worin n und $R_3$ bis $R_7$ die gleiche Bedeutung wie in der Formel (I) haben;

d) die Reduktion zu

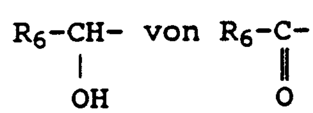

von Verbindungen der Formel:

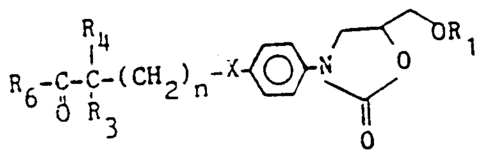

worin X, n, $R_1$, $R_3$, $R_4$ und $R_6$ die gleiche Bedeutung wie in der Formel (I) haben, $R_6$ kann aber nicht die Bedeutung $CF_3$ haben ;

e) die Kondensation eines $C_1$-$C_4$-Alkylcarbonats mit den Verbindungen der Formel:

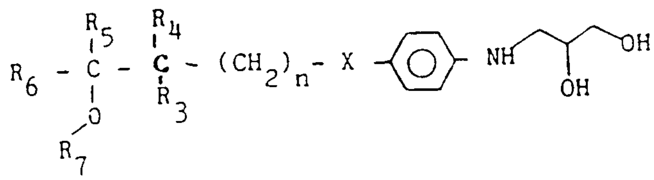

worin n, X und $R_3$ bis $R_7$ die gleiche Bedeutung wie in der Formel (I) haben;
f) die Alkylierung von Verbindungen der Formel:

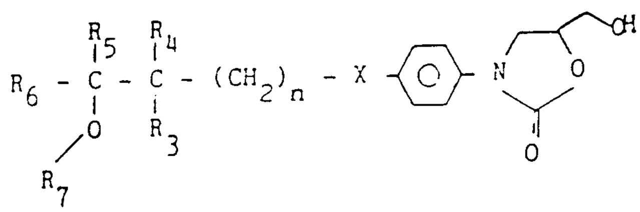

worin n, X und $R_3$ bis $R_7$ die gleiche Bedeutung wie in der Formel (I) haben, mit einem $C_1$-$C_4$-Alkylhalogenid;
g) die Kondensation von Verbindungen der Formel:

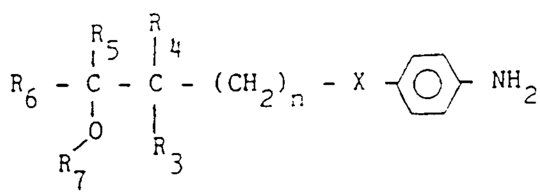

worin n, X und $R_3$ bis $R_7$ die gleiche Bedeutung wie in der Formel (I) haben, mit einer Verbindung der Formel:

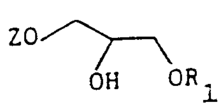

worin $R_1$ = $C_1$-$C_4$-Alkyl und Z = Ts oder Ms;
h) die Kondensation von Verbindungen der Formel:

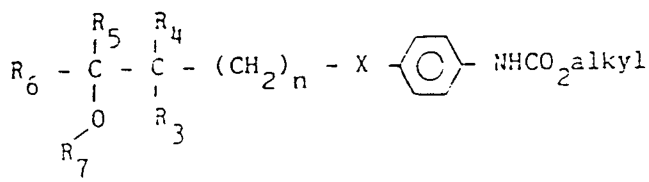

worin n, X und $R_3$ bis $R_7$ die gleiche Bedeutung wie in der Formel (I) haben, mit einer Verbindung der Formel:

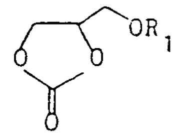 oder 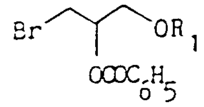

worin $R_1$ = $C_1$-$C_4$-Alkyl;

i) die Hydrolyse der Verbindung der Formel:

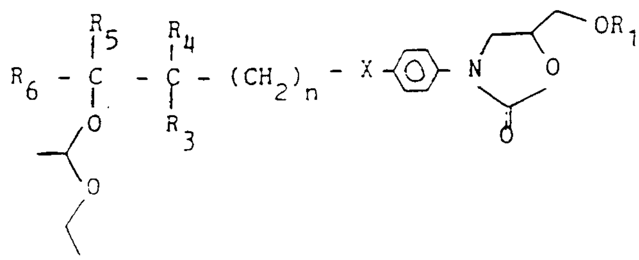

worin $R_1$ = $C_1$-$C_4$-Alkyl und n, X und $R_3$ bis $R_6$ die gleiche Bedeutung wie in der Formel (I) haben, mit einer wäßrigen Säure;

j) die Desacylierung durch basische Hydrolyse von Verbindungen der Formel:

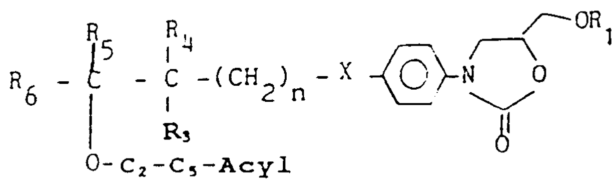

worin die verschiedenen Parameter die gleiche Bedeutung wie in der Formel (I) haben; oder

k) die O-Alkylierung von Verbindungen der Formel

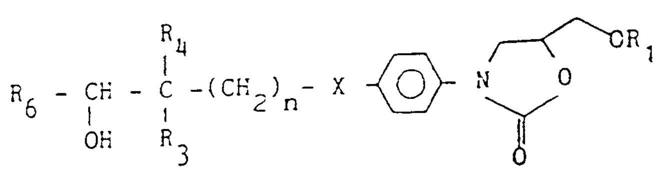

worin $R_1$ = $C_1$-$C_4$-Alkyl und X, n, $R_3$, $R_4$ und $R_6$ die gleiche Bedeutung wie in der Formel I haben, mit einem $C_1$-$C_4$-Alkylhalogenid oder -Sulfat oder einem Benzylhalogenid;

wobei auf die vorstehenden Reaktionen a) bis k) gegebenenfalls die Einwirkung eines Halogens auf die durch diese Reaktionen erhaltenen Verbindungen folgt.

**2.** Verfahren nach Anspruch 1, zur Herstellung von Derivaten der Formel (I), worin $R_1$ = H oder $CH_3$; $R_2$ = H; X = Sauerstoff oder $CH_2$; n = 1 oder 2; $R_3$ und $R_4$ H oder $CH_3$ bedeuten; $R_5$ = H; $R_6$ $CF_3$ bedeutet; und $R_7$ H oder $CH_3$ bedeutet.

**3.** Verfahren nach Anspruch 1, zur Herstellung von Derivaten der Formel (I), worin $R_1$ = H oder $CH_3$; $R_2$ = H; X = Sauerstoff oder $CH_2$; n = 1 oder 2; $R_3$ und $R_4$ H oder $CH_3$ bedeuten; $R_5$ und $R_6$ = $CH_3$; und $R_7$ = H oder $CH_3$.

4. Verfahren nach Anspruch 1, zur Herstellung von Derivaten der Formel (I), worin $R_1 = CH_3$; $R_2 = H$; X = Sauerstoff; n = 1 oder 2; $R_3 = R_4 = R_5 = H$; $R_6 = CF_3$; und $R_7 = H$.

5. Verfahren nach Anspruch 1, zur Herstellung von Derivaten der Formel (I), worin $R_1 = CH_3$; $R_2 = H$; X = Methylen; n = 1 oder 2; $R_3 = R_4 = R_5 = H$; $R_6 = CF_3$; und $R_7 = H$.

6. Verfahren nach Anspruch 1, zur Herstellung von Derivaten der Formel (I), worin $R_1 = CH_3$; $R_2 = H$; X CH=CH bedeutet; n = 0 oder 1; $R_3 = R_4 = R_5 = H$; $R_6 = CF_3$ und $R_7 = H$.

7. Verfahren nach Anspruch 1, zur Herstellung des Derivats der Formel (I), worin $R_1 = CH_3$; $R_2 = R_3 = R_4 = R_5 = R_7 = H$; n = 1, $R_6 = CF_3$ und X = Sauerstoff.

8. Verfahren nach Anspruch 7, zur Herstellung des Derivats, worin die beiden asymmetrischen Kohlenstoffatome die (R)-Konfiguration haben.